# EUROPEAN PATENT APPLICATION

(11) **EP 4 382 528 A1**
(43) Date of publication of application: **12.06.2024**
(21) Application number: 22852140.7
(22) Date of filing: 02.08.2022
(51) Int. Cl.: C07D 471/04, C07D 401/14, A61K 31/4353, A61K 31/4375, A61P 31/00

(54) **CRYSTAL FORM OF RIPK1 INHIBITOR, ACID SALT THEREOF, AND CRYSTAL FORM OF ACID SALT THEREOF**

(30) Priority: 02.08.2021 CN 202110882757
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: ZHOU, Fusheng, Shanghai 201203 (CN); TAO, Yuanzhi, Shanghai 201203 (CN); ZHAO, Jinzhu, Shanghai 201203 (CN); LAN, Jiong, Shanghai 201203 (CN)
(74) Representative: Henderson, Helen Lee
(86) International application number: PCT/CN2022/109562
(87) International publication number: WO 2023/011428

(57) **Abstract**

Disclosed are a crystal form of a RIPK1 inhibitor, an acid salt thereof, and a crystal form of an acid salt thereof. The crystal form of the RIPK1 inhibitor, the acid salt thereof, and the crystal form of the acid salt thereof have advantages of good stability and low hygroscopicity, and same have good pharmaceutical prospects.

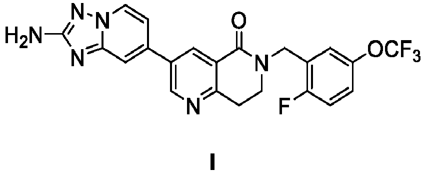

## Description

The present application claims the priority of Chinese patent application 2021108827578 filed on August 2, 2021. The contents of the above Chinese patent application are incorporated herein by reference in its entirety.

### TECHNICAL FIELD

The present disclosure relates to the field of pharmaceutical technology, specifically relates to a crystal form of a RIPK1 inhibitor, an acid salt thereof, and a crystal form of the acid salt thereof.

### BACKGROUND

Receptor-interacting protein 1 (RIP1) kinase is a TKL family serine/threonine protein kinase involved in innate immune signaling. RIP1 kinase is a RHIM domain containing protein, with an N-terminalkinase domain and a C- terminal death domain. The death domain of RIP1 mediates interactions with other proteins that contain death domains, and the proteins include Fas and TNFR-1, TRAIL-R1 and TRAIL-R2 and TRADD. The RHIM domain is essential for binding to other proteins that contain RHIM domains, such as TRIF, DAI, and RIP3, and realizes many functions through these interactions.

The role of RIP1 in cell signaling has been evaluated under different conditions, including TLR3, TLR4, TRAIL, and FAS, but is best understood in the context of mediating signals downstream of the death receptor TNFR1. Engagement of the TNFR by TNF leads to oligomerization, recruits multiple proteins, including linear K63-linked polyubiquitinated RIP1, TRAF2/5, TRADD, and cIAPs, to the cytoplasmic tail region of the receptor. This complex, which depends on RIP1 and acts as a scaffolding protein (i.e., kinase-independent), is called complex I. It provides a platform for pro-survival signaling by activating the NFκB and MAP kinase pathways. In addition, binding of TNF to its receptor under conditions promoting the deubiquitination of RIP1 (by proteins such as A20 and CYLD or inhibition of the cIAPs) results in receptor internalization and the formation of complex II or DISC (death-inducing signaling complex). Formation of the DISC, which contains RIP1, TRADD, FADD, and caspase 8, results in the activation of caspase 8 and the onset of programmed apoptotic cell death also in a RIP1 kinase independent fashion. Apoptosis is largely a quiescent form of cell death that participates in routine processes such as development and intracellular homeostasis.

Under conditions where the DISC forms and RIP3 is expressed, but apoptosis is inhibited (such as FADD/caspase-8 deletion, caspase inhibition, or viral infection), a third RIP1 kinase dependence may exist. Now, RIP3 can enter this complex, be phosphorylated by RIP1, and initiate a caspase-independent programmed necrotic cell death through the activation of MLKL and PGAM5. In contrast to apoptosis, programmed necrosis (not to be confused with non-programmed passive necrosis) results in the release of danger-associated molecular patterns (DAMP) from cells. These DAMPs can provide a "danger signal" to surrounding cells and tissues, inducing a pro-inflammatory response, including inflammasome activation, cytokine production, and cell recruitment responses.

Using RIP3 knockout mice (in which RIP1-mediated programmed necrosis is completely blocked) and Necrostatin-1 (a tool inhibitor of RIP1 kinase activity with poor oral bioavailability), it has been demonstrated that dysregulation of RIP1 kinase-mediated programmed cell death is associated with various inflammatory conditions. RIP3 knockout mice have been shown to be protective against inflammatory bowel diseases (including ulcerative colitis and Crohn's disease), psoriasis, photoreceptor cell death induced by retinal detachment, retinitis pigmentosa, acute pancreatitis induced by caerulein, and sepsis/systemic inflammatory response syndrome. Necrostatin-1 has been shown to be effective in alleviating ischemic brain injury, retinal ischemia/reperfusion injury, Huntington's disease, renal ischemia-reperfusion injury, cisplatin-induced kidney injury, and traumatic brain injury. Other diseases or disorders regulated at least in part by RIP1-dependent apoptosis, necrosis, or cytokine production include: malignant tumors of the blood and solid organs, bacterial and viral infections (including but not limited to tuberculosis and influenza), lysosomal storage diseases (especially Gaucher disease).

An effective, selective, small molecule RIP1 kinase inhibitor that can block RIP1-dependent necroptosis, thereby providing therapeutic effects for diseases or events associated with DAMP, cell death, and/or inflammation.

### CONTENT OF THE PRESENT INVENTION

The technical problem to be solved by the present disclosure is to overcome the deficiency of few types of RIP1 inhibitors in the prior art. To this end, a crystal form of a RIPK1 inhibitor, an acid salt thereof, and a crystal form of the acid salt thereof are provided. The crystal form of the RIPK1 inhibitor, the acid salt thereof, and the crystal form of the acid salt thereof in the present disclosure have the advantages of low hygroscopicity and good stability, and have good prospects for druggability.

The first aspect of the present disclosure provides a crystal form A of a compound of formula I, which has an X-ray powder diffraction pattern expressed at a 2θ angle comprising diffraction peaks at the following 2θ angles: 10.94 ± 0.2°, 16.43 ± 0.2°, 19.12 ± 0.2°, and 19.81 ± 0.2°;

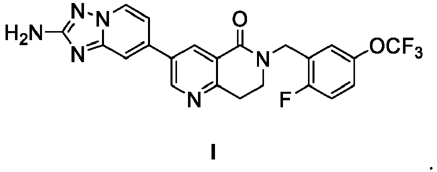

In a certain preferred embodiment, the crystal form **A** of the compound of formula I has an X-ray powder diffraction pattern expressed at a 2θ angle further comprising diffraction peaks at one or more than one of the following 2θ angles: 21.25 ± 0.2°, 22.32 ± 0.2°, 27.55 ± 0.2°, 20.23 ± 0.2°, and 18.41 ± 0.2°.

Preferably, the crystal form **A** of the compound of formula I has an X-ray powder diffraction pattern expressed at a 2θ angle further comprising diffraction peaks at one or more than one of the following 2θ angles: 18.13 ± 0.2°, 33.21 ± 0.2°, 17.86 ± 0.2°, 23.47 ± 0.2°, 26.91 ± 0.2°, and 16.77 ± 0.2°.

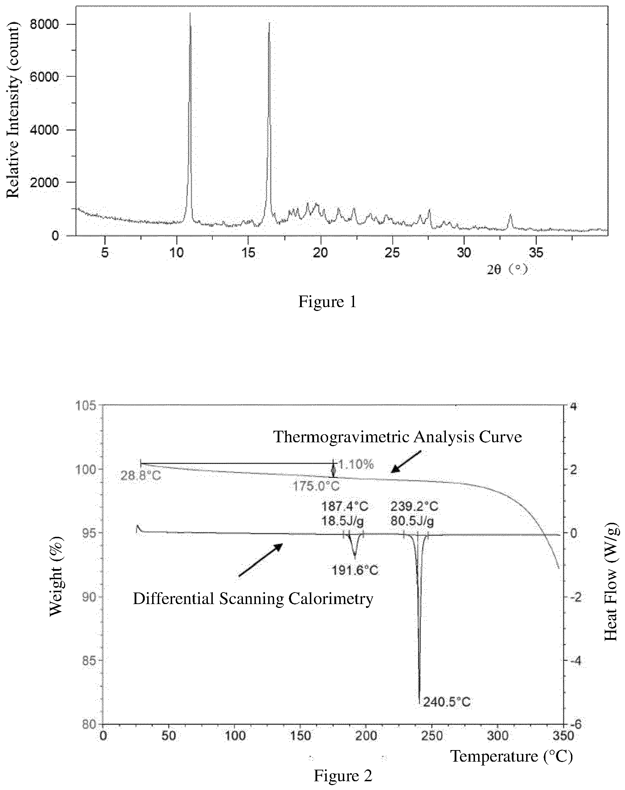

In a certain preferred embodiment, the crystal form **A** of the compound of formula I has an X-ray powder diffraction pattern expressed at a 2θ angle basically as shown in Figure 1.

In a certain preferred embodiment, the thermogravimetric analysis pattern (TGA) of the crystal form **A** of the compound of formula I has a weight loss of 0.5% to 2% (such as 1.1%) when heated from an onset to 175 ± 5°C (the percentage of the weight loss is the percentage of the weight loss of the sample relative to the weight of the sample prior to this weight loss).

In a certain preferred embodiment, the crystal form **A** of the compound of formula I has a differential scanning calorimetry pattern showing an endothermic peak at 191.6 ± 5°C and/or 240.5 ± 5°C.

In a certain preferred embodiment, the crystal form **A** of the compound of formula I has a differential scanning calorimetry pattern and a thermogravimetric analysis pattern basically as shown in Figure 2.

The second aspect of the present disclosure further provides a crystal form **B** of a compound of formula I, which has an X-ray powder diffraction pattern expressed at a 2θ angle comprising diffraction peaks at the following 2θ angles: 27.64 ± 0.2°, 24.48 ± 0.2°, 3.76 ± 0.2°, and 19.30 ± 0.2°;

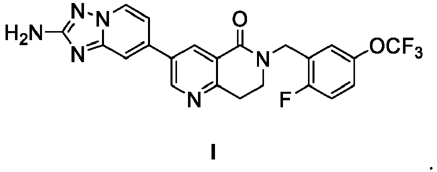

In a certain preferred embodiment, the crystal form **B** of the compound of formula I has an X-ray powder diffraction pattern expressed at a 2θ angle further comprising diffraction peaks at one or more than one of the following 2θ angles: 16.11 ± 0.2°, 18.48 ± 0.2°, 21.23 ± 0.2°, 17.80 ± 0.2°, and 21.41 ± 0.2°.

Preferably, the crystal form **B** of the compound of formula I has an X-ray powder diffraction pattern expressed at a 2θ angle further comprising diffraction peaks at one or more than one of the following 2θ angles: 17.01 ± 0.2°, 28.03 ± 0.2°, 21.96 ± 0.2°, 22.66 ± 0.2°, 30.78 ± 0.2°, and 19.66 ± 0.2°.

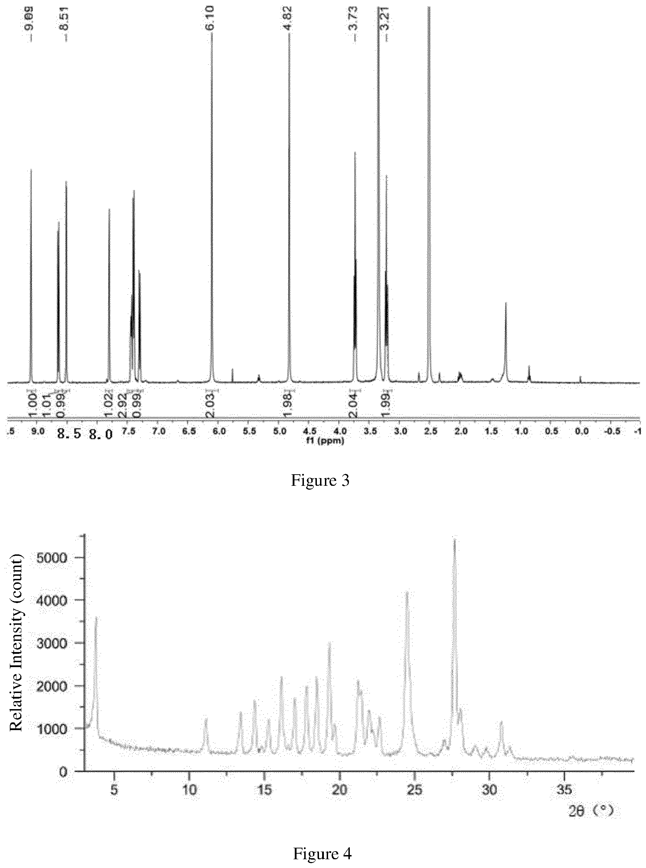

In a certain preferred embodiment, the crystal form **B** of the compound of formula I has an X-ray powder diffraction pattern expressed at a 2θ angle basically as shown in Figure 4.

In a certain preferred embodiment, the thermogravimetric analysis pattern of the crystal form **B** of the compound of formula I has a weight loss of 1% to 3% (such as 2.2%) when heated from an onset to 200 ± 5°C (the percentage of the weight loss is the percentage of the weight loss of the sample relative to the weight of the sample prior to this weight loss).

In a certain preferred embodiment, the crystal form **B** of the compound of formula I has a differential scanning calorimetry pattern showing one endothermic peak at 240.1 ± 5°C.

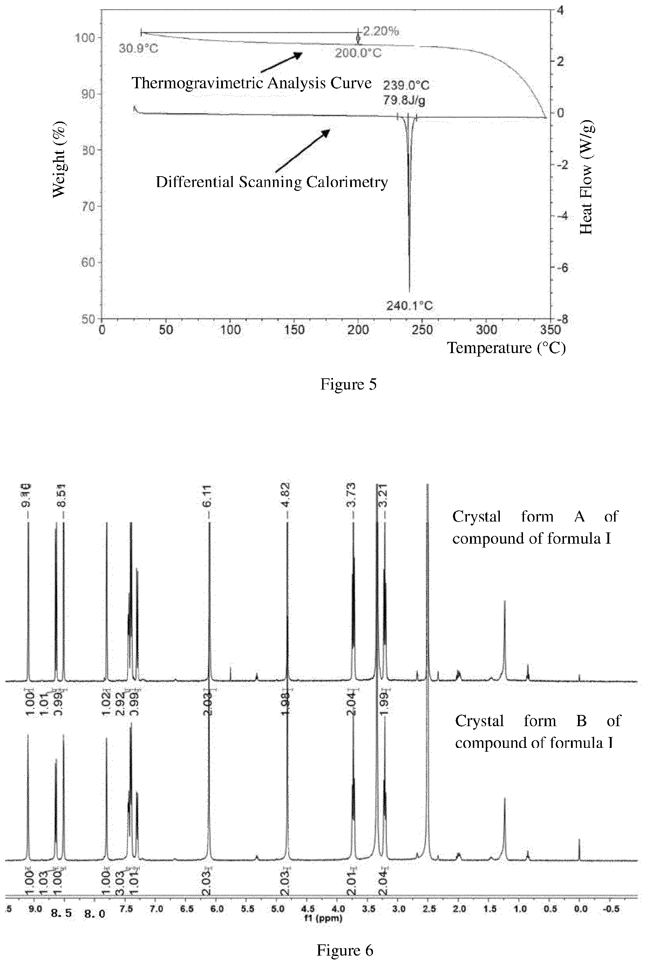

In a certain preferred embodiment, the crystal form **B** of the compound of formula I has a differential scanning calorimetry pattern and a thermogravimetric analysis pattern basically as shown in Figure 5.

The third aspect of the present disclosure provides a crystal form **C** of a compound of formula I, which has an X-ray powder diffraction pattern expressed at a 2θ angle comprising diffraction peaks at the following 2θ angles: 19.55 ± 0.2°, 29.51 ± 0.2°, 19.99 ± 0.2°, and 20.26 ± 0.2°;

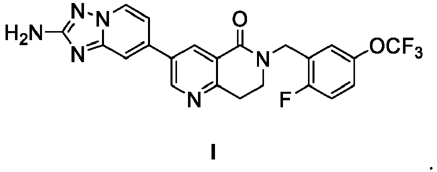

In a certain preferred embodiment, the crystal form **C** of the compound of formula I has an X-ray powder diffraction pattern expressed at a 2θ angle further comprising diffraction peaks at one or more than one of the following 2θ angles: 29.92 ± 0.2°, 23.30 ± 0.2°, 20.57 ± 0.2°, 31.49 ± 0.2°, and 10.11 ± 0.2°.

Preferably, the crystal form **C** of the compound of formula I has an X-ray powder diffraction pattern expressed at a 2θ angle further comprising diffraction peaks at one or more than one of the following 2θ angles: 24.93 ± 0.2°, 20.82 ± 0.2°, 11.60 ± 0.2°, 9.77 ± 0.2°, 10.98 ± 0.2°, and 26.96 ± 0.2°.

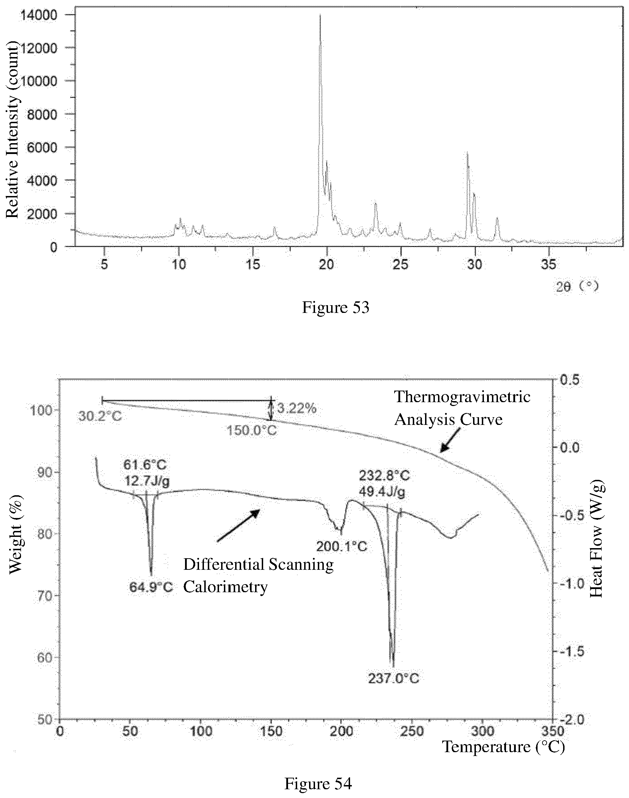

In a certain preferred embodiment, the crystal form **C** of the compound of formula I has an X-ray powder diffraction pattern expressed at a 2θ angle basically as shown in Figure 53.

In a certain preferred embodiment, the thermogravimetric analysis pattern of the crystal form **C** of the compound of formula I has a weight loss of 3% to 4% (such as 3.2%) when heated from an onset to 150 ± 5°C (the percentage of the weight loss is the percentage of the weight loss of the sample relative to the weight of the sample prior to this weight loss).

In a certain preferred embodiment, the crystal form **C** of the compound of formula I has a differential scanning calorimetry pattern showing an endothermic peak at one or more than one of 64.9 ± 5°C, 200.1 ± 5°C, and 237.0 ± 5°C.

In a certain preferred embodiment, the crystal form **C** of the compound of formula I has a differential scanning calorimetry pattern and a thermogravimetric analysis pattern basically as shown in Figure 54.

The fourth aspect of the present disclosure provides a pharmaceutically acceptable salt of a compound of formula I; the pharmaceutically acceptable salt is a salt formed by the compound of formula I and an acid; the acid is an inorganic acid or an organic acid;

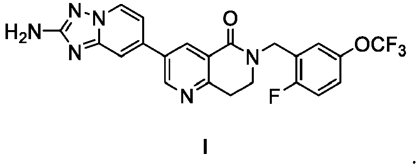

In a certain preferred embodiment, the molar ratio of the compound of formula I to the acid is 1:(0.3-2), such as 1:0.3, 1:0.8, 1:0.9, 1:1, 1:1.1, 1:1.3, or 1:2.

In a certain preferred embodiment, the inorganic acid is hydrochloric acid and/or sulfuric acid.

In a certain preferred embodiment, the organic acid is one or more than one of fumaric acid, maleic acid, citric acid, methanesulfonic acid, *p*-toluenesulfonic acid, cyclamic acid, mucic acid, glycolic acid, malic acid, and hippuric acid.

Preferably, the organic acid is one or more than one of *p*-toluenesulfonic acid, methanesulfonic acid, maleic acid, fumaric acid, citric acid, and cyclamic acid.

More preferably, the organic acid is *p*-toluenesulfonic acid.

In a certain preferred embodiment, the pharmaceutically acceptable salt of the compound of formula I is any one of the following:
(1) a hydrochloride salt of the compound of formula I; wherein the molar ratio of the compound of formula I to hydrochloric acid is 1:2;
(2) a sulfate salt of the compound of formula I; wherein the molar ratio of the compound of formula I to sulfuric acid is 1: (1-2), such as 1: (1-1.3), and further such as 1:1, 1:1.1, 1:1.3, or 1:2;
(3) a citrate salt of the compound of formula I; wherein the molar ratio of the compound of formula I to citric acid is 1:1;
(4) a maleate salt of the compound of formula I; wherein the molar ratio of the compound of formula I to maleic acid is 1: (0.5-1), such as 1:0.8;
(5) a fumarate salt of the compound of formula I; wherein the molar ratio of the compound of formula I to fumaric acid is 1: (0.5-1), such as 1:0.9;
(6) a methanesulfonate salt of the compound of formula I; wherein the molar ratio of the compound of formula I to methanesulfonic acid is 1: (0.1-1), such as 1: (0.3-1), and further such as 1:0.3 or 1:1;
(7) a *p*-toluenesulfonate salt of the compound of formula I; wherein the molar ratio of the compound of formula I to *p*-toluenesulfonic acid is 1: (0.1-1), such as 1:0.9;
(8) a cyclamate salt of the compound of formula I; wherein the molar ratio of the compound of formula I to cyclamic acid is 1: (0.1-1), such as 1:0.9.

The pharmaceutically acceptable salt of the compound of formula I can be prepared by a conventional salt-forming reaction in the art. For example, the pharmaceutically acceptable salt of the compound of formula I can be prepared by the following method:
performing a salt-forming reaction on the compound of formula I and an acid in a solvent to obtain the pharmaceutically acceptable salt of the compound of formula I;
wherein, when the acid is hydrochloric acid, the molar ratio of the compound of formula I to hydrochloric acid is 1:2;
when the acid is sulfuric acid, the molar ratio of the compound of formula I to sulfuric acid is 1: (1-2), such as 1: (1-1.3), and further such as 1:1, 1:1.1, or 1:2;
when the acid is citric acid, the molar ratio of the compound of formula I to citric acid is 1:1;
when the acid is maleic acid, the molar ratio of the compound of formula I to maleic acid is 1: (0.5-1), such as 1:0.8;
when the acid is fumaric acid, the molar ratio of the compound of formula I to fumaric acid is 1: (0.5-1), such as 1:0.9;
when the acid is methanesulfonic acid, the molar ratio of the compound of formula I to methanesulfonic acid is 1: (0.1-1), such as 1: (0.3-1), and further such as 1:0.3 or 1:1;
when the acid is *p*-toluenesulfonic acid, the molar ratio of the compound of formula I to p-toluenesulfonic acid is 1: (0.1-1), such as 1:0.9;
when the acid is cyclamic acid, the molar ratio of the compound of formula I to cyclamic acid is 1: (0.1-1), such as 1:0.9.

The fifth aspect of the present disclosure provides a crystal form **A** of the hydrochloride salt of the compound of formula I, which has an X-ray powder diffraction pattern expressed at a 2θ angle comprising diffraction peaks at the following 2θ angles: 8.83 ± 0.2°, 13.24 ± 0.2°, 24.25 ± 0.2°, and 18.31 ± 0.2°; the hydrochloride salt of the compound of formula I is as described above;

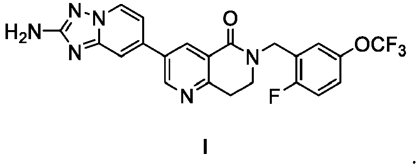

In a certain preferred embodiment, the crystal form **A** of the hydrochloride salt of the compound of formula I has a molar ratio of the compound of formula I to hydrochloric acid of 1:2.

In a certain preferred embodiment, the crystal form **A** of the hydrochloride salt of the compound of formula I has an X-ray powder diffraction pattern expressed at a 2θ angle further comprising diffraction peaks at one or more than one of the following 2θ angles: 26.66 ± 0.2°, 22.21 ± 0.2°, 4.39 ± 0.2°, 31.19 ± 0.2°, and 19.54 ± 0.2°.

Preferably, the crystal form **A** of the hydrochloride salt of the compound of formula I has an X-ray powder diffraction pattern expressed at a 2θ angle further comprising diffraction peaks at one or more than one of the following 2θ angles: 35.77 ± 0.2°, 19.71 ± 0.2°, 27.3 ± 0.2°, 32.11 ± 0.2°, 28.38 ± 0.2°, and 21.52 ± 0.2°.

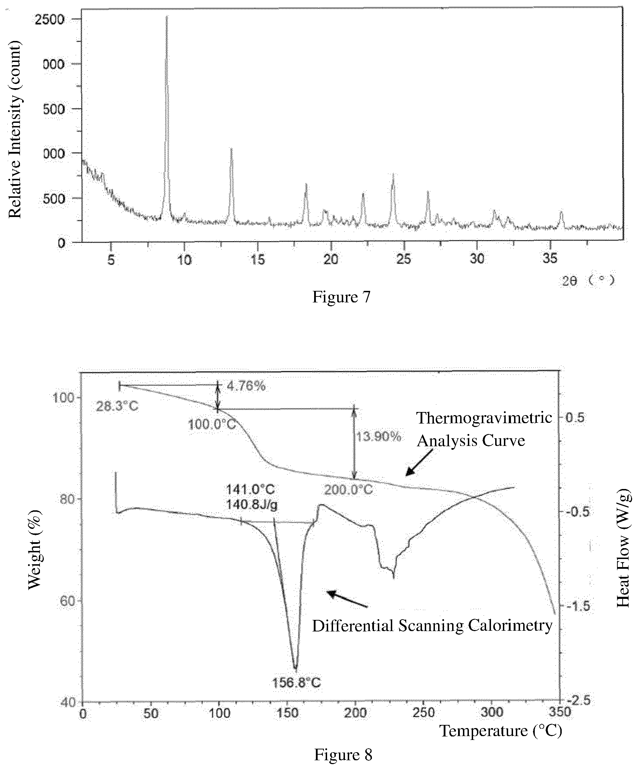

In a certain preferred embodiment, the crystal form **A** of the hydrochloride salt of the compound of formula I has an X-ray powder diffraction pattern expressed at a 2θ angle basically as shown in Figure 7.

In a certain preferred embodiment, the thermogravimetric analysis pattern of the crystal form **A** of the hydrochloride salt of the compound of formula I has a weight loss of 4% to 5.5% (such as 4.8%) when heated from an onset to 100 ± 5°C (the percentage of the weight loss is the percentage of the weight loss of the sample relative to the weight of the sample prior to this weight loss).

In a certain preferred embodiment, the crystal form **A** of the hydrochloride salt of the compound of formula I has a differential scanning calorimetry pattern showing an endothermic peak at 156.8 ± 5°C.

In a certain preferred embodiment, the crystal form **A** of the hydrochloride salt of the compound of formula I has a differential scanning calorimetry pattern and a thermogravimetric analysis pattern basically as shown in Figure 8.

The sixth aspect of the present disclosure provides a crystal form **A** of the citrate salt of the compound of formula I, which has an X-ray powder diffraction pattern expressed at a 2θ angle comprising diffraction peaks at the following 2θ angles: 17.87 ± 0.2°, 14.97 ± 0.2°, 17.45 ± 0.2°, and 17.01 ± 0.2°; the citrate salt of the compound of formula I is as described above;

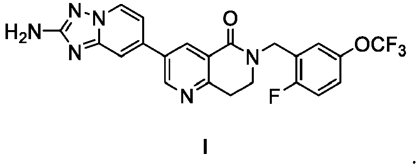

In a certain preferred embodiment, the crystal form **A** of the citrate salt of the compound of formula I has a molar ratio of the compound of formula I to citric acid of 1:1.

In a certain preferred embodiment, the crystal form **A** of the citrate salt of the compound of formula I has an X-ray powder diffraction pattern expressed at a 2θ angle further comprising diffraction peaks at one or more than one of the following 2θ angles: 4.45 ± 0.2°, 11.14 ± 0.2°, 19.08 ± 0.2°, 8.91 ± 0.2°, and 13.38 ± 0.2°.

Preferably, the crystal form **A** of the citrate salt of the compound of formula I has an X-ray powder diffraction pattern expressed at a 2θ angle further comprising diffraction peaks at one or more than one of the following 2θ angles: 21.23 ± 0.2°, 25.56 ± 0.2°, 21.00 ± 0.2°, 24.25 ± 0.2°, 28.48 ± 0.2°, and 29.9 ± 0.2°.

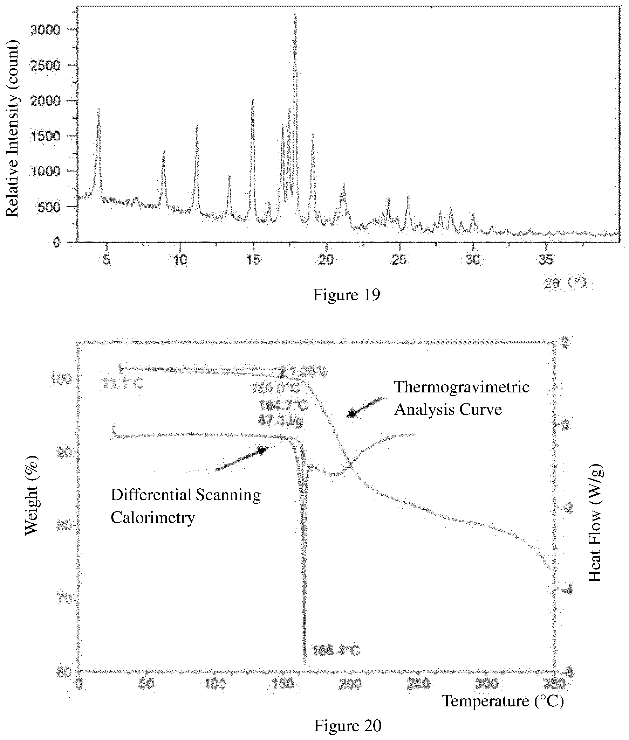

In a certain preferred embodiment, the crystal form **A** of the citrate salt of the compound of formula I has an X-ray powder diffraction pattern expressed at a 2θ angle basically as shown in Figure 19.

In a certain preferred embodiment, the thermogravimetric analysis pattern of the crystal form **A** of the citrate salt of the compound of formula I has a weight loss of 0.5% to 2% (such as 1.1%) when heated from an onset to 150 ± 5°C (the percentage of the weight loss is the percentage of the weight loss of the sample relative to the weight of the sample prior to this weight loss).

In a certain preferred embodiment, the crystal form **A** of the citrate salt of the compound of formula I has a differential scanning calorimetry pattern showing an endothermic peak at 166.4 ± 5°C.

In a certain preferred embodiment, the crystal form **A** of the citrate salt of the compound of formula I has a differential scanning calorimetry pattern and a thermogravimetric analysis pattern basically as shown in Figure 20.

The sixth aspect of the present disclosure provides a crystal form **A** of the maleate salt of the compound of formula I, which has an X-ray powder diffraction pattern expressed at a 2θ angle comprising diffraction peaks at the following 2θ angles: 16.47 ± 0.2°, 4.58 ± 0.2°, 10.97 ± 0.2°, and 22.91 ± 0.2°; the maleate salt of the compound of formula I is as described above;

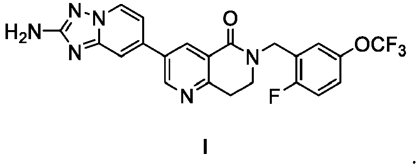

In a certain preferred embodiment, the crystal form **A** of the maleate salt of the compound of formula I has a molar ratio of the compound of formula I to maleic acid of 1: (0.5-1), such as 1:0.8.

In a certain preferred embodiment, the crystal form **A** of the maleate salt of the compound of formula I has an X-ray powder diffraction pattern expressed at a 2θ angle further comprising diffraction peaks at one or more than one of the following 2θ angles: 18.2 ± 0.2°, 19.87 ± 0.2°, 24.64 ± 0.2°, 22.35 ± 0.2°, and 20.26 ± 0.2°.

Preferably, the crystal form **A** of the maleate salt of the compound of formula I has an X-ray powder diffraction pattern expressed at a 2θ angle further comprising diffraction peaks at one or more than one of the following 2θ angles: 19.56 ± 0.2°, 28.43 ± 0.2°, 26.52 ± 0.2°, 15.04 ± 0.2°, 27.52 ± 0.2°, and 18.86 ± 0.2°.

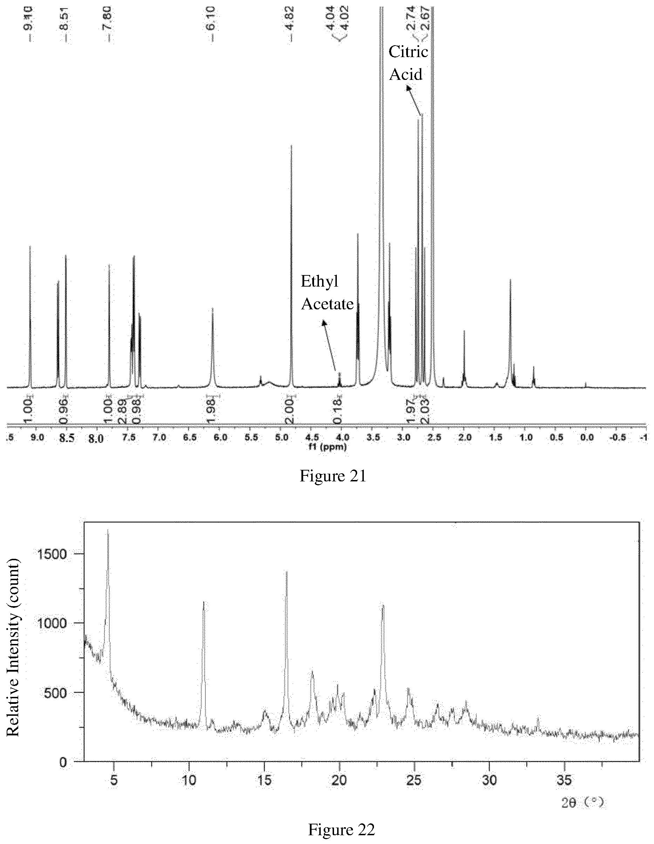

In a certain preferred embodiment, the crystal form **A** of the maleate salt of the compound of formula I has an X-ray powder diffraction pattern expressed at a 2θ angle basically as shown in Figure 22.

In a certain preferred embodiment, the crystal form **A** of the maleate salt of the compound of formula I has a thermogravimetric analysis pattern with a weight loss of 3%-4% (such as 3.3%) at 150 ± 5°C and a weight loss of 10%-15% (such as 13.6%) when heated from 150°C to 250°C.

In a certain preferred embodiment, the crystal form **A** of the maleate salt of the compound of formula I has a differential scanning calorimetry pattern showing an endothermic peak at 176.0 ± 5°C and/or 210.7 ± 5°C.

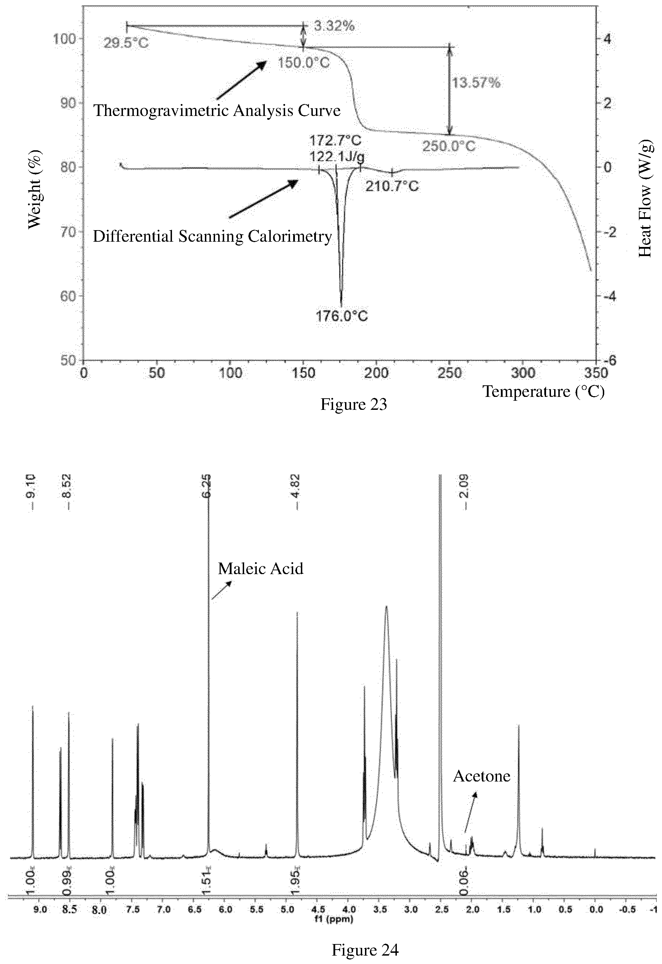

In a certain preferred embodiment, the crystal form **A** of the maleate salt of the compound of formula I has a differential scanning calorimetry pattern and a thermogravimetric analysis pattern basically as shown in Figure 23.

The seventh aspect of the present disclosure provides a crystal form **A** of the fumarate salt of the compound of formula I, which has an X-ray powder diffraction pattern expressed at a 2θ angle comprising diffraction peaks at the following 2θ angles: 28.83 ± 0.2°, 5.03 ± 0.2°, 16.15 ± 0.2°, and 13.11 ± 0.2°; the fumarate salt of the compound of formula I is as described above;

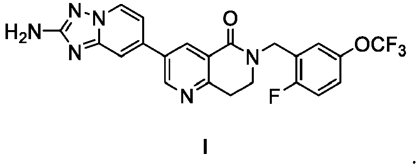

In a certain preferred embodiment, the crystal form **A** of the fumarate salt of the compound of formula I has a molar ratio of the compound of formula I to fumaric acid of 1: (0.5-1), such as 1:0.9.

In a certain preferred embodiment, the crystal form **A** of the fumarate salt of the compound of formula I has an X-ray powder diffraction pattern expressed at a 2θ angle further comprising diffraction peaks at one or more than one of the following 2θ angles: 22.86 ± 0.2°, 12.39 ± 0.2°, 29.44 ± 0.2°, 17.78 ± 0.2°, and 9.69 ± 0.2°.

Preferably, the crystal form **A** of the fumarate salt of the compound of formula I has an X-ray powder diffraction pattern expressed at a 2θ angle further comprising diffraction peaks at one or more than one of the following 2θ angles: 19.82 ± 0.2°, 10.73 ± 0.2°, 3.25 ± 0.2°, 17.09 ± 0.2°, 6.54 ± 0.2°, and 11.22 ± 0.2°.

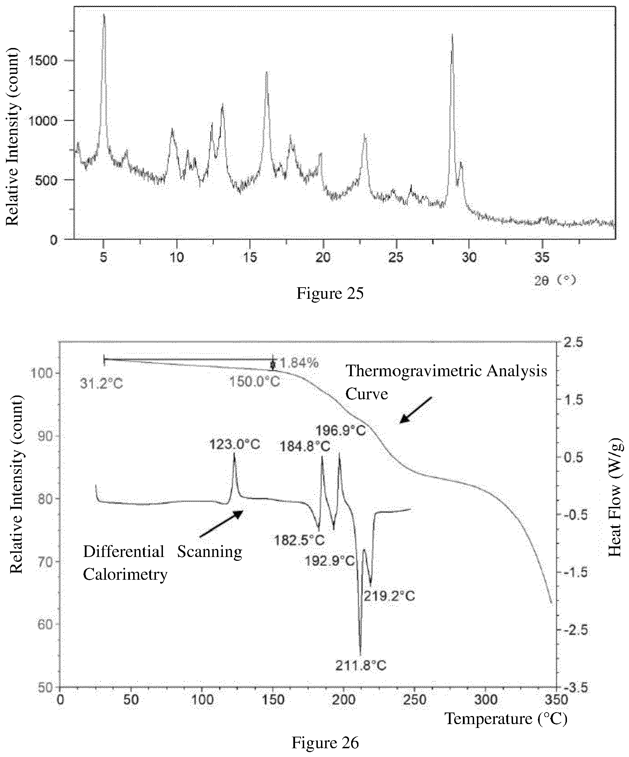

In a certain preferred embodiment, the crystal form **A** of the fumarate salt of the compound of formula I has an X-ray powder diffraction pattern expressed at a 2θ angle basically as shown in Figure 25.

In a certain preferred embodiment, the thermogravimetric analysis pattern of the crystal form **A** of the fumarate salt of the compound of formula I has a weight loss of 1% to 3% (such as 1.8%) when heated from an onset to 150 ± 5°C (the percentage of the weight loss is the percentage of the weight loss of the sample relative to the weight of the sample prior to this weight loss).

In a certain preferred embodiment, the crystal form **A** of the fumarate salt of the compound of formula I has a differential scanning calorimetry pattern showing an endothermic peak at one or more than one of 182.5 ± 5°C, 192.9 ± 5°C, 211.8 ± 5°C, and 219.2 ± 5°C, and/or, an exothermic peak at one or more than one of 123.0 ± 5°C, 184.8 ± 5°C, and 196.9 ± 5°C.

In a certain preferred embodiment, the crystal form **A** of the fumarate salt of the compound of formula I has a differential scanning calorimetry pattern and a thermogravimetric analysis pattern basically as shown in Figure 26.

The eighth aspect of the present disclosure provides a crystal form A of the methanesulfonate salt of the compound of formula I, which has an X-ray powder diffraction pattern expressed at a 2θ angle comprising diffraction peaks at the following 2θ angles: 5.76 ± 0.2°, 14.75 ± 0.2°, 5.11 ± 0.2°, and 17.54 ± 0.2°; the methanesulfonate salt of the compound of formula I is as described above;

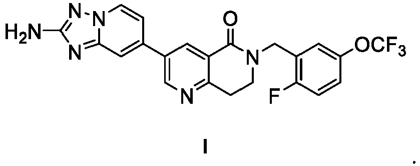

In a certain preferred embodiment, the crystal form **A** of the methanesulfonate salt of the compound of formula I has a molar ratio of the compound of formula I to methanesulfonic acid of 1: (0.1-1), such as 1:1.

In a certain preferred embodiment, the crystal form **A** of the methanesulfonate salt of the compound of formula I has an X-ray powder diffraction pattern expressed at a 2θ angle further comprising diffraction peaks at one or more than one of the following 2θ angles: 20.29 ± 0.2°, 20.49 ± 0.2°, 16.84 ± 0.2°, 7.51 ± 0.2°, and 16.51 ± 0.2°.

Preferably, the crystal form **A** of the methanesulfonate salt of the compound of formula I has an X-ray powder diffraction pattern expressed at a 2θ angle further comprising diffraction peaks at one or more than one of the following 2θ angles: 21.72 ± 0.2°, 19.60 ± 0.2°, 15.51 ± 0.2°, 18.43 ± 0.2°, 15.86 ± 0.2°, and 12.84 ± 0.2°.

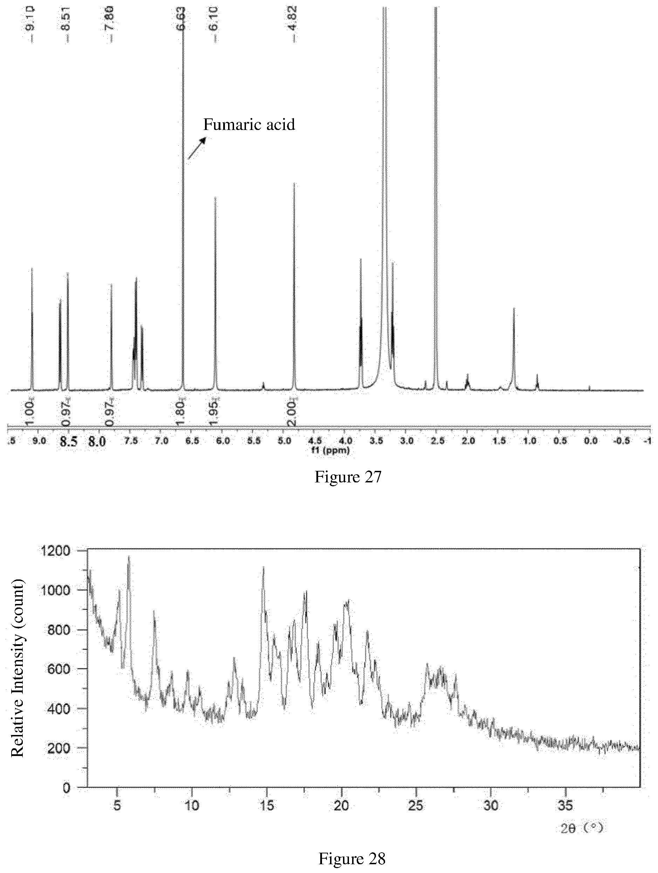

In a certain preferred embodiment, the crystal form **A** of the methanesulfonate salt of the compound of formula I has an X-ray powder diffraction pattern expressed at a 2θ angle basically as shown in Figure 28.

In a certain preferred embodiment, the thermogravimetric analysis pattern of the crystal form **A** of the methanesulfonate salt of the compound of formula I has a weight loss of 2% to 4% (such as 2.9%) when heated from an onset to 110 ± 5°C (the percentage of the weight loss is the percentage of the weight loss of the sample relative to the weight of the sample prior to this weight loss).

In a certain preferred embodiment, the crystal form **A** of the methanesulfonate salt of the compound of formula I has a differential scanning calorimetry pattern showing an endothermic peak at 92.5 ± 5°C and/or 138.6 ± 5°C.

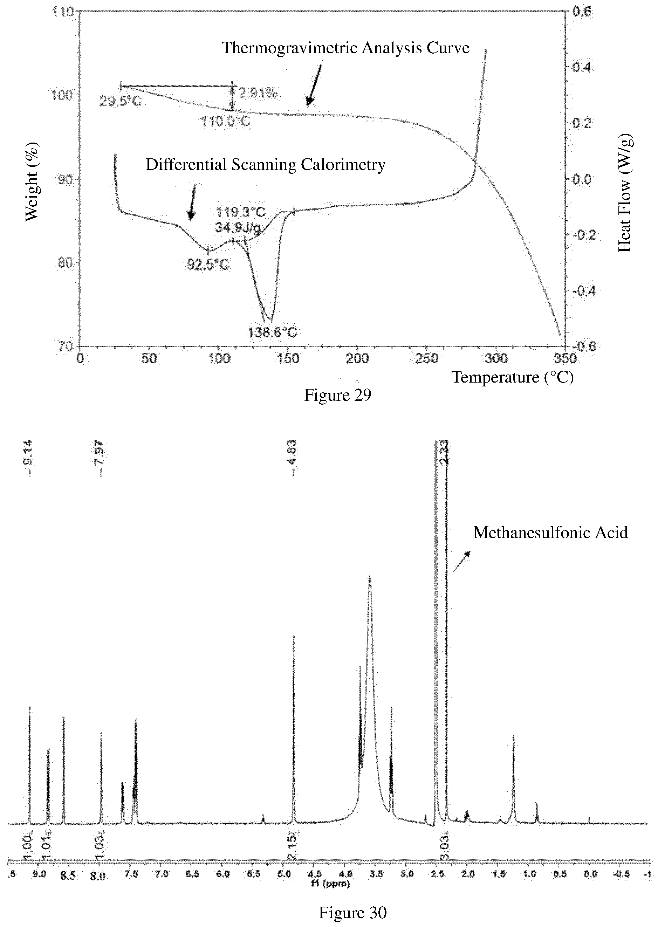

In a certain preferred embodiment, the crystal form **A** of the methanesulfonate salt of the compound of formula I has a differential scanning calorimetry pattern and a thermogravimetric analysis pattern basically as shown in Figure 29.

The ninth aspect of the present disclosure provides a crystal form **B** of the methanesulfonate salt of the compound of formula I, which has an X-ray powder diffraction pattern expressed at a 2θ angle comprising diffraction peaks at the following 2θ angles: 18.83 ± 0.2°, 16.51 ± 0.2°, 25.51 ± 0.2°, and 10.98 ± 0.2°; the methanesulfonate salt of the compound of formula I is as described above;

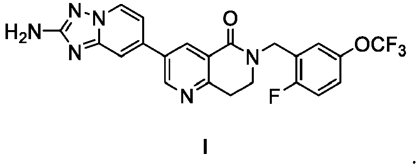

In a certain preferred embodiment, the crystal form **B** of the methanesulfonate salt of the compound of formula I has a molar ratio of the compound of formula I to methanesulfonic acid of 1: (0.1-1), such as 1:1.

In a certain preferred embodiment, the crystal form **B** of the methanesulfonate salt of the compound of formula I has an X-ray powder diffraction pattern expressed at a 2θ angle further comprising diffraction peaks at one or more than one of the following 2θ angles: 22.32 ± 0.2°, 5.49 ± 0.2°, 20.34 ± 0.2°, 26.34 ± 0.2°, and 16.94 ± 0.2°.

Preferably, the crystal form **B** of the methanesulfonate salt of the compound of formula I has an X-ray powder diffraction pattern expressed at a 2θ angle further comprising diffraction peaks at one or more than one of the following 2θ angles: 19.48 ± 0.2°, 21.35 ± 0.2°, 15.06 ± 0.2°, 19.93 ± 0.2°, 12.75 ± 0.2°, and 18.05 ± 0.2°.

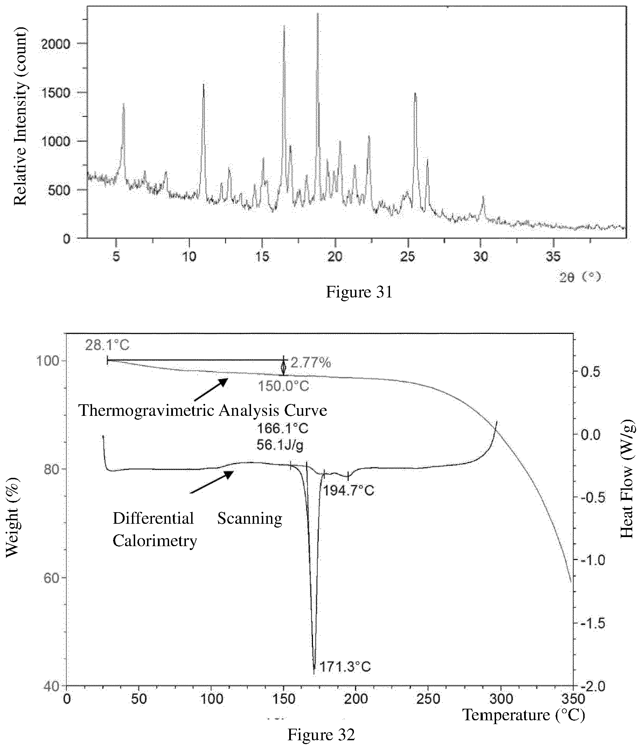

In a certain preferred embodiment, the crystal form **B** of the methanesulfonate salt of the compound of formula I has an X-ray powder diffraction pattern expressed at a 2θ angle basically as shown in Figure 31.

In a certain preferred embodiment, the thermogravimetric analysis pattern of the crystal form **B** of the methanesulfonate salt of the compound of formula I has a weight loss of 2% to 4% (such as 2.8%) when heated from an onset to 150 ± 5°C (the percentage of the weight loss is the percentage of the weight loss of the sample relative to the weight of the sample prior to this weight loss).

In a certain preferred embodiment, the crystal form **B** of the methanesulfonate salt of the compound of formula I has a differential scanning calorimetry pattern showing an endothermic peak at 171.3 ± 5°C and/or 194.7 ± 5°C.

In a certain preferred embodiment, the crystal form **B** of the methanesulfonate salt of the compound of formula I has a differential scanning calorimetry pattern and a thermogravimetric analysis pattern basically as shown in Figure 32.

The tenth aspect of the present disclosure provides a crystal form **C** of the methanesulfonate salt of the compound of formula I, which has an X-ray powder diffraction pattern expressed at a 2θ angle comprising diffraction peaks at the following 2θ angles: 19.57 ± 0.2°, 19.98 ± 0.2°, 16.50 ± 0.2°, and 18.20 ± 0.2°;

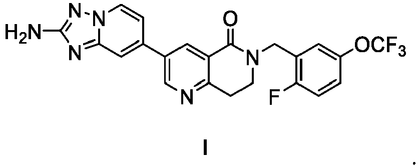

In a certain preferred embodiment, the crystal form **C** of the methanesulfonate salt of the compound of formula I has a molar ratio of the compound of formula I to methanesulfonic acid of 1: (0.1-1), such as 1:0.3.

In a certain preferred embodiment, the crystal form **C** of the methanesulfonate salt of the compound of formula I has an X-ray powder diffraction pattern expressed at a 2θ angle further comprising diffraction peaks at one or more than one of the following 2θ angles: 20.26 ± 0.2°, 23.30 ± 0.2°, 29.52 ± 0.2°, 10.99 ± 0.2°, and 26.94 ± 0.2°.

Preferably, the crystal form **C** of the methanesulfonate salt of the compound of formula I has an X-ray powder diffraction pattern expressed at a 2θ angle further comprising diffraction peaks at one or more than one of the following 2θ angles: 22.36 ± 0.2°, 20.86 ± 0.2°, 24.62 ± 0.2°, 24.92 ± 0.2°, 27.41 ± 0.2°, and 18.45 ± 0.2°.

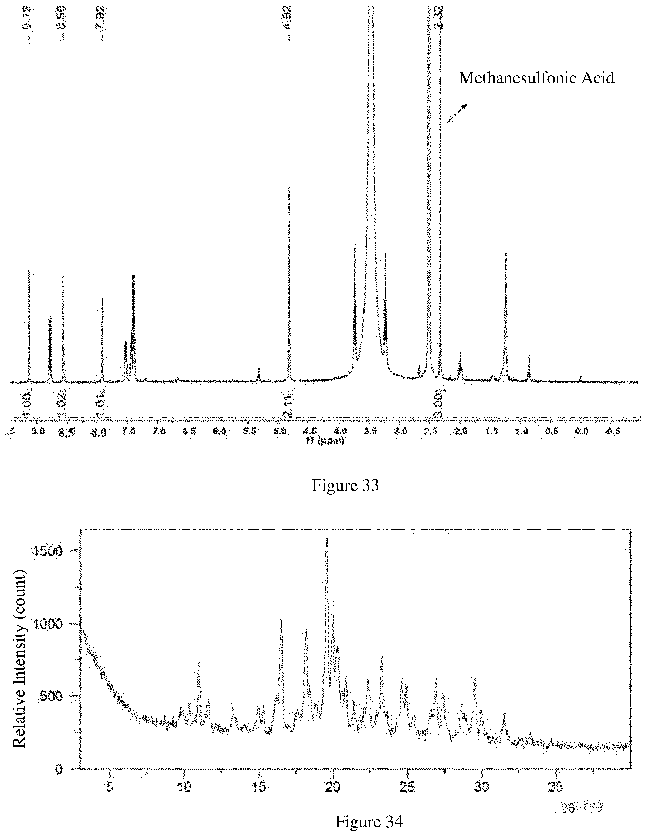

In a certain preferred embodiment, the crystal form **C** of the methanesulfonate salt of the compound of formula I has an X-ray powder diffraction pattern expressed at a 2θ angle basically as shown in Figure 34.

In a certain preferred embodiment, the thermogravimetric analysis pattern of the crystal form **C** of the methanesulfonate salt of the compound of formula I has a weight loss of 4% to 6% (such as 4.9%) when heated from an onset to 150 ± 5°C (the percentage of the weight loss is the percentage of the weight loss of the sample relative to the weight of the sample prior to this weight loss).

In a certain preferred embodiment, the crystal form **C** of the methanesulfonate salt of the compound of formula I has a differential scanning calorimetry pattern showing an endothermic peak at one or more than one of 108.2 ± 5°C, 181.9 ± 5°C, and 233.2 ± 5°C.

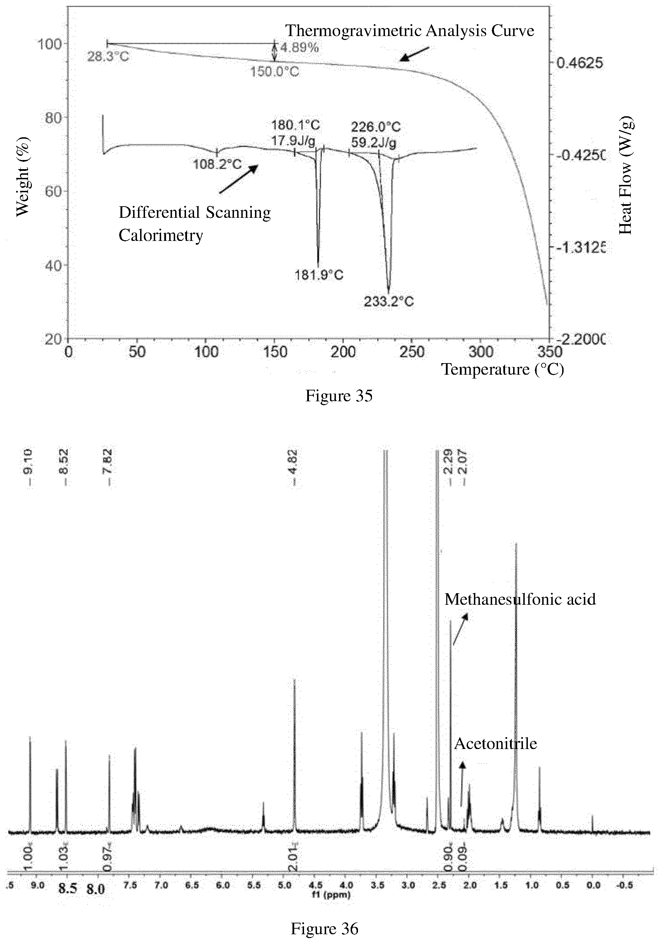

In a certain preferred embodiment, the crystal form **C** of the methanesulfonate salt of the compound of formula I has a differential scanning calorimetry pattern and a thermogravimetric analysis pattern basically as shown in Figure 35.

The eleventh aspect of the present disclosure provides a crystal form **A** of the *p-*toluenesulfonate salt of the compound of formula I, which has an X-ray powder diffraction pattern expressed at a 2θ angle comprising diffraction peaks at the following 2θ angles: 5.80 ± 0.2°, 19.70 ± 0.2°, 22.36 ± 0.2°, and 11.57 ± 0.2°; the *p*-toluenesulfonate salt of the compound of formula I is as described above;

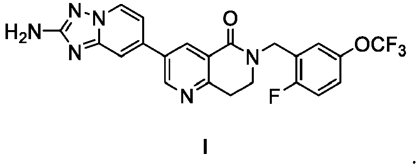

In a certain preferred embodiment, the crystal form **A** of the *p*-toluenesulfonate salt of the compound of formula I has a molar ratio of the compound of formula I to *p*-toluenesulfonic acid of 1: (0.1-1), such as 1:0.9.

In a certain preferred embodiment, the crystal form **A** of the *p*-toluenesulfonate salt of the compound of formula I has an X-ray powder diffraction pattern expressed at a 2θ angle further comprising diffraction peaks at one or more than one of the following 2θ angles: 27.22 ± 0.2°, 14.81 ± 0.2°, 16.83 ± 0.2°, 27.91 ± 0.2°, and 15.18 ± 0.2°.

Preferably, the crystal form **A** of the *p*-toluenesulfonate salt of the compound of formula I has an X-ray powder diffraction pattern expressed at a 2θ angle further comprising diffraction peaks at one or more than one of the following 2θ angles: 19.21 ± 0.2°, 18.16 ± 0.2°, 13.07 ± 0.2°, 30.66 ± 0.2°, 32.47 ± 0.2°, and 18.47 ± 0.2°.

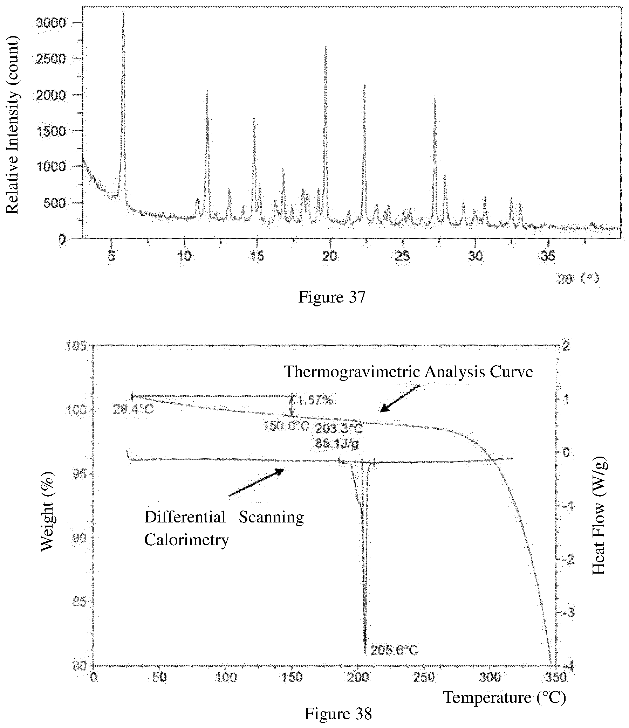

In a certain preferred embodiment, the crystal form **A** of the *p*-toluenesulfonate salt of the compound of formula I has an X-ray powder diffraction pattern expressed at a 2θ angle basically as shown in Figure 37.

In a certain preferred embodiment, the thermogravimetric analysis pattern of the crystal form **A** of the *p*-toluenesulfonate salt of the compound of formula I has a weight loss of 1% to 3% (such as 1.6%) when heated from an onset to 150 ± 5°C (the percentage of the weight loss is the percentage of the weight loss of the sample relative to the weight of the sample prior to this weight loss).

In a certain preferred embodiment, the crystal form **A** of the *p*-toluenesulfonate salt of the compound of formula I has a differential scanning calorimetry pattern showing an endothermic peak at 205.6 ± 5°C.

In a certain preferred embodiment, the crystal form **A** of the *p*-toluenesulfonate salt of the compound of formula I has a differential scanning calorimetry pattern and a thermogravimetric analysis pattern basically as shown in Figure 38.

The twelfth aspect of the present disclosure provides a crystal form **A** of the cyclamate salt of the compound of formula I, which has an X-ray powder diffraction pattern expressed at a 2θ angle comprising diffraction peaks at the following 2θ angles: 6.41 ± 0.2°, 16.47 ± 0.2°, 18.57 ± 0.2°, and 10.95 ± 0.2°; the cyclamate salt of the compound of formula I is as described above;

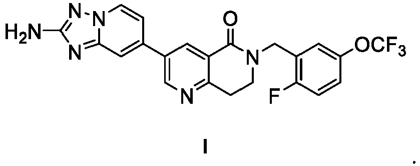

In a certain preferred embodiment, the crystal form **A** of the cyclamate salt of the compound of formula I has a molar ratio of the compound of formula I to cyclamic acid of 1: (0.1-1), such as 1:0.9.

In a certain preferred embodiment, the crystal form **A** of the cyclamate salt of the compound of formula I has an X-ray powder diffraction pattern expressed at a 2θ angle further comprising diffraction peaks at one or more than one of the following 2θ angles: 18.16 ± 0.2°, 19.58 ± 0.2°, 20.24 ± 0.2°, 22.33 ± 0.2°, and 23.27 ± 0.2°.

In a certain preferred embodiment, the crystal form **A** of the cyclamate salt of the compound of formula I has an X-ray powder diffraction pattern expressed at a 2θ angle further comprising diffraction peaks at one or more than one of the following 2θ angles: 24.63 ± 0.2°, 26.94 ± 0.2°, 27.39 ± 0.2°, 21.39 ± 0.2°, 20.83 ± 0.2°, and 24.93 ± 0.2°.

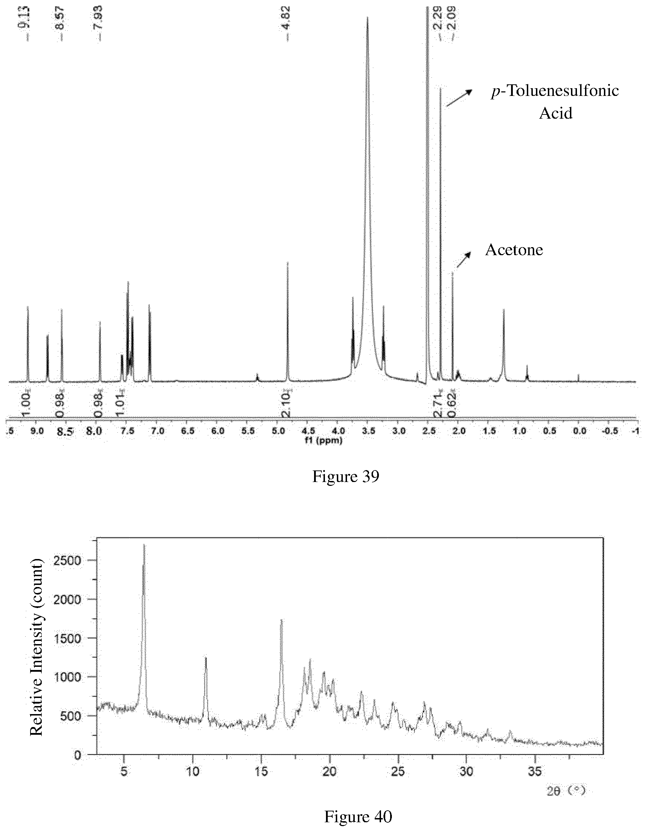

In a certain preferred embodiment, the crystal form **A** of the cyclamate salt of the compound of formula I has an X-ray powder diffraction pattern expressed at a 2θ angle basically as shown in Figure 40.

In a certain preferred embodiment, the thermogravimetric analysis pattern of the crystal form **A** of the cyclamate salt of the compound of formula I has a weight loss of 0.1% to 2% (such as 0.7%) when heated from an onset to 150 ± 5°C (the percentage of the weight loss is the percentage of the weight loss of the sample relative to the weight of the sample prior to this weight loss).

In a certain preferred embodiment, the crystal form **A** of the cyclamate salt of the compound of formula I has a differential scanning calorimetry pattern showing an endothermic peak at 202.3 ± 5°C and/or 231.3 ± 5°C; and/or, an exothermic peak at 118.2 ± 5°C and/or 205.4 ± 5°C.

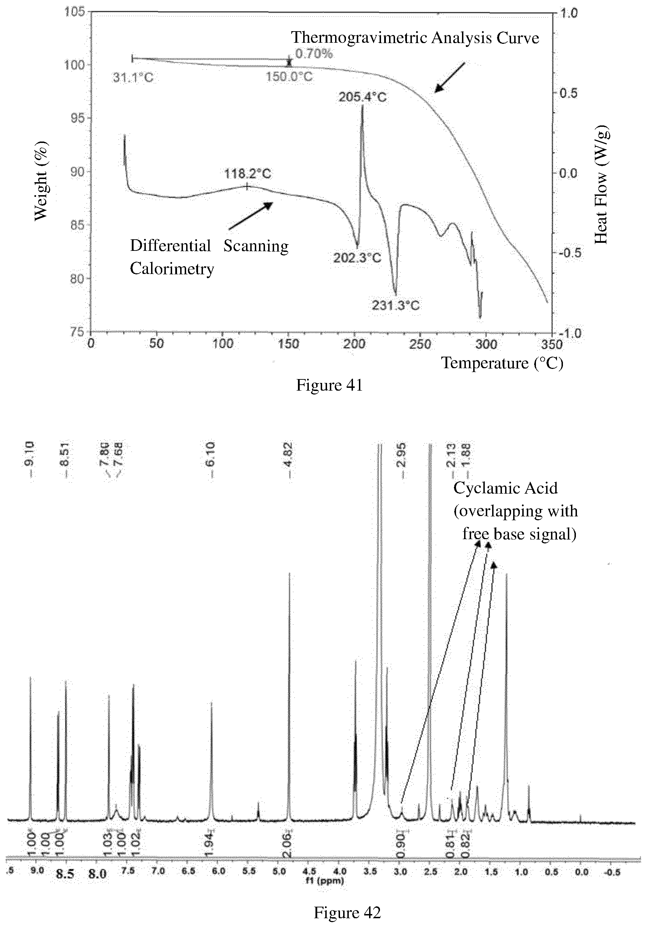

In a certain preferred embodiment, the crystal form **A** of the cyclamate salt of the compound of formula I has a differential scanning calorimetry pattern and a thermogravimetric analysis pattern basically as shown in Figure 41.

The thirteenth aspect of the present disclosure provides a crystal form **A** of the sulfate salt of the compound of formula I, which has an X-ray powder diffraction pattern expressed at a 2θ angle comprising diffraction peaks at the following 2θ angles: 14.60 ± 0.2°, 11.67 ± 0.2°, 21.09 ± 0.2°, and 13.32 ± 0.2°; the sulfate salt of the compound of formula I is as described above;

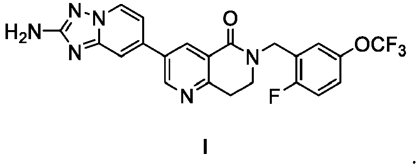

In a certain preferred embodiment, the crystal form **A** of the sulfate salt of the compound of formula I has a molar ratio of the compound of formula I to sulfuric acid of 1: (1-2), such as 1: (1-1.3), 1: (1-1.1), or 1:1.

In a certain preferred embodiment, the crystal form **A** of the sulfate salt of the compound of formula I has an X-ray powder diffraction pattern expressed at a 2θ angle further comprising diffraction peaks at one or more than one of the following 2θ angles: 17.38 ± 0.2°, 21.65 ± 0.2°, 26.08 ± 0.2°, 23.45 ± 0.2°, and 26.44 ± 0.2°.

Preferably, the crystal form **A** of the sulfate salt of the compound of formula I has an X-ray powder diffraction pattern expressed at a 2θ angle further comprising diffraction peaks at one or more than one of the following 2θ angles: 15.76 ± 0.2°, 8.75 ± 0.2°, 37.29 ± 0.2°, 24.42 ± 0.2°, 22.08 ± 0.2°, and 29.39 ± 0.2°.

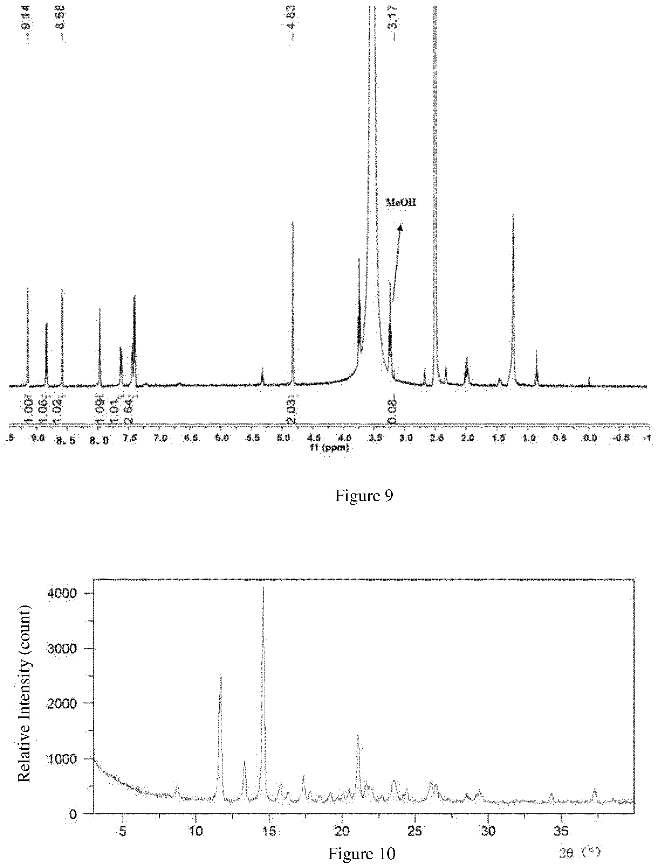

In a certain preferred embodiment, the crystal form **A** of the sulfate salt of the compound of formula I has an X-ray powder diffraction pattern expressed at a 2θ angle basically as shown in Figure 10.

In a certain preferred embodiment, the thermogravimetric analysis pattern of the crystal form **A** of the sulfate salt of the compound of formula I has a weight loss of 2% to 4% (such as 3.4%) when heated from an onset to 175 ± 5°C (the percentage of the weight loss is the percentage of the weight loss of the sample relative to the weight of the sample prior to this weight loss).

In a certain preferred embodiment, the crystal form **A** of the sulfate salt of the compound of formula I has a differential scanning calorimetry pattern showing three endothermic peaks at 70.6 ± 5°C, 123.3 ± 5°C, and 205.1 ± 5°C.

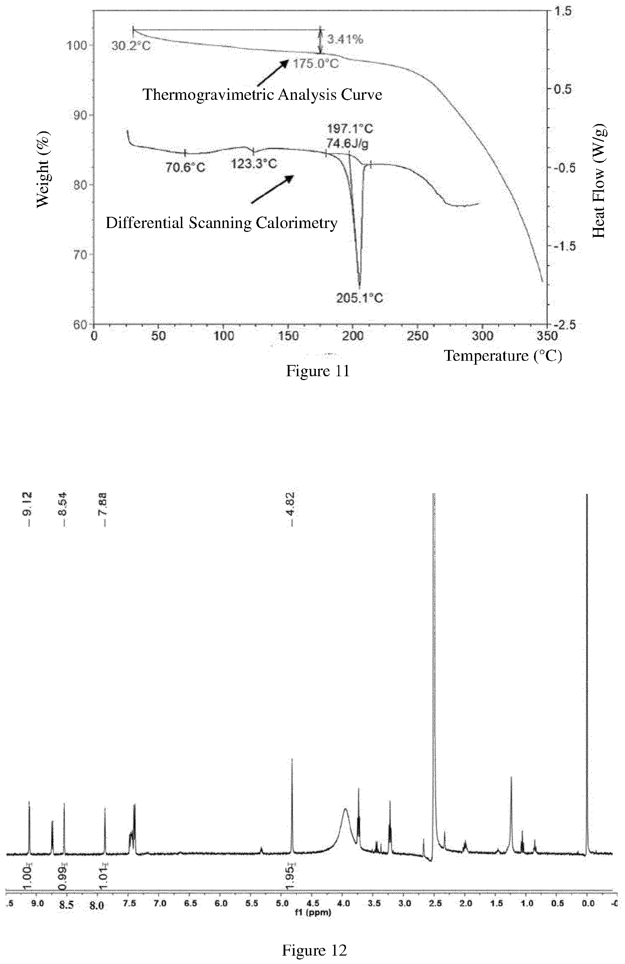

In a certain preferred embodiment, the crystal form **A** of the sulfate salt of the compound of formula I has a differential scanning calorimetry pattern and a thermogravimetric analysis pattern basically as shown in Figure 11.

The fourteenth aspect of the present disclosure provides a crystal form **D** of the sulfate salt of the compound of formula I, which has an X-ray powder diffraction pattern expressed at a 2θ angle comprising diffraction peaks at the following 2θ angles: 14.60 ± 0.2°, 25.31 ± 0.2°, 19.85 ± 0.2°, and 20.07 ± 0.2°; the sulfate salt of the compound of formula I is as described above;

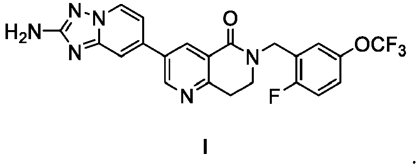

In a certain preferred embodiment, the crystal form **D** of the sulfate salt of the compound of formula I has a molar ratio of the compound of formula I to sulfuric acid of 1: (1-2), such as 1: (1-1.3), 1: (1-1.1), 1:1, or 1:1.1.

In a certain preferred embodiment, the crystal form **D** of the sulfate salt of the compound of formula I has an X-ray powder diffraction pattern expressed at a 2θ angle further comprising diffraction peaks at one or more than one of the following 2θ angles: 21.96 ± 0.2°, 19.31 ± 0.2°, 18.15 ± 0.2°, 13.18 ± 0.2°, and 6.59 ± 0.2°.

Preferably, the crystal form **D** of the sulfate salt of the compound of formula I has an X-ray powder diffraction pattern expressed at a 2θ angle further comprising diffraction peaks at one or more than one of the following 2θ angles: 15.02 ± 0.2°, 31.39 ± 0.2°, 22.14 ± 0.2°, 24.63 ± 0.2°, 25.63 ± 0.2°, and 9.83 ± 0.2°.

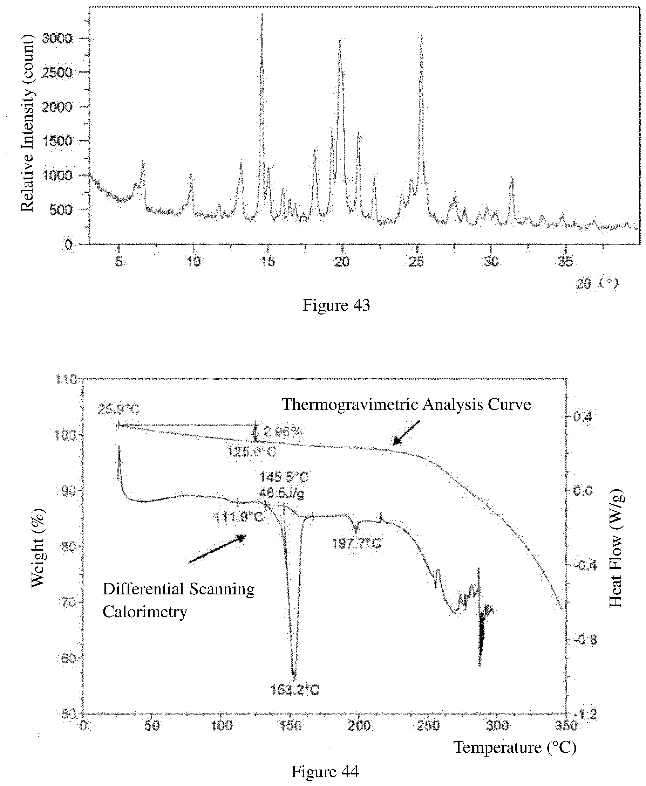

In a certain preferred embodiment, the crystal form **D** of the sulfate salt of the compound of formula I has an X-ray powder diffraction pattern expressed at a 2θ angle basically as shown in Figure 43.

In a certain preferred embodiment, the thermogravimetric analysis pattern of the crystal form **D** of the sulfate salt of the compound of formula I has a weight loss of 1% to 4% (such as 3%) when heated from an onset to 125 ± 5°C (the percentage of the weight loss is the percentage of the weight loss of the sample relative to the weight of the sample prior to this weight loss).

In a certain preferred embodiment, the crystal form **D** of the sulfate salt of the compound of formula I has a differential scanning calorimetry pattern showing three endothermic peaks at 111.9 ± 5°C, 153.2 ± 5°C, and 197.7 ± 5°C.

In a certain preferred embodiment, the crystal form **D** of the sulfate salt of the compound of formula I has a differential scanning calorimetry pattern and a thermogravimetric analysis pattern basically as shown in Figure 44.

The fifteenth aspect of the present disclosure provides a crystal form **I** of the sulfate salt of the compound of formula I, which has an X-ray powder diffraction pattern expressed at a 2θ angle comprising diffraction peaks at the following 2θ angles: 12.92 ± 0.2°, 26.90 ± 0.2°, 15.86 ± 0.2°, and 23.02 ± 0.2°; the sulfate salt of the compound of formula I is as described above;

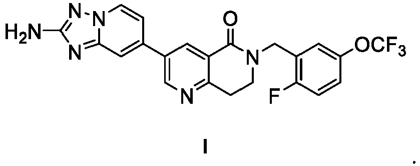

In a certain preferred embodiment, the crystal form **I** of the sulfate salt of the compound of formula I has a molar ratio of the compound of formula I to sulfuric acid of 1: (1-2), such as 1: (1-1.3), 1: (1-1.1), or 1:1.

In a certain preferred embodiment, the crystal form **I** of the sulfate salt of the compound of formula I has an X-ray powder diffraction pattern expressed at a 2θ angle further comprising diffraction peaks at one or more than one of the following 2θ angles: 21.09 ± 0.2°, 14.99 ± 0.2°, 17.26 ± 0.2°, 19.34 ± 0.2°, and 24.61 ± 0.2°.

Preferably, the crystal form **I** of the sulfate salt of the compound of formula I has an X-ray powder diffraction pattern expressed at a 2θ angle further comprising diffraction peaks at one or more than one of the following 2θ angles: 20.79 ± 0.2°, 21.75 ± 0.2°, 18.31 ± 0.2°, 18.47 ± 0.2°, 21.61 ± 0.2° and 19.75 ± 0.2°.

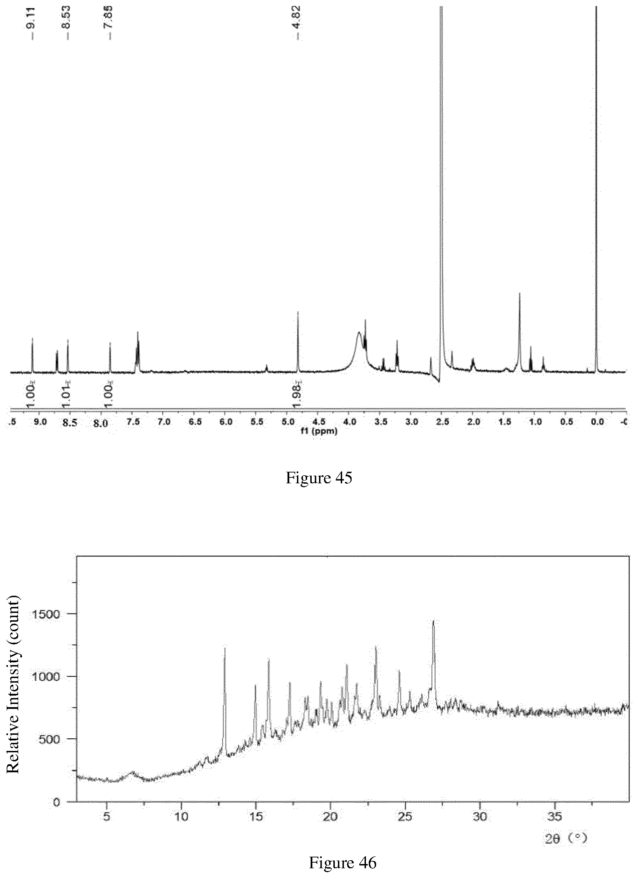

In a certain preferred embodiment, the crystal form **I** of the sulfate salt of the compound of formula I has an X-ray powder diffraction pattern expressed at a 2θ angle basically as shown in Figure 46.

The sixteenth aspect of the present disclosure provides a crystal form **B** of the sulfate salt of the compound of formula I, which has an X-ray powder diffraction pattern expressed at a 2θ angle comprising diffraction peaks at the following 2θ angles: 25.15 ± 0.2°, 18.71 ± 0.2°, 3.13 ± 0.2°, and 23.28 ± 0.2°; the sulfate salt of the compound of formula I is as described above;

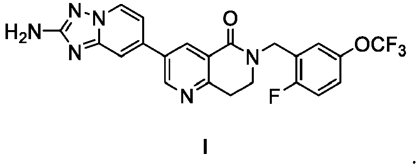

In a certain preferred embodiment, the crystal form **B** of the sulfate salt of the compound of formula I has a molar ratio of the compound of formula I to sulfuric acid of 1: (1-2), such as 1: (1-1.3), 1: (1-1.1), 1:1, or 1:1.1.

In a certain preferred embodiment, the crystal form **B** of the sulfate salt of the compound of formula I has an X-ray powder diffraction pattern expressed at a 2θ angle comprising diffraction peaks at one or more than one of the following 2θ angles: 20.27 ± 0.2°, 24.82 ± 0.2°, 15.17 ± 0.2°, 14.82 ± 0.2°, and 23.67 ± 0.2°.

Preferably, the crystal form **B** of the sulfate salt of the compound of formula I has an X-ray powder diffraction pattern expressed at a 2θ angle comprising diffraction peaks at one or more than one of the following 2θ angles: 21.03 ± 0.2°, 19.05 ± 0.2°, 18.34 ± 0.2°, 12.20 ± 0.2°, 24.18 ± 0.2°, and 17.21 ± 0.2°.

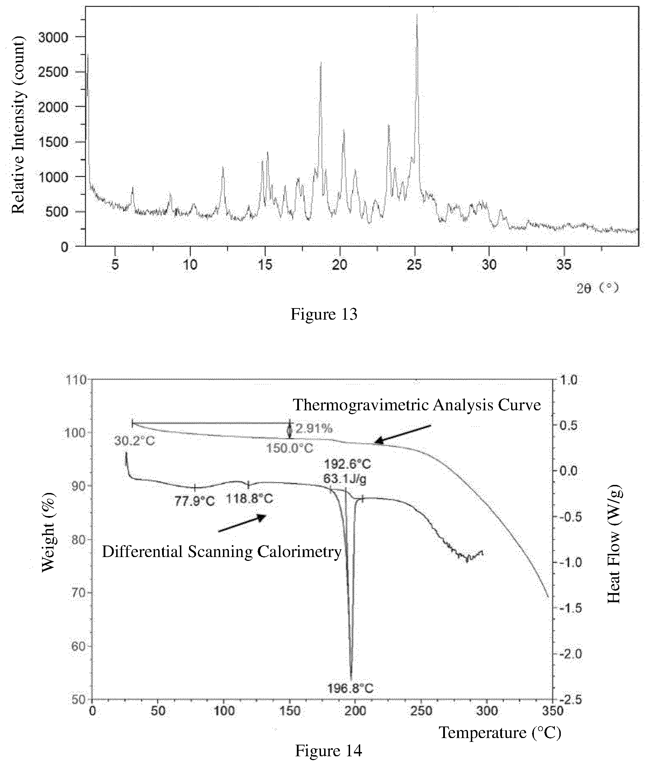

In a certain preferred embodiment, the crystal form **B** of the sulfate salt of the compound of formula I has an X-ray powder diffraction pattern expressed at a 2θ angle basically as shown in Figure 13.

In a certain preferred embodiment, the thermogravimetric analysis pattern of the crystal form **B** of the sulfate salt of the compound of formula I has a weight loss of 2% to 4% (such as 2.9%) when heated from an onset to 150 ± 5°C (the percentage of the weight loss is the percentage of the weight loss of the sample relative to the weight of the sample prior to this weight loss).

In a certain preferred embodiment, the crystal form **B** of the sulfate salt of the compound of formula I has a differential scanning calorimetry pattern showing three endothermic peaks at 77.9 ± 5°C, 118.8 ± 5°C, and 196.8 ± 5°C.

In a certain preferred embodiment, the crystal form **B** of the sulfate salt of the compound of formula I has a differential scanning calorimetry pattern and a thermogravimetric analysis pattern basically as shown in Figure 14.

The seventeenth aspect of the present disclosure provides a crystal form **C** of the sulfate salt of the compound of formula I, which has an X-ray powder diffraction pattern expressed at a 2θ angle comprising diffraction peaks at the following 2θ angles: 5.55 ± 0.2°, 14.72 ± 0.2°, 16.75 ± 0.2°, and 27.35 ± 0.2°; the sulfate salt of the compound of formula I is as described above;

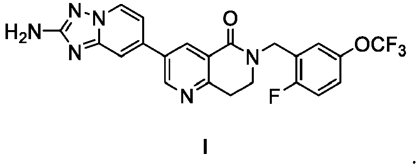

In a certain preferred embodiment, in the crystal form **C** of the sulfate salt of the compound of formula I, the sulfate salt of the compound of formula I is a hydrate, the molar ratio of the compound of formula I to water is 1: (1-2), such as 1: (1-1.3), also such as 1: (1-1.1), and further such as 1:1.

In a certain preferred embodiment, the crystal form **C** of the sulfate salt of the compound of formula I has a molar ratio of the compound of formula I to sulfuric acid of 1: (1-2), such as 1: (1-1.3), 1: (1-1.1) or 1:1.

In a certain preferred embodiment, in the crystal form **C** of the sulfate salt of the compound of formula I, the sulfate salt of the compound of formula I is a monohydrate, the molar ratio of the compound of formula I to the sulfate salt is 1:1.

In a certain preferred embodiment, the crystal form **C** of the sulfate salt of the compound of formula I has an X-ray powder diffraction pattern expressed at a 2θ angle further comprising diffraction peaks at one or more than one of the following 2θ angles: 22.92 ± 0.2°, 20.90 ± 0.2°, 21.13 ± 0.2°, 19.63 ± 0.2°, and 20.37 ± 0.2°.

Preferably, the crystal form **C** of the sulfate salt of the compound of formula I has an X-ray powder diffraction pattern expressed at a 2θ angle further comprising diffraction peaks at one or more than one of the following 2θ angles: 20.72 ± 0.2°, 12.52 ± 0.2°, 19.45 ± 0.2°, 19.96 ± 0.2°, 25.16 ± 0.2°, and 12.75 ± 0.2°.

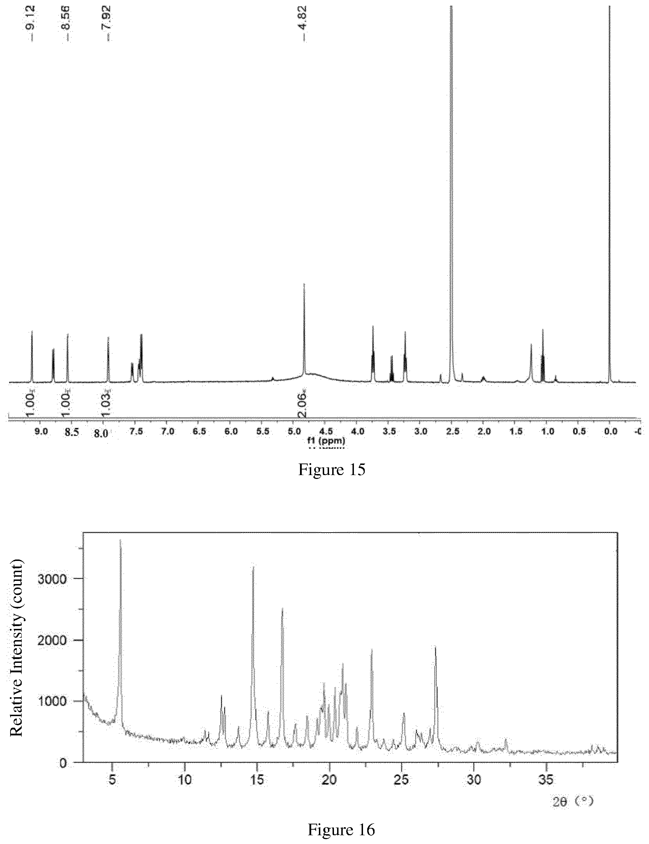

In a certain preferred embodiment, the crystal form **C** of the sulfate salt of the compound of formula I has an X-ray powder diffraction pattern expressed at a 2θ angle basically as shown in Figure 16.

In a certain preferred embodiment, the thermogravimetric analysis pattern of the crystal form **C** of the sulfate salt of the compound of formula I has a weight loss of 3% to 4.5% when heated from an onset to 175 ± 5°C; for example, a weight loss of 3.9% when heated from an onset to 175 ± 5°C (the percentage of the weight loss is the percentage of the weight loss of the sample relative to the weight of the sample prior to this weight loss).

In a certain preferred embodiment, the crystal form **C** of the sulfate salt of the compound of formula I has a differential scanning calorimetry pattern showing three endothermic peaks at 129.2 ± 5°C, 152.8 ± 5°C, and 200.2 ± 5°C.

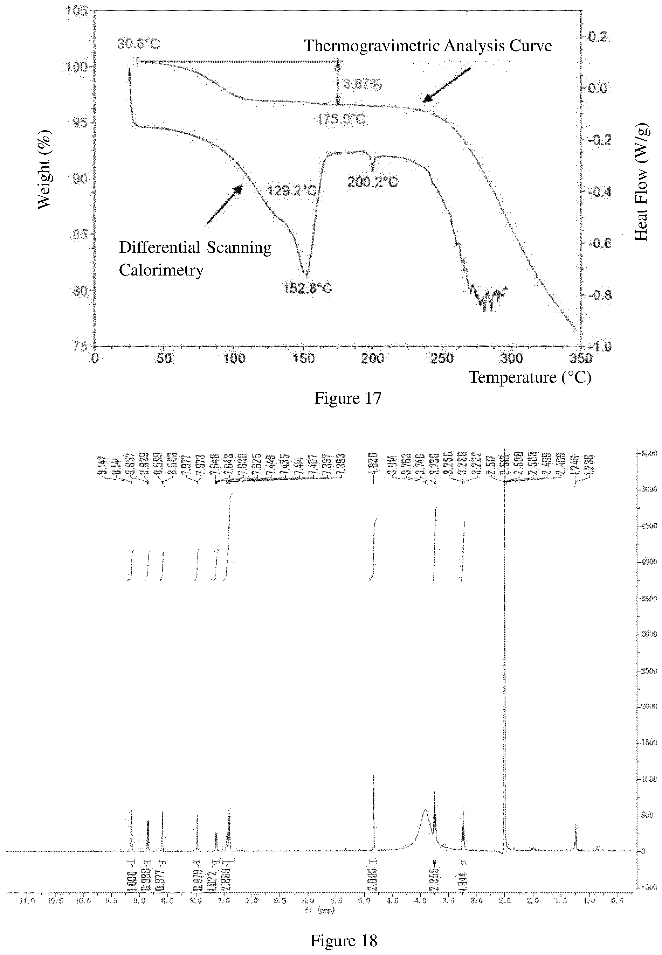

In a certain preferred embodiment, the crystal form **C** of the sulfate salt of the compound of formula I has a differential scanning calorimetry pattern and a thermogravimetric analysis pattern basically as shown in Figure 17.

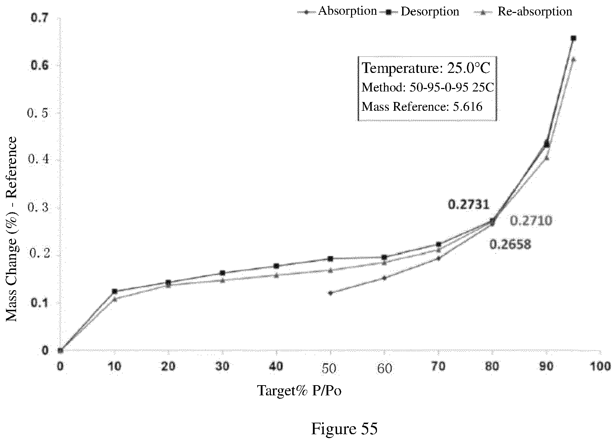

In a certain preferred embodiment, the crystal form **C** of the sulfate salt of the compound of formula I has a dynamic vapor sorption pattern basically as shown in Figure 55.

The eighteenth aspect of the present disclosure provides a crystal form **E** of the sulfate salt of the compound of formula I, which has an X-ray powder diffraction pattern expressed at a 2θ angle comprising diffraction peaks at the following 2θ angles: 16.39 ± 0.2°, 12.75 ± 0.2°, 14.32 ± 0.2°, and 20.28 ± 0.2°; the sulfate salt of the compound of formula I is as described above;

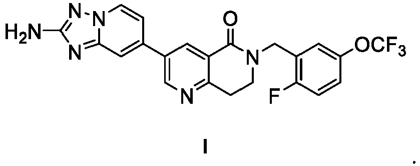

In a certain preferred embodiment, the crystal form **E** of the sulfate salt of the compound of formula I has a molar ratio of the compound of formula I to sulfuric acid of 1: (1-2), such as 1: (1-1.3), and also such as 1:1.3.

In a certain preferred embodiment, the crystal form **E** of the sulfate salt of the compound of formula I has an X-ray powder diffraction pattern expressed at a 2θ angle further comprising diffraction peaks at one or more than one of the following 2θ angles: 17.76 ± 0.2°, 23.45 ± 0.2°, 26.20 ± 0.2°, 17.47 ± 0.2°, and 19.76 ± 0.2°;

preferably, the crystal form **E** of the sulfate salt of the compound of formula I has an X-ray powder diffraction pattern expressed at a 2θ angle further comprising diffraction peaks at one or more than one of the following 2θ angles: 11.46 ± 0.2°, 25.88 ± 0.2°, 21.90 ± 0.2°, 23.00 ± 0.2°, 20.79 ± 0.2°, and 18.25 ± 0.2°.

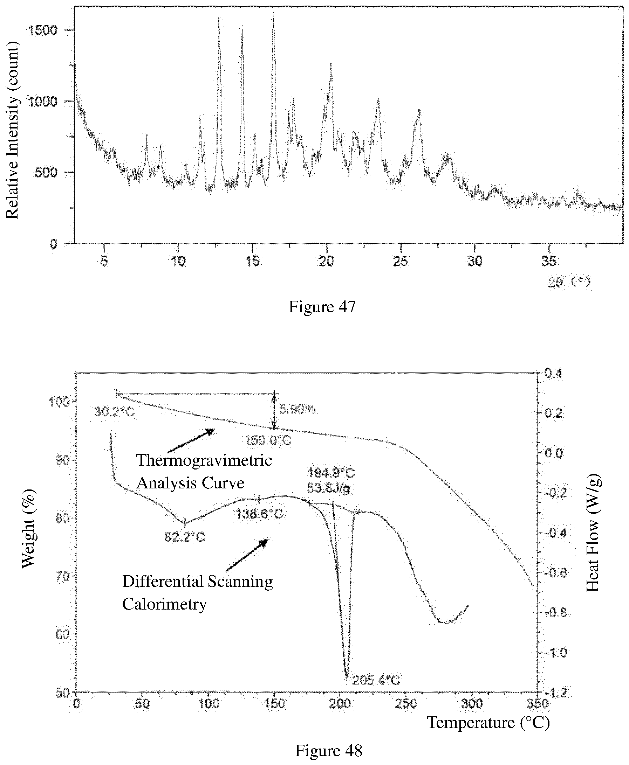

In a certain preferred embodiment, the crystal form **E** of the sulfate salt of the compound of formula I has an X-ray powder diffraction pattern expressed at a 2θ angle basically as shown in Figure 47.

In a certain preferred embodiment, the thermogravimetric analysis pattern of the crystal form **E** of the sulfate salt of the compound of formula I has a weight loss of 5% to 6.5% (such as 5.9%) when heated from an onset to 150 ± 5°C (the percentage of the weight loss is the percentage of the weight loss of the sample relative to the weight of the sample prior to this weight loss).

In a certain preferred embodiment, the crystal form **E** of the sulfate salt of the compound of formula I has a differential scanning calorimetry pattern showing three endothermic peaks at 82.2 ± 5°C, 138.6 ± 5°C, and 205.4 ± 5°C.

In a certain preferred embodiment, the crystal form **E** of the sulfate salt of the compound of formula I has a differential scanning calorimetry pattern and a thermogravimetric analysis pattern basically as shown in Figure 48.

The nineteenth aspect of the present disclosure provides a crystal form **F** of the sulfate salt of the compound of formula I, which has an X-ray powder diffraction pattern expressed at a 2θ angle comprising diffraction peaks at the following 2θ angles: 24.91 ± 0.2°, 20.60 ± 0.2°, 27.62 ± 0.2°, and 16.04 ± 0.2°; the sulfate salt of the compound of formula I is as described above;

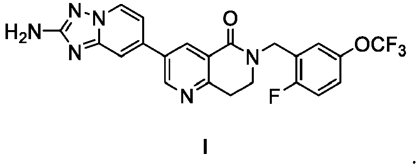

In a certain preferred embodiment, the crystal form **F** of the sulfate salt of the compound of formula I has a molar ratio of the compound of formula I to sulfuric acid of 1: (1-2), such as 1: (1-1.3), and also such as 1:1.1.

In a certain preferred embodiment, the crystal form **F** of the sulfate salt of the compound of formula I has an X-ray powder diffraction pattern expressed at a 2θ angle further comprising diffraction peaks at one or more than one of the following 2θ angles: 24.26 ± 0.2°, 24.14 ± 0.2°, 18.63 ± 0.2°, 17.01 ± 0.2°, and 21.53 ± 0.2°;
preferably, the crystal form **F** of the sulfate salt of the compound of formula I has an X-ray powder diffraction pattern expressed at a 2θ angle further comprising diffraction peaks at one or more than one of the following 2θ angles: 23.10 ± 0.2°, 19.94 ± 0.2°, 19.73 ± 0.2°, 15.29 ± 0.2°, 28.29 ± 0.2°, and 20.12 ± 0.2°.

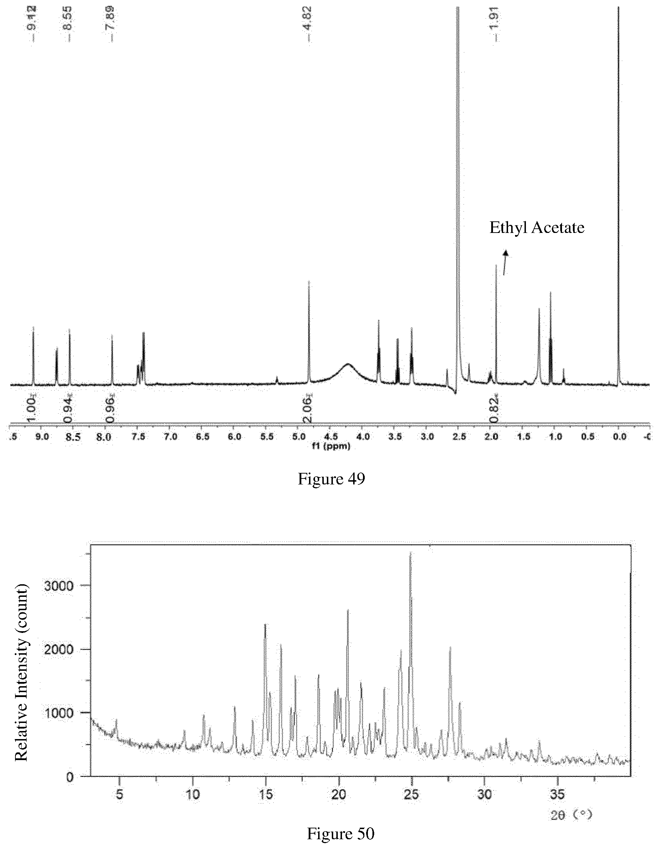

In a certain preferred embodiment, the crystal form **F** of the sulfate salt of the compound of formula I has an X-ray powder diffraction pattern expressed at a 2θ angle basically as shown in Figure 50.

In a certain preferred embodiment, the thermogravimetric analysis pattern of the crystal form **F** of the sulfate salt of the compound of formula I has a weight loss of 5% to 7% (such as 6.4%) when heated from an onset to 160°C, and a weight loss of 6% to 8% (such as 6.9%) when heated from 160°C to 225°C (the percentage of the weight loss is the percentage of the weight loss of the sample relative to the weight of the sample prior to this weight loss).

In a certain preferred embodiment, the crystal form **F** of the sulfate salt of the compound of formula I has a differential scanning calorimetry pattern showing two endothermic peaks at 123.6 ± 5°C and 185.2 ± 5°C and one exothermic peak at 206.0 ± 5°C.

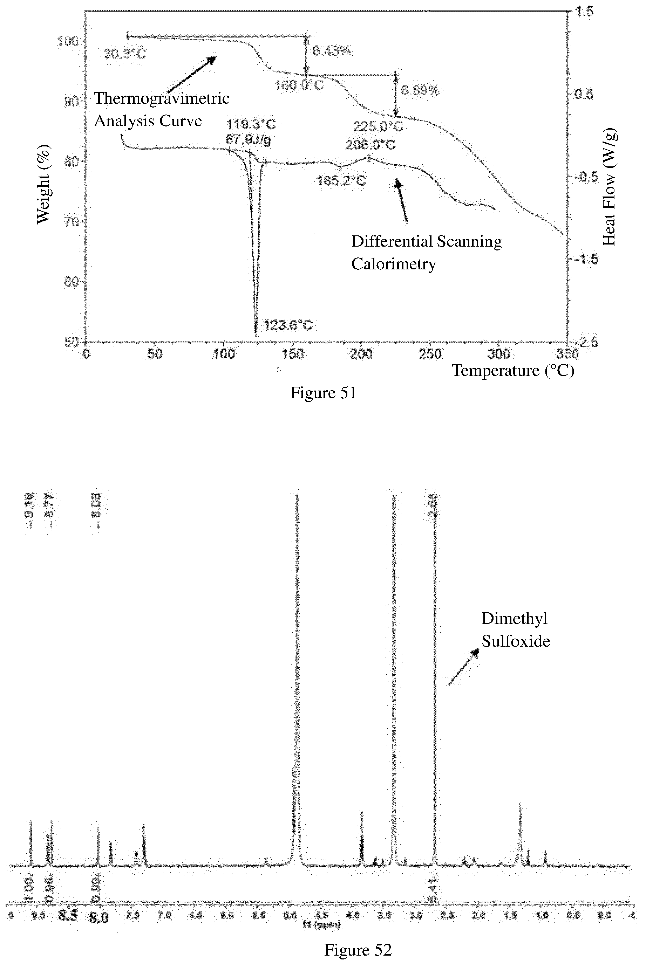

In a certain preferred embodiment, the crystal form **F** of the sulfate salt of the compound of formula I has a differential scanning calorimetry pattern and a thermogravimetric analysis pattern basically as shown in Figure 51.

The preparation method for the crystal form of the RIPK1 inhibitor, the pharmaceutically acceptable salt thereof, and the crystal form of the pharmaceutically acceptable salt thereof in the present disclosure can be obtained by many methods known in the art.

The preparation method for the polymorph of a sulfate salt includes, but is not limited to, using anti-solvent addition, anti-anti-solvent addition, gas-solid diffusion, gas-liquid permeation, stirring in suspension, slow volatilization, stirring in suspension with cyclic heating and cooling, polymer induction, grinding, and other methods to crystallize the sample of the sulfate salt of the compound of formula I.

Anti-solvent addition: The starting sample of the sulfate salt is weighed and dissolved in a good solvent, then an anti-solvent is added to the resulting clear solution, and the dropwise addition is continued with stirring until the solid is precipitated; if no solid precipitates after adding 4.5 mL of the anti-solvent, the dropwise addition is stopped. The solid is separated by centrifugation and tested by XRPD. Among them, the volume to mass ratio of the good solvent to the starting sample of the sulfate salt is 5 to 75 times.

Anti-anti-solvent addition: The starting sample of the sulfate salt is weighed and dissolved in a good solvent, and the resulting clear solution is added dropwise to an anti-solvent while stirring. The solid is separated by centrifugation and tested by XRPD. Among them, the volume to mass ratio of the good solvent to the starting sample of the sulfate salt is 5 to 75 times.

Slow volatilization: A sample of the sulfate salt is weighed, added with a solvent, shaken until clear; the resulting clear solution is sealed in a vial with a sealing film, and 5 small holes are punched in the film; the vial is then placed at room temperature for slow evaporation. The solid obtained by volatilization is collected and tested by XRPD. Among them, the volume to mass ratio of the solvent to the starting sample of the sulfate salt is 50 to 100 times.

Stirring in suspension at room temperature: The starting sample of the sulfate salt is weighed and added with a solvent, and the resulting suspension is magnetically stirred (about 750 rpm) for about 5 days at room temperature, then the solid is separated by centrifugation and tested by XRPD. Among them, the volume to mass ratio of the solvent to the starting sample of the sulfate salt is 25 to 50 times.

Stirring in suspension at 50°C: The starting sample of the sulfate salt is weighed and added with a solvent, and the resulting suspension is magnetically stirred (about 750 rpm) for about 3 days at 50°C, then the solid is separated by centrifugation and tested by XRPD. Among them, the volume to mass ratio of the solvent to the starting sample of the sulfate salt is 30 times.

Slow cooling: The starting sample of the sulfate salt is weighed and added with a solvent, stirred for about 2 hours at 50°C and filtered to obtain a filtrate (using a PTFE filter membrane with a pore size of 0.45 µm); the resulting filtrate is placed in a biochemical incubator and cooled from 50°C to 5°C at a rate of 0.1°C/minute; the precipitated solid is collected and tested by XRPD. Among them, the volume to mass ratio of the solvent to the starting sample of the sulfate salt is 50 times.

Gas-solid diffusion: The starting sample of the sulfate salt is weighed into a 3 mL vial, and the solvent is added to another 20 mL vial; the 3 mL vial is placed open-mouthed in the 20 mL vial, and the 20 mL vial is then sealed. After standing at room temperature for about 7 days, the solid is collected and tested by XRPD. Among them, the volume to mass ratio of the solvent to the starting sample of the sulfate salt is 200 times.

Gas-liquid diffusion: 20 mg of the starting sample of the sulfate salt is weighed into a 3 mL vial, and a clear solution is obtained using 0.05 mL to 2.2 mL of the solvent, which is filtered to obtain a filtrate, and the filtrate is transferred to a 3 mL vial; about 3 mL of anti-solvent is added to another 20 mL vial, and the 3 mL vial containing the filtrate is placed open-mouthed in the 20 mL vial, then the 20 mL vial is sealed and stood at room temperature. When solid precipitation is observed, the solid is collected and tested by XRPD.

Stirring in suspension with cyclic heating and cooling at 50°C to 5°C: The starting sample of the sulfate salt is weighed, added with a solvent, and magnetically stirred for 2 hours (about 750 rpm) at 50°C. The mixture is cooled to 5°C at a rate of 0.1°C/min and stirred for 0.5 hours at 5°C, and then heated to 50°C at a rate of 0.1°C/min and stirred for another 0.5 hours at 50°C. After the above steps are repeated once, the mixture is cooled to 5°C at a rate of 0.1°C/min and maintained at 5°C. The solid is collected and tested by XRPD. Among them, the volume to mass ratio of the solvent to the starting sample of the sulfate salt is 25 times.

Grinding: The starting sample of the sulfate salt is weighed into an agate mortar, added with 0.05 mL of solvent, manually ground for about 15 minutes, and then the solid is collected and tested by XRPD. Among them, the volume to mass ratio of the solvent to the starting sample of the sulfate salt is 2.5 times.

Polymer induction: About 20 mg of the starting sample of the sulfate salt is weighed into a 3 mL vial and dissolved in about 2.0 mL of solvent until the solution is clear, and added with 2 mg of mixed polymer; the vial containing the filtrate is sealed with a sealing film, and 3-5 small holes are punched in the film, and then the vial is placed at room temperature for slow volatilization; the resulting solid is collected and tested by XRPD.

The mixed polymer is mixed polymer A and mixed polymer B. The mixed polymer A is a mixture of polyvinylpyrrolidone, polyvinyl alcohol, polyvinyl chloride, polyvinyl acetate, hydroxypropyl methylcellulose, and methylcellulose, with equal weights of each component. The mixed polymer B is a mixture of polycaprolactone, polyethylene glycol, polymethyl methacrylate, sodium alginate, and hydroxyethyl cellulose, with equal weights of each component.

The solvents include, but are not limited to the following solvents, for example, alcohols, halogenated hydrocarbons, nitriles, ketones, ethers, sulfoxides, amides, lipids, water, alkanes, aromatics, and the mixture thereof. Alcohols are preferably methanol, ethanol, propanol, isopropanol, and butanol; halogenated hydrocarbons are preferably dichloromethane and chloroform; nitriles are preferably acetonitrile. Ketones are preferably acetone, 2-butanone, methyl isobutyl ketone, and *N*-methyl pyrrolidone; ethers are preferably methyl *tert-*butyl ether, tetrahydrofuran, 2-methyltetrahydrofuran, cyclopentyl methyl ether, 1,4-dioxane, and anisole; sulfoxides are preferably dimethyl sulfoxide; amides are preferably *N,N-*dimethylformamide and *N,N*-dimethylacetamide; lipids are preferably ethyl acetate and isopropyl acetate; alkanes are preferably n-heptane; aromatics are preferably toluene and xylene.

The twenty-second aspect of the present disclosure provides a preparation method for the crystal form **A** of the sulfate salt of the compound of formula I, which is method **a** or method **b;**
method **a,** which comprises the following steps: crystallizing the compound of formula I and sulfuric acid in methanol at room temperature to obtain the crystal form **A** of the sulfate salt of the compound of formula I;
method **b,** which comprises the following steps: crystallizing the sulfate salt of the compound of formula I in a solvent to obtain the crystal form **A** of the sulfate salt of the compound of formula I; the solvent is a solvent of C₁₋₃ alcohol - 2-methyltetrahydrofuran.

In method **a,** the compound of formula I is the crystal form A of the compound of formula I.

In method **b,** the sulfate salt of the compound of formula I is the crystal form C of the sulfate salt of the compound of formula I.

In method **b,** the crystallization preferably comprises the following steps: dissolving the sulfate salt of the compound of formula I in the solvent of C₁₋₃ alcohol to obtain a mixed solution, and adding (e.g., adding by dropwise addition) 2-methyltetrahydrofuran to the resulting mixed solution (i.e., anti-solvent addition). In the preparation method for the crystal form **A** of the sulfate salt of the compound of formula I, the solvent of C₁₋₃ alcohol in the solvent of C₁₋₃ alcohol - 2-methyltetrahydrofuran is preferably one or more than one of methanol, ethanol, and isopropanol, such as methanol.

The amount of the solvent of C₁₋₃ alcohol may not be specifically defined, and it is standard that the sulfate salt of the compound of formula I forms a clear solution in the solvent of C₁₋₃ alcohol.

The 2-methyltetrahydrofuran may not be specifically defined, and it is standard that the mixed solution and 2-methyltetrahydrofuran form a clear solution.

In method a, the crystallization preferably comprises the following steps: suspending, stirring, and crystallizing (i.e., stirring in suspension) the compound of formula I and sulfuric acid (e.g., the molar ratio of the compound of formula I to sulfuric acid is 1:1) in the methanol.

The crystallization can be carried out by crystallization with stirring (e.g., stirring in suspension).

After the crystallization is completed, separation can be carried out according to conventional operating methods in the art, such as centrifugation or filtration.

The twenty-third aspect of the present disclosure provides the crystal form A of the sulfate salt of the compound of formula I prepared by the above preparation method for the crystal form **A** of the sulfate salt of the compound of formula I.

The twenty-fourth aspect of the present disclosure provides a preparation method for the crystal form **C** of the sulfate salt of the compound of formula I, which is method **1** or method 2:
method **1,** which comprises the following steps: dissolving the compound of formula I to clear in a nitrile solvent with a sulfuric acid aqueous solution and then cooling to obtain the crystal form **C** of the sulfate salt of the compound of formula I;
method **2,** which comprises the following steps: crystallizing the compound of formula I and sulfuric acid in a water-nitrile solvent at room temperature to obtain the crystal form **C** of the sulfate salt of the compound of formula I.

In method **1** or **2,** the compound of formula I is the crystal form A of the compound of formula I.

In method **1,** the molar ratio of the compound of formula I to sulfuric acid is 1:(1-2) (e.g., 1:(1-1.1)).

In method **2,** the nitrile solvent in the water-nitrile solvent is preferably acetonitrile.

In method **1,** the nitrile solvent is preferably acetonitrile.

In method **1** or **2,** the mass to volume ratio of the compound of formula I to the nitrile solvent is 120-20 mg/mL, such as 100-40 mg/mL.

After the crystallization is completed, separation can be carried out according to conventional operating methods in the art, such as centrifugation or filtration.

The twenty-fifth aspect of the present disclosure provides the crystal form **C** of the sulfate salt of the compound of formula I prepared by the above preparation method for the crystal form **C** of the sulfate salt of the compound of formula I.

The twenty-sixth aspect of the present disclosure provides a preparation method for the crystal form **D** of the sulfate salt of the compound of formula I, which comprises the following steps: forming a suspension of the sulfate salt of the compound of formula I in propanol for crystal form transition (stirring in suspension at 50°C) to obtain the crystal form **D** of the sulfate salt of the compound of formula I.

The sulfate salt of the compound of formula I is the crystal form **C** of the sulfate salt of the compound of formula I.

The propanol is preferably *n*-propanol and/or isopropanol, such as *n*-propanol.

The mass to volume ratio of the sulfate salt of the compound of formula I to the propanol is preferably 25-40 mg/mL, such as 33.3 mg/mL.

After the crystal form transition is completed, separation can be carried out according to conventional operating methods in the art, such as centrifugation or filtration.

The twenty-seventh aspect of the present disclosure provides the crystal form **D** of the sulfate salt of the compound of formula I prepared by the above preparation method for the crystal form **D** of the sulfate salt of the compound of formula I.

The twenty-eighth aspect of the present disclosure provides a preparation method for the crystal form **E** of the sulfate salt of the compound of formula I, which comprises the following steps: forming a suspension of the monohydrate sulfate salt of the compound of formula I (e.g., the monohydrate crystal form **C** of the sulfate salt of the compound of formula I) in the mixed solvent of ester-water (e.g., a mixed solvent of ethyl acetate-water with a volume ratio of 19:1) for crystal form transition (stirring in suspension at 50°C) to obtain the crystal form **E** of the sulfate salt of the compound of formula I.

The twenty-ninth aspect of the present disclosure provides the crystal form **E** of the sulfate salt of the compound of formula I prepared by the above preparation method for the crystal form **E** of the sulfate salt of the compound of formula I.

The thirtieth aspect of the present disclosure provides a preparation method for the crystal form **F** of the sulfate salt of the compound of formula I, which comprises the following steps: volatilizing the monohydrate sulfate salt of the compound of formula I (e.g., the monohydrate crystal form **C** of the sulfate salt of the compound of formula I) in a sulfoxide solvent (e.g., dimethyl sulfoxide (DMSO)) to obtain the crystal form **E** of the sulfate salt of the compound of formula I.

The thirty-first aspect of the present disclosure provides the crystal form **F** of the sulfate salt of the compound of formula I prepared by the above preparation method for the crystal form **F** of the sulfate salt of the compound of formula I.

The thirty-second aspect of the present disclosure provides a pharmaceutical composition, comprising a therapeutically effective dose of substance **A** and a pharmaceutically acceptable excipient; the substance **A** is the crystal form of the compound of formula I (the crystal form **A** of the compound of formula I, the crystal form **B** of the compound of formula I, or the crystal form **C** of the compound of formula I), the pharmaceutically acceptable salt of the compound of formula I or the crystal form of the pharmaceutically acceptable salt of the compound of formula I (the crystal form **A** of the hydrochloride salt of the compound of formula I, the crystal form **A** of the citrate salt of the compound of formula I, the crystal form **A** of the maleate salt of the compound of formula I, the crystal form **A** of the fumarate salt of the compound of formula I, the crystal form **A** of the methanesulfonate salt of the compound of formula I, the crystal form **B** of the methanesulfonate salt of the compound of formula I, the crystal form **C** of the methanesulfonate salt of the compound of formula I, the crystal form **A** of the *p*-toluenesulfonate salt of the compound of formula I, the crystal form **A** of the cyclamate salt of the compound of formula I, the crystal form **A** of the sulfate salt of the compound of formula I, the crystal form **D** of the sulfate salt of the compound of formula I, the crystal form **I** of the sulfate salt of the compound of formula I, the crystal form **B** of the sulfate salt of the compound of formula I, the crystal form C of the sulfate salt of the compound of formula I, the crystal form **E** of the sulfate salt of the compound of formula I, or the crystal form **F** of the sulfate of the compound of formula I).

The thirty-third aspect of the present disclosure provides a use of substance **B** in the manufacture of a drug for treating and/or preventing a disease, the substance **B** is the pharmaceutical composition, the crystal form of the compound of formula I (the crystal form **A** of the compound of formula I, the crystal form **B** of the compound of formula I, or the crystal form **C** of the compound of formula I), the pharmaceutically acceptable salt of the compound of formula I or the crystal form of the pharmaceutically acceptable salt of the compound of formula I (the crystal form **A** of the hydrochloride salt of the compound of formula I, the crystal form **A** of the citrate salt of the compound of formula I, the crystal form **A** of the maleate salt of the compound of formula I, the crystal form **A** of the fumarate salt of the compound of formula I, the crystal form **A** of the methanesulfonate salt of the compound of formula I, the crystal form **B** of the methanesulfonate salt of the compound of formula I, the crystal form **C** of the methanesulfonate salt of the compound of formula I, the crystal form **A** of the *p-*toluenesulfonate salt of the compound of formula I, the crystal form **A** of the cyclamate salt of the compound of formula I, the crystal form **A** of the sulfate salt of the compound of formula I, the crystal form **D** of the sulfate salt of the compound of formula I, the crystal form **I** of the sulfate salt of the compound of formula I, the crystal form **B** of the sulfate salt of the compound of formula I, the crystal form **C** of the sulfate salt of the compound of formula I, the crystal form **E** of the sulfate salt of the compound of formula I, or the crystal form **F** of the sulfate of the compound of formula I).

The disease is preferably a RIPK1-mediated disease, and the disease is preferably one or more than one of stroke, inflammatory bowel disease, ulcerative colitis, Crohn's disease, psoriasis, rheumatoid arthritis, NASH, and heart failure.

Or, the disease is preferably one or more than one of stroke, inflammatory bowel disease, ulcerative colitis, Crohn's disease, psoriasis, rheumatoid arthritis, NASH, and heart failure.

The dosage of substance **B** is an effective therapeutic amount.

The thirty-fourth aspect of the present disclosure provides a use of substance **B** in the manufacture of a RIPK1 inhibitor, the substance **B** is the pharmaceutical composition, the crystal form **A** of the compound of formula I, the crystal form **B** of the compound of formula I, the crystal form **C** of the compound of formula I, the pharmaceutically acceptable salt of the compound of formula I, the crystal form **A** of the hydrochloride salt of the compound of formula I, the crystal form **A** of the citrate salt of the compound of formula I, the crystal form **A** of the maleate salt of the compound of formula I, the crystal form **A** of the fumarate salt of the compound of formula I, the crystal form **A** of the methanesulfonate salt of the compound of formula I, the crystal form **B** of the methanesulfonate salt of the compound of formula I, the crystal form **C** of the methanesulfonate salt of the compound of formula I, the crystal form **A** of the *p*-toluenesulfonate salt of the compound of formula I, the crystal form **A** of the cyclamate salt of the compound of formula I, the crystal form A of the sulfate salt of the compound of formula I, the crystal form **D** of the sulfate salt of the compound of formula I, the crystal form **I** of the sulfate salt of the compound of formula I, the crystal form **B** of the sulfate salt of the compound of formula I, the crystal form **C** of the sulfate salt of the compound of formula I, the crystal form **E** of the sulfate salt of the compound of formula I, or the crystal form **F** of the sulfate of the compound of formula I.

In the use, the RIPK1 inhibitor is preferably an *in vivo* or *in vitro* RIPK1 inhibitor.

In the use, the RIPK1 inhibitor is preferably used for treating a RIPK1-related disease or disorder (e.g., a disease or disorder mediated by RIPK1).

The RIPK1-related disease or disorder (e.g., a disease or disorder mediated by RIPK1) includes, but is not limited to, inflammatory diseases (e.g., Crohn's disease and ulcerative colitis, inflammatory bowel disease, asthma, graft-versus-host disease, chronic obstructive pulmonary disease), autoimmune diseases (e.g., Graves' disease, rheumatoid arthritis, systemic lupus erythematosus, psoriasis; destructive bone diseases, such as bone resorptive diseases, osteoarthritis, osteoporosis, multiple myeloma-associated bone disease); proliferative diseases (e.g., acute myeloid leukemia, chronic granulocytic leukemia); angiogenic disorders (e.g., angiogenic disorders including solid tumors, ocular neovascularization, and infantile hemangiomas); infectious diseases (e.g., septicemia, infectious shock, and shigellosis); neurodegenerative diseases (e.g., Alzheimer's disease, Parkinson's disease, amyotrophic lateral sclerosis, cerebral ischemia, or neurodegenerative diseases caused by traumatic injury), tumors, and viral diseases (e.g., metastatic melanoma, Kaposi's sarcoma, multiple myeloma, fibroblastoma, lymphocytoma, HIV infection and CMV retinitis, AIDS).

The RIPK1-related disease or disorder (e.g., a disease or disorder mediated by RIPK1) includes, but is not limited to, pancreatitis (acute or chronic), asthma, allergy, adult respiratory distress syndrome, chronic obstructive pulmonary disease, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, amyotrophic lateral sclerosis, multiple sclerosis, inflammatory bowel disease, ulcerative colitis, Crohn's disease, psoriasis, graft-versus-host disease, endotoxin-induced inflammatory response, tuberculosis, atherosclerosis, muscular degeneration, cachexia, psoriasis arthritis, Reiter's syndrome, gout, traumatic arthritis, rubella arthritis, acute synovitis, pancreatic beta-cell disease; diseases characterized by massive neutrophilic infiltration; rheumatoid spondylitis, gouty arthritis, and other arthritic disorders, cerebral malaria, chronic pulmonary inflammation, silicosis, lung sarcoma, bone resorption disease, allograft rejection, fever and myalgia due to infection, cachexia secondary to infection, luteinization, scar tissue formation, ulcerative colitis, fever, influenza, osteoporosis, osteoarthritis, acute myeloid leukemia, chronic myeloid leukemia, metastatic melanoma, Kaposi's sarcoma, multiple myeloma, sepsis, septic shock, and shigellosis; Alzheimer's disease, Parkinson's disease, cerebral ischemia, or neurodegenerative diseases caused by traumatic injury; angiogenic disorders including solid tumors, ocular neovascularization, and infantile hemangiomas; viral diseases including acute hepatitis infections (including hepatitis A, hepatitis B, and hepatitis C), HIV viral infections and CMV retinitis, AIDS, ARC or malignant tumors and herpes; stroke, myocardial ischemia, stroke heart disease attack, organ ischemia, vascular proliferation, cardiac and renal reperfusion injury, thrombosis, cardiac hypertrophy, thrombin-induced platelet aggregation, endotoxemia and/or toxic shock syndrome, diseases associated with prostaglandin-endoperoxide synthase-2, and pemphigus vulgaris.

The RIPK1-related disease or disorder (e.g., a disease or disorder mediated by RIPK1) is selected from: stroke, inflammatory bowel disease, Crohn's disease, ulcerative colitis, allograft rejection, rheumatoid arthritis, psoriasis, ankylosing spondylitis, psoriatic arthritis, and pemphigus vulgaris. Or, the disorder is preferably selected from ischemia-reperfusion injuries, including cerebral ischemia-reperfusion injuries caused by stroke and myocardial ischemia-reperfusion injuries caused by myocardial infarction.

### Definitions and explanations of terms

All literatures cited in the present disclosure are incorporated herein by reference in their entirety, and if the meaning expressed in these literatures is inconsistent with the present disclosure, the present disclosure shall prevail. In addition, various terms and phrases used in the present disclosure have general meanings known to those skilled in the art. Even so, the present disclosure still hopes to provide a more detailed description and explanation of these terms and phrases here. If the terms and phrases mentioned are inconsistent with the commonly known meaning, the meaning expressed in the present disclosure shall prevail.

The polymorph of the compound of formula 1 in the present disclosure has an X-ray powder diffraction characteristic peak expressed at a 2θ angle, wherein "±0.20°" is the allowable measurement error range.

The polymorph of the compound of formula I in the present disclosure may be used in combination with other active ingredients, provided that it does not produce other unfavorable effects, such as allergic responses.

In the present disclosure, the term "composition" refers to include a product that contains the specified ingredients in the specified amounts, or that is produced directly or indirectly from a combination of the specified ingredients in the specified amounts.

Those skilled in the art can prepare a suitable pharmaceutical composition with the polymorph of the compound of formula I of the present disclosure by using known drug carriers. The pharmaceutical composition may be specially formulated for oral administration, parenteral injection, or rectal administration in solid or liquid form.

The pharmaceutical composition can be formulated into a variety of dosage forms for convenient administration, such as oral formulations (e.g., tablets, capsules, solutions, or suspensions); injectable formulations (e.g., injectable solutions or suspensions, or injectable dry powders that can be immediately used after the addition of a solvent of the drug before injection).

The term "therapeutically and/or prophylactically effective dose" as used in the present disclosure is the amount of a drug or pharmaceutical formulation that elicits the biological or medical response in an tissue, system, animal, or human that is sought by a researcher, veterinarian, physician, or other person.

When used for therapeutic and/or prophylactic purposes, the total daily dose of the polymorph of the compound of formula I and pharmaceutical composition of the present disclosure is required to be determined by the attending physician within the scope of reliable medical judgment. For any specific patient, the specific therapeutically effective dose level depends on a variety of factors, including a disorder being treated and the severity of the disorder; activity of a specific compound used; a specific composition used; age, weight, general health status, sex and diet of the patient; administration time, administration route, and excretion rate of the specific compound used; duration of treatment; drugs used in combination or simultaneously with the specific compounds used; and similar factors known in the medical field. For example, it is common practice in the art to start with a dose of the compound that is lower than the level required to achieve the desired therapeutic effect, and then gradually increase the dose until the desired effect is achieved.

The term "polymorphy" or "polymorph" as used in the present disclosure refers to a crystal form with the same chemical composition, but a different spatial arrangement of the molecules, atoms, and/or ions that constitute the crystal. Although polymorphs have the same chemical composition, they have different stacking and geometric arrangements, and may exhibit different physical properties, such as melting point, shape, color, density, hardness, deformability, stability, solubility, dissolution rate, and similar properties. According to their temperature-stability relationship, two polymorphs can be monotropic or enantiotropic. For the monotropic system, the relative stability between the two solid phases remains constant as the temperature changes. Conversely, in an enantiotropic system, there exists a transition temperature at which the stability of the two phases switches ((Theory and Origin of Polymorphism in "Polymorphism in Pharmaceutical Solids" (1999) ISBN: )-8247-0237). The phenomenon of a compound existing in different crystal structures is known as drug polymorphism.

The term "room temperature" or "RT" as used herein refers to an ambient temperature of 20°C to 25°C (68°F to 77°F).

Abbreviation description: MeOH: methanol; 2-MeTHF: 2-methyltetrahydrofuran; EtOH: ethanol; 1,4-Dioxane: 1,4-dioxane; IPA: isopropanol; ACN: acetonitrile; Acetone: acetone; DCM: dichloromethane; MIBK: methyl isobutyl ketone: Toluene: toluene; EtOAc: ethyl acetate; *n*-Heptane: *n*-heptane; IPAc: isopropyl acetate; DMSO: dimethyl sulfoxide; MTBE: methyl *tert*-butyl ether; DMAc: dimethylacetamide; THF: tetrahydrofuran; NMP: *N-*methylpyrrolidone.

The above preferred conditions can be combined arbitrarily to obtain preferred embodiments of the present disclosure without violating common knowledge in the art.

The reagents and starting materials used in the present disclosure are all commercially available.

The positive progressive effect of the present disclosure is that the crystal form of the RIPK1 inhibitor, the acid salt thereof, and the crystal form of the acid salt thereof of the present disclosure have the advantages of good inhibitory activity against RIPK1 enzyme, high inhibitory activity against U937 cells, low hygroscopicity and good stability, and have good prospects for druggability.

### BRIEF DESCRIPTION OF THE DRAWINGS

Figure 1 is the XRPD pattern of the anhydrous crystal form A of the compound of formula I.
Figure 2 is the TGA/DSC pattern of the anhydrous crystal form A of the compound of formula I.
Figure 3 is the ¹H NMR pattern of the anhydrous crystal form A of the compound of formula I.
Figure 4 is the XRPD pattern of the anhydrous crystal form B of the compound of formula I.
Figure 5 is the TGA/DSC pattern of the anhydrous crystal form B of the compound of formula I.
Figure 6 is the ¹H NMR comparison pattern of the anhydrous crystal form B of the compound of formula I with the anhydrous crystal form A of the compound of formula I.
Figure 7 is the XRPD pattern of the crystal form A of the hydrochloride salt of the compound of formula I.
Figure 8 is the TGA/DSC pattern of the crystal form A of the hydrochloride salt of the compound of formula I.
Figure 9 is the ¹H NMR pattern of the crystal form A of the hydrochloride salt of the compound of formula I.
Figure 10 is the XRPD pattern of the crystal form A of the sulfate salt of the compound of formula I.
Figure 11 is the TGA/DSC pattern of the crystal form A of the sulfate salt of the compound of formula I.
Figure 12 is the ¹H NMR pattern of the crystal form A of the sulfate salt of the compound of formula I.
Figure 13 is the XRPD pattern of the crystal form B of the sulfate salt of the compound of formula I.
Figure 14 is the TGA/DSC pattern of the crystal form B of the sulfate salt of the compound of formula I.
Figure 15 is the ¹H NMR pattern of the crystal form B of the sulfate salt of the compound of formula I.
Figure 16 is the XRPD pattern of the crystal form C of the sulfate salt of the compound of formula I.
Figure 17 is the TGA/DSC pattern of the crystal form C of the sulfate salt of the compound of formula I.
Figure 18 is the ¹H NMR pattern of the crystal form C of the sulfate salt of the compound of formula I.
Figure 19 is the XRPD pattern of the crystal form A of the citrate salt of the compound of formula I.
Figure 20 is the TGA/DSC pattern of the crystal form A of the citrate salt of the compound of formula I.
Figure 21 is the ¹H NMR pattern of the crystal form A of the citrate salt of the compound of formula I.
Figure 22 is the XRPD pattern of the crystal form A of the maleate salt of the compound of formula I.
Figure 23 is the TGA/DSC pattern of the crystal form A of the maleate salt of the compound of formula I.
Figure 24 is the ¹H NMR pattern of the crystal form A of the maleate salt of the compound of formula I.
Figure 25 is the XRPD pattern of the crystal form A of the fumarate salt of the compound of formula I.
Figure 26 is the TGA/DSC pattern of the crystal form A of the fumarate salt of the compound of formula I.
Figure 27 is the ¹H NMR pattern of the crystal form A of the fumarate salt of the compound of formula I.
Figure 28 is the XRPD pattern of the crystal form A of the methanesulfonate salt of the compound of formula I.
Figure 29 is the TGA/DSC pattern of the crystal form A of the methanesulfonate salt of the compound of formula I.
Figure 30 is the ¹H NMR pattern of the crystal form A of the methanesulfonate salt of the compound of formula I.
Figure 31 is the XRPD pattern of the crystal form B of the methanesulfonate salt of the compound of formula I.
Figure 32 is the TGA/DSC pattern of the crystal form B of the methanesulfonate salt of the compound of formula I.
Figure 33 is the ¹H NMR pattern of the crystal form B of the methanesulfonate salt of the compound of formula I.
Figure 34 is the XRPD pattern of the crystal form C of the methanesulfonate salt of the compound of formula I.
Figure 35 is the TGA/DSC pattern of the crystal form C of the methanesulfonate salt of the compound of formula I.
Figure 36 is the ¹H NMR pattern of the crystal form C of the methanesulfonate salt of the compound of formula I.
Figure 37 is the XRPD pattern of the crystal form A of the p-toluenesulfonate salt of the compound of formula I.
Figure 38 is the TGA/DSC pattern of the crystal form A of the p-toluenesulfonate salt of the compound of formula I.
Figure 39 is the ¹H NMR pattern of the crystal form A of the p-toluenesulfonate salt of the compound of formula I.
Figure 40 is the XRPD pattern of the crystal form A of the cyclamate salt of the compound of formula I.
Figure 41 is the TGA/DSC pattern of the crystal form A of the cyclamate salt of the compound of formula I.
Figure 42 is the ¹H NMR pattern of the crystal form A of the cyclamate salt of the compound of formula I.
Figure 43 is the XRPD pattern of the crystal form D of the sulfate salt of the compound of formula I.
Figure 44 is the TGA/DSC pattern of the crystal form D of the sulfate salt of the compound of formula I.
Figure 45 is the ¹H NMR pattern of the crystal form D of the sulfate salt of the compound of formula I.
Figure 46 is the XRPD pattern of the crystal form I of the sulfate salt of the compound of formula I.
Figure 47 is the XRPD pattern of the crystal form E of the sulfate salt of the compound of formula I.
Figure 48 is the TGA/DSC pattern of the crystal form E of the sulfate salt of the compound of formula I.
Figure 49 is the ¹H NMR pattern of the crystal form E of the sulfate salt of the compound of formula I.
Figure 50 is the XRPD pattern of the crystal form F of the sulfate salt of the compound of formula I.
Figure 51 is the TGA/DSC pattern of the crystal form F of the sulfate salt of the compound of formula I.
Figure 52 is the ¹H NMR pattern of the crystal form F of the sulfate salt of the compound of formula I.
Figure 53 is the XRPD pattern of the crystal form C of the compound of formula I.
Figure 54 is the TGA/DSC pattern of the crystal form C of the compound of formula I.
Figure 55 is the DVS pattern of the crystal form C prepared in Example 9.

### DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples should be selected according to conventional methods and conditions, or according to product specifications.

### Instruments and methods

The obtained solid samples were analyzed using various detection and analytical methods, such as X-ray powder diffraction (XRPD), differential scanning calorimetry (DSC), thermogravimetric analysis (TGA), dynamic vapor sorption (DVS), and ¹H solution nuclear magnetic resonance (¹H solution NMR).
(1) X-ray powder diffraction (XRPD): The XRPD results were collected on PANalytical Empyrean and X'Pert3 X-ray powder diffraction analyzers with scanning parameters as shown in Table 1.

**Table 1 XRPD test parameters (I/II)**

| **Instrument model** | **X'Pert3 (reflection)** | **Empyrean (reflection/variable temperature)** |
|---|---|---|
| X-ray | Cu, kα; Kα1 (Å): 1.540598, Kα2 (Å): 1.544426 | Cu, kα; Kα1 (Å): 1.540598, Kα2 (Å): 1.544426 |
| | Intensity ratio Kα2/Kα1: 0.50 | Intensity ratio Kα2/Kα1: 0.50 |
| X-ray tube setting | 45 kV, 40 mA | 45 kV, 40 mA |
| Divergence slit | 1/8° | Automatic |
| Scan mode | Continuous | Continuous |
| Scan range (°2TH) | 3° to 40° | 3° to 40° |
| Scan step (°2TH) | 0.0263 | 0.0167 |
| Scan time per step (s) | 46.665 | 33.020 |
| Scan time (s) | 5 min 03 s | 10 min 12 s/10 min 13 s |

(2) Thermogravimetric analysis (TGA) and differential scanning calorimetry (DSC): TGA and DSC patterns were collected on a TA Q5000/Discovery 5500 thermogravimetric analyzer and a TA Q2000/Discovery 2500 differential scanning calorimeter, respectively, and the test parameters are listed in Table 2.

**Table 2 TGA and DSC test parameters**

| **Parameters** | **TGA** | **DSC** |
|---|---|---|
| Method | Linear heating | Linear heating |
| Sample tray | Aluminum tray, open | Aluminum tray, sealed |
| Temperature range | RT-350°C | RT-Target temperature |
| Heating rate | 10°C/min | 10°C/min |
| Protective gas | Nitrogen | Nitrogen |

(3) Dynamic vapor sorption (DVS): Dynamic vapor sorption (DVS) curves were collected on DVS Intrinsic from SMS (surface measurement systems). The relative humidity at 25°C was corrected using the deliquescence points of LiCl, Mg(NO₃)₂, and KCl. DVS test parameters are listed in Table 3.

**Table 3 DVS test parameters**

| **Parameters** | **DVS** |
|---|---|
| Temperature | 25°C |
| Sample weight | 20 mg to 40 mg |
| Protective gas and flow rate | N₂, 200 mL/min |
| dm/dt | 0.002%/min |
| Minimum dm/dt equilibration time | 10 min |
| Maximum equilibrium time | 180 min |
| RH range | 0%RH to 95%RH to 0%RH (anhydrous crystal form) |
| | Room humidity to 95%RH to 0%RH to 95%RH (hydrate) |
| RH gradient | 10%RH (0%RH to 90%RH & 90%RH to 0%RH) |
| | 5%RH (90%RH to 95%RH & 95%RH to 90%RH) |

(4) H solution NMR: ¹H solution NMR patterns were collected on a Bruker 400M NMR spectrometer using DMSO-*d₆* as a solvent.

(5) Moisture titration (KF): Moisture titration was carried out by the Metrohm 870 KF Titrinoplus instrument, calibrated with ultrapure water, and the titration reagent was Hydranal^{®} R-Composite 5 with the manufacturer of Sigma-aldrich. Methanol of HPLC grade was used to dissolve solid samples.

(6) High performance liquid chromatography (HPLC): Purity and solubility in the test were determined by Agilent 1260 high performance liquid chromatograph, and the analytical conditions are shown in Table 4.

**Table 4 High performance liquid chromatography test conditions for purity testing**

| **HPLC** | **Agilent 1260** (**DAD detector**) | | | |
|---|---|---|---|---|
| Chromatographic column | Waters Xbridge C18, 150 × 4.6 mm, 5 µm | | | |
| Mobile phase | A: 0.05% TFA in H₂O | | | |
| | B: 0.05% TFA in ACN | | | |
| Elution gradient | Purity test | | Molar ratio test | |
| | Time (min) | %B | Time (min) | %B |
| | 0.0 | 10 | 0.0 | 10 |
| | 6.0 | 48 | 10.00 | 90 |
| | 10.5 | 48 | 12.00 | 90 |
| | 16.0 | 90 | 12.01 | 10 |
| | 20.5 | 90 | 15.00 | 10 |
| | 21.0 | 10 | -- | -- |
| | 26.0 | 10 | -- | -- |
| Run time | 26.0 min | | 15 min | |
| Post-run time | 0.0 min | | | |
| Flow rate for mobile phase | 1.0 mL/min | | | |
| Injection volume | 5 µL | | | |
| Detection wavelength | UV at 225 nm | | | |
| Column temperature | 35°C | | | |
| Injector temperature | RT | | | |
| Diluent | MeOH | | | |

(7) Ion chromatography (IC): ThermoFisher ICS-1100 ion chromatograph was used in the test to analyze the ion content, and the specific conditions are shown in Table 5.

**Table 5 Conditions and parameters of ion chromatography**

| **IC** | **ThermoFisher ICS-1100** |
|---|---|
| Chromatographic column | IonPac AS18 Analytical Column (4 × 250 mm) |
| Mobile phase | 25 mM NaOH |
| Injection volume | 25 µL |
| Flow rate | 1.0 mL/min |
| Sample pool temperature | 35°C |
| Column temperature | 35°C |
| Current | 80 mA |
| Run time | 6.0 min (Cl⁻), 11.0 min (SO₄²⁻) |

**Preparation Example 1: Preparation of compound of formula I**

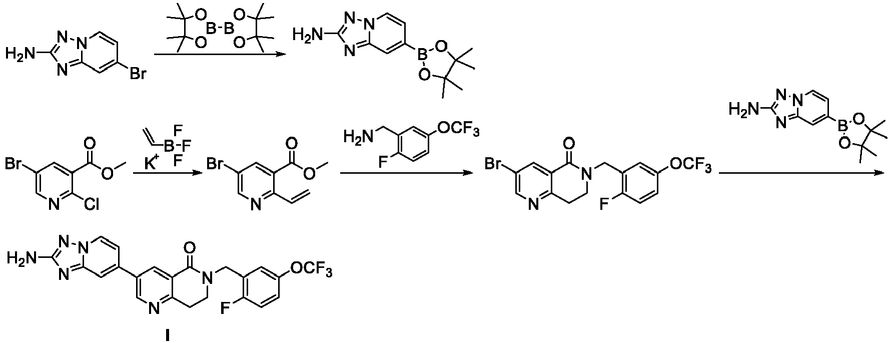

Step 1: Synthesis of 7-(4,4,5,5-tetramethyl-1,3,2-dioxaborolan-2-yl)-[1,2,4]triazolo[1,5-*a*]pyridin-2-amine: 7-Bromo-[1,2,4]triazolo[1,5-*a*]pyridin-2-amine (4.0 g, 18.78 mmol), bis(pinacolato)diboron (5.723 g, 22.53 mmol), potassium acetate (4.601 g, 46.95 mmol), [1,1'-bis(diphenylphosphino)ferrocene]dichloropalladium(II) dichloromethane complex (767 mg, 0.939 mmol), and 1,4-dioxane (50 mL) were added to a 250 mL round-bottom flask, then the reaction system was replaced three times with nitrogen. The reaction mixture was reacted at 90°C overnight, and cooled to room temperature, and filtered under reduced pressure. The filter cake was washed three times with ethyl acetate to obtain the filtrate, which was concentrated to obtain the crude product of 7-(4,4,5,5-tetramethyl-1,3,2-dioxaborolan-2-yl)-[1,2,4]triazolo[1,5-a]pyridin-2-amine (2.6 g, Y: 78%). ES-API:[M+H]⁺= 179.1.

Step 2: Synthesis of methyl 5-bromo-2-vinylnicotinate: Methyl 5-bromo-2-chloronicotinate (10 g, 39.923 mmol), potassium vinyltrifluoroborate (5.348 g, 39.923 mmol), triethylamine (5.56 mL, 39.923 mmol), and [1,1'-bis(diphenylphosphino)ferrocene]dichloropalladium(II) (585 mg, 0.798 mmol) were dissolved in 200 mL of EtOH under nitrogen atmosphere, then the reaction system was replaced three times with nitrogen. The reaction mixture was reacted for 1 hour at 80°C, cooled to room temperature, filtered, and the filtrate was subjected to rotary evaporation until dryness. The residue was dissolved in 300 mL of ethyl acetate and 300 mL of water, separated, then the organic phase was concentrated, and the resulting residue was purified using automated flash chromatography (EtOAc/PE 0 to 20%) on silica gel to obtain methyl 5-bromo-2-vinylnicotinate (5.186 g, Y: 54%). ES-API:[M+H]⁺= 242.1.

Step 3: Synthesis of 3-bromo-6-(2-fluoro-5-(trifluoromethoxy)benzyl)-7,8-dihydro-1,6-naphthyridin-5(6*H*)-one: In a microwave vial, methyl 5-bromo-2-vinylnicotinate (2.5 g, 10.328 mmol) and (2-fluoro-5-(trifluoromethoxy)phenyl)methylamine (4.32 g, 20.656 mmol) were mixed in DMA (75 mL). The reaction mixture was then subjected to microwave radiation for 3 hours at 150°C, cooled to room temperature, added with 100 mLof ethyl acetate, and then washed with water (30 mL × 3) and saturated brine (30 mL × 3). The organic phases were combined, concentrated, and purified using automated flash chromatography (EtOAc/PE 0 to 20%) on silica gel to obtain 3-bromo-6-(2-fluoro-5-(trifluoromethoxy)benzyl)-7,8-dihydro-1,6-naphthyridin-5(6*H*)-one (2.356 g, Y: 54%). ES-API:[M+H]⁺= 419.1.

Step 4: Synthesis of 3-(2-amino-[1,2,4]triazolo[1,5-*a*]pyridin-7-yl)-6-(2-fluoro-5-(trifluoromethoxy)benzyl)-7,8-dihydro-1,6-naphthyridin-5(6*H*)-one: 3-Bromo-6-(2-fluoro-5-(trifluoromethoxy)benzyl)-7,8-dihydro-1,6-naphthyridin-5(6*H*)-one (1.885 g, 4.499 mmol), 7-(4,4,5,5-tetramethyl-1,3,2-dioxaborolan-2-yl)-[1,2,4]triazolo[1,5-*a*]pyridin-2-amine (1.602 g, 8.998 mmol), sodium carbonate (1.192 g, 11.248 mmol), and [1,1'-bis(diphenylphosphino)ferrocene]dichloropalladium(II) dichloromethane complex (184 mg, 0.225 mmol) were dissolved in 30 mL of dioxane and 6 mL of H₂O under nitrogen atmosphere, then the reaction system was replaced three times with nitrogen. The reaction mixture was reacted overnight at 95°C. The reaction mixture was cooled to room temperature, added with 50 mL of EtOAc, and then washed with water (30 mL × 3) and saturated brine (30 mL × 3). The organic phases were combined, concentrated, and the residue was purified using automated flash chromatography (EtOAc/PE 0 to 100%, followed by DCM/MeOH 0 to 4%) on silica gel to obtain the light yellow compound of 3-(2-amino-[1,2,4]triazolo[1,5-a]pyridin-7-yl)-6-(2-fluoro-5-(trifluoromethoxy)benzyl)-7,8-dihydro-1,6-naphthyridin-5(6*H*)-one (compound of formula I, 1.426 g, Y: 67%, purity of 95%). ES-API:[M+H]⁺= 473.0. ¹H NMR (400 MHz, DMSO-d6) δ 9.10 (d, J = 2.4 Hz, 1 H), 8.64 (d, J = 6.8 Hz, 1 H), 8.52 (d, J = 2.4 Hz,1 H), 7.80 (d, J = 1.6 Hz, 1 H), 7.40-7.45 (m, 3 H), 7.30 (dd, J1 = 2.0 Hz, J2= 6.8 Hz, 1 H), 6.10 (s, 2 H), 4.82 (s, 2 H), 3.73 (t, J = 6.8 Hz, 2 H), 3.21 (t, J = 6.8 Hz, 2 H).

### Biological test

The U937 cell line used in the following test examples was derived from ATCC, No.: CRL-1593.2, batch No.: 63479999, culture medium: RPMI-1640 + 10% FBS. The L929 cell line used in the following test examples was derived from ATCC, No.: CCL-1, batch No.: 70001022, culture medium: MEM + 10% FBS+ 1% PS. The reagents used, their suppliers, and item numbers are as follows: RPMI-1640, Gibco, 11875-093; FBS, Gibco, 10099-141; Trypsin-EDTA, Gibco, 25200-072; PS, Gibco, 15140-122; CellTiter Glo, Promega, G7573; DMSO, VWR AMRESCO, 0231-500ML; TNF-α protein (human, recombinant), Peprotech, 300-01A; Q-VD-Oph, MCE, HY-12305; V-bottom plate, Corning, 3894; 384-well low flange white flat bottom microplate, Corning, 3570; RIPK1, Eurofins, 16-022; MOPS, BDH, 441644J; EDTA, Sigma, E5134; myelin basic protein, Sigma, M1891-25.00 MG; magnesium acetate, Merck, DU008026; ATP (non-radioactive labelled), Sigma, A-7699; ATP (radioactive labelled), Hartmann Analytic, DU008054; phosphoric acid, Metlab, DU003000; Z-VAD: Shanghai Twochem, YA02401.

### Test Example 1: Inhibitory activity of compound of formula I against TNF-α-induced programmed cell necrosis

The test compound was dissolved in DMSO and diluted to a series of concentration gradients with DMSO. The U937 cells were inoculated in a 384-well white plate at 5000 cells/well. The compound at the corresponding concentration was added to each well, and was mixed with the cells evenly. Human TNF-α and Q-VD-Oph were added simultaneously to induce programmed necroptosis of the cells. The cells were placed in the incubator at 37°C and 5% CO₂ for further culture of 48 hours. Celltiter-Glo reagent was used for detection. After sufficient lysis reaction, the chemiluminescence readings were recorded by a microplate reader. The survival rate was calculated from the detection results using the equation as follows: SR (%) = (RLU compound - RLU blank) / (RLU high control - RLU blank) × 100%. The survival rate and the final concentration of the corresponding compound were plotted into a curve, using four-parameter fitting to calculate the inhibitory IC₅₀ of the compound against programmed necroptosis of cells induced by TNF-α. As a result, the compound of formula I has a high inhibitory activity against U937 cells with an IC₅₀ value of 6 nM.

### Test Example 2: Inhibitory activity of compound of formula I against RIPK1 enzyme

The test compound was dissolved in DMSO to prepare a 10 mM stock solution, and diluted 3.16-fold with DMSO to a series of gradient concentrations. Then, the solution was diluted 50-fold with MOPS buffer (pH 7.0) to prepare a working solution. The resulting working solution was mixed with 36 nM RIPK1 (final concentration) and 0.33 mg/mL substrate MBP evenly. Thereafter, 10 mM magnesium ion and 155 µM P³³ isotope-labeled ATP were added for reaction. The final concentration of DMSO was 2%. After the reaction was carried out at room temperature for 2 hours, phosphoric acid was added thereto to terminate the reaction. The final reaction system was detected using a liquid scintillation counter after treatment. The result after detection was subtracted from the blank control and converted to the percentage of activity compared with the reading of the control group. The percentage of activity and the final concentration of the compound were plotted into a curve, using four-parameter fitting, to obtain the inhibitory IC₅₀ of the compound against RIPK1 enzyme activity. As a result, the compound of formula I has a high inhibitory activity against RIPK1 with an IC₅₀ value of 39 nM.

### Test Example 3: Inhibitory activity of compound of formula I against TNF-α-induced programmed necrosis of L929 cells

The compound was dissolved in DMSO to prepare a 10 mM stock solution and diluted 3.16-fold with DMSO to a series of gradient concentrations. Then, the solution was diluted 100-fold with culture medium to prepare a working solution. The L929 cells were inoculated in a 384-well white plate at 10000 cells/well. The compound at the corresponding concentration was added to each well, and was mixed with the cells evenly. 30 ng/mL mouse TNF-α and 15 µM Z-VAD were added simultaneously to induce programmed necroptosis of the cells, and the final concentration of DMSO was 0.2%. The cells were placed in the incubator at 37°C and 5% CO₂ for further culture of 6 hours. Celltiter-Glo reagent was used for detection. After sufficient lysis reaction, the chemiluminescence readings were recorded by a microplate reader. The survival rate was calculated from the detection results using the equation as follows: SR (%) = (RLU compound - RLU blank) / (RLU high control - RLU blank) × 100%. The survival rate and the final concentration of the corresponding compound were plotted into a curve, using four-parameter fitting to calculate the inhibitory IC₅₀ of the compound against programmed necroptosis of cells induced by TNF-α. As a result, the compound of formula I has a high inhibitory activity against L929 cells with an IC₅₀ value of 3 nM.

### Example 1: Preparation of anhydrous crystal form A of compound of formula I (free base)

3-Bromo-6-(2-fluoro-5-(trifluoromethoxy)benzyl)-7,8-dihydro-1,6-naphthyridin-5(6*H*)-one (prepared in Steps 2 to 3 of Preparation Example 1, 106 g, 252.88 mmol), 7-(4,4,5,5-tetramethyl-1,3,2-dioxaborolan-2-yl)-[1,2,4]triazolo[1,5-*a*]pyridin-2-amine (prepared in Step 1 of Preparation Example 1, 111.82 g, 429.89 mmol), sodium carbonate (67 g, 632.2 mmol), and [1,1'-bis(diphenylphosphino)ferrocene]dichloropalladium(II) dichloromethane complex (10.3 g, 12.64 mmol) were dissolved in 1.5 L of dioxane and 300 mL of H₂O under nitrogen atmosphere, then the reaction system was replaced three times with nitrogen. The reaction mixture was reacted overnight at 100°C. The reaction mixture was cooled to room temperature, filtered with diatomite, and 20 g of activated carbon was added to the filtrate. The resulting mixture was heated to 100°C and stirred for 20 minutes. After filtration, the filtrate was concentrated to dryness, added with methanol (500 mL), slurried at room temperature, filtered, and the filter cake was dried for 2 hours at 50°C. The crude product was added with a solvent (30-fold the mass volume of dichloromethane/methanol = 2:1) and 15 g of activated carbon. The mixture was refluxed and stirred for 30 minutes, and then filtered while still hot. The filtrate was cooled and filtered, and the filter cake was slurried with methanol (500 mL). The slurry was filtered, and the filter cake was dried for 4 hours at 50°C to obtain the target product of 3-(2-amino-[1,2,4]triazolo[1,5-*a*]pyridin-7-yl)-6-(2-fluoro-5-(trifluoromethoxy)benzyl)-7,8-dihydro-1,6-naphthyridin-5(6*H*)-one (compound of formula I, 103 g, yield of 86.5%, purity of 99.03%).

The target product was characterized by XRPD, TGA, DSC, ¹H NMR, and HPLC. The XPRD is shown in Figure 1, and the sample is a crystal, named as free base anhydrous crystal form A. The X-ray powder diffraction data are shown in Table 6 below. The results of TGA/DSC and ¹H NMR (DMSO-*d₆*) are shown in Figures 2 and 3. The TGA results show a 1.1% weight loss of the sample before 175°C and the DSC results show two endothermic peaks at 191.6°C and 240.5°C (peak temperature).

**Table 6**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 10.94 | 8.09 | 100.00 | 14 | 22.32 | 3.98 | 9.12 |
| 2 | 13.24 | 6.68 | 1.83 | 15 | 23.47 | 3.79 | 6.87 |
| 3 | 14.64 | 6.05 | 2.31 | 16 | 23.85 | 3.73 | 5.23 |
| 4 | 15.24 | 5.82 | 2.62 | 17 | 24.50 | 3.63 | 5.74 |
| 5 | 16.43 | 5.39 | 96.13 | 18 | 24.88 | 3.58 | 4.53 |
| 6 | 16.77 | 5.29 | 6.01 | 19 | 25.78 | 3.46 | 3.24 |
| 7 | 17.86 | 4.97 | 7.07 | 20 | 26.91 | 3.31 | 6.40 |
| 8 | 18.13 | 4.89 | 8.29 | 21 | 27.55 | 3.24 | 9.01 |
| 9 | 18.41 | 4.82 | 8.85 | 22 | 28.58 | 3.12 | 3.42 |
| 10 | 19.12 | 4.64 | 11.23 | 23 | 28.98 | 3.08 | 3.16 |
| 11 | 19.81 | 4.48 | 10.56 | 24 | 29.49 | 3.03 | 2.42 |
| 12 | 20.23 | 4.39 | 8.52 | 25 | 33.21 | 2.70 | 7.08 |
| 13 | 21.25 | 4.18 | 9.13 | | | | |

### Example 2: Preparation of anhydrous crystal form B of compound of formula I (free base)

A heating test was performed on the free base anhydrous crystal form A of the compound of formula I obtained in Example 1, and the sample was heated to 210°C under nitrogen atmosphere using DSC and then cooled to room temperature to obtain the anhydrous crystal form B of the compound of formula I. The resulting solid was characterized by XRPD, TGA, DSC, and ¹H NMR (DMSO-*d₆*). The XRPD and TGA/DSC results are shown in Figures 4 and 5, and the X-ray powder diffraction data are shown in Table 7 below. The TGA results show a 2.2% weight loss of the sample before 200°C and the DSC results show one sharp endothermic peak at 240.1°C (peak temperature). The comparisons regarding NMR patterns of free base anhydrous crystal form B and free base anhydrous crystal form A are shown in Figure 6, and the results show that the NMR patterns of the two samples are consistent.

**Table 7**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 3.76 | 23.50 | 58.24 | 15 | 21.41 | 4.15 | 28.56 |
| 2 | 11.09 | 7.98 | 15.61 | 16 | 21.96 | 4.05 | 20.77 |
| 3 | 13.39 | 6.61 | 19.14 | 17 | 22.27 | 3.99 | 10.75 |
| 4 | 14.35 | 6.17 | 24.06 | 18 | 22.66 | 3.92 | 18.09 |
| 5 | 14.79 | 5.99 | 3.48 | 19 | 24.48 | 3.64 | 75.85 |
| 6 | 15.25 | 5.81 | 15.62 | 20 | 26.04 | 3.42 | 1.96 |
| 7 | 16.11 | 5.50 | 35.99 | 21 | 26.98 | 3.31 | 7.40 |
| 8 | 16.58 | 5.35 | 4.42 | 22 | 27.64 | 3.23 | 100.00 |
| 9 | 17.01 | 5.21 | 25.35 | 23 | 28.03 | 3.18 | 22.46 |
| 10 | 17.80 | 4.98 | 31.43 | 24 | 29.02 | 3.08 | 5.76 |
| 11 | 18.48 | 4.80 | 35.71 | 25 | 29.76 | 3.00 | 4.93 |
| 12 | 19.30 | 4.60 | 51.52 | 26 | 30.78 | 2.91 | 16.68 |
| 13 | 19.66 | 4.52 | 14.07 | 27 | 31.35 | 2.85 | 5.72 |
| 14 | 21.23 | 4.18 | 34.52 | | | | |

### Example 3: Preparation of crystal form A of hydrochloride salt of compound of formula I

A sample of free base anhydrous crystal form A of the compound of formula I (20 mg, 0.0424 mmol) obtained in Example 1 and a 2-fold molar amount of hydrochloric acid (3.09 mg, 0.085 mmol) were added to an HPLC vial, then 0.5 mL of methanol was added and mixed to obtain a suspension, and the resulting suspension was magnetically stirred (about 750 rpm) for about 5 days at room temperature, and magnetically stirred (about 750 rpm) for 4 days at 5°C. An anti-solvent (ethyl acetate) was added to the resulting clear solution, then the solid was separated, which was dried for one day at room temperature under vacuum to obtain the crystal form A of the hydrochloride salt of the compound of formula I. The molar ratio of Cl⁻ to API was determined to be 2.0:1 through the HPLC and IC test results. The XRPD, TGA/DSC, and ¹H NMR characterization results are shown in Figure 7, Figure 8, and Figure 9, respectively. The X-ray powder diffraction data for the crystal form A of the hydrochloride salt of the compound of formula I are shown in Table 8 below. TGA shows that the crystal form A of the hydrochloride salt has a 4.8% weight loss when heated to 100°C. DSC shows that the sample has one endothermic peak at 156.8°C (peak temperature).

**Table 8**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 4.39 | 20.11 | 16.40 | 13 | 22.21 | 4.00 | 16.65 |
| 2 | 8.83 | 10.01 | 100.00 | 14 | 24.25 | 3.67 | 26.06 |
| 3 | 9.99 | 8.86 | 3.55 | 15 | 26.66 | 3.34 | 18.59 |
| 4 | 13.24 | 6.69 | 37.66 | 16 | 27.30 | 3.27 | 6.87 |
| 5 | 15.78 | 5.61 | 3.72 | 17 | 28.02 | 3.18 | 2.92 |
| 6 | 18.31 | 4.85 | 20.67 | 18 | 28.38 | 3.14 | 4.86 |
| 7 | 19.54 | 4.54 | 8.08 | 19 | 29.69 | 3.01 | 3.00 |
| 8 | 19.71 | 4.51 | 7.05 | 20 | 31.19 | 2.87 | 8.99 |
| 9 | 20.23 | 4.39 | 4.04 | 21 | 32.11 | 2.79 | 5.38 |
| 10 | 20.70 | 4.29 | 3.06 | 22 | 33.46 | 2.68 | 1.38 |
| 11 | 21.06 | 4.22 | 2.45 | 23 | 35.77 | 2.51 | 7.99 |
| 12 | 21.52 | 4.13 | 4.40 | | | | |

### Example 4: Preparation of monohydrate crystal form C of sulfate salt of compound of formula I

The free base anhydrous crystal form A of the compound of formula I (6.15 g, 13.02 mmol) prepared in Example 1 and acetonitrile (62 mL) were added to a 100 mL round-bottom flask, and heated to 80°C in an oil bath. A 10% sulfuric acid aqueous solution (14 g, 14.29 mmol) was added dropwise thereto while stirring. The mixture was heated to reflux until the solid dissolved to clear, then naturally cooled to room temperature while stirring, filtered, and the filter cake was rinsed with acetonitrile and dried to obtain the monohydrate crystal form C of the sulfate salt of the compound of formula I (7.3 g, yield of 98%). The molar ratio of SO₄²⁻ to API was determined to be 1.0:1 through HPLC and IC results. The XRPD, TGA/DSC, and ¹H NMR (DMSO-*d₆*) characterization results are shown in Figure 16, Figure 17, and Figure 18, respectively, and their X-ray powder diffraction data are shown in Table 9 below. The ¹H NMR of the monohydrate crystal form C of the sulfate salt of the compound of formula I: δ 9.14 (d, *J*=2.4Hz, 1H), 8.85(d, *J*=7.2Hz, 1H), 8.58(d, *J*=2.4Hz, 1H), 7.97(d, *J*=1.6Hz, 1H), 7.64(d, *J*=2.0Hz, 1H), 7.44(m, 3H), 4.83(s, 2H), 3.76(t, *J*=6.8Hz, 2H), 3.24(t, *J*=6.8Hz, 2H). The TGA results show a 3.9% weight loss when heated to 175°C and the DSC results show an overlapping endothermic peak at 129.2°C (peak temperature) and 152.8°C (peak temperature), and a smaller endothermic peak at 200.2°C (peak temperature). ¹H NMR results show that no solvent residue is detected. The KF test results show that the water content of the sample is 3.18%.

**Table 9**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 5.55 | 15.91 | 100.00 | 21 | 21.90 | 4.06 | 10.46 |
| 2 | 9.90 | 8.93 | 2.10 | 22 | 22.92 | 3.88 | 48.68 |
| 3 | 11.42 | 7.75 | 6.46 | 23 | 23.25 | 3.83 | 4.71 |
| 4 | 11.65 | 7.60 | 5.38 | 24 | 23.77 | 3.74 | 5.49 |
| 5 | 12.52 | 7.07 | 24.06 | 25 | 24.43 | 3.64 | 5.02 |
| 6 | 12.75 | 6.94 | 17.53 | 26 | 25.16 | 3.54 | 18.61 |
| 7 | 13.70 | 6.46 | 9.31 | 27 | 26.06 | 3.42 | 9.57 |
| 8 | 14.72 | 6.02 | 88.71 | 28 | 26.36 | 3.38 | 8.66 |
| 9 | 15.77 | 5.62 | 17.21 | 29 | 26.96 | 3.31 | 11.19 |
| 10 | 16.75 | 5.29 | 70.41 | 30 | 27.35 | 3.26 | 53.24 |
| 11 | 17.64 | 5.03 | 11.46 | 31 | 28.70 | 3.11 | 2.09 |
| 12 | 18.48 | 4.80 | 15.94 | 32 | 29.79 | 3.00 | 2.45 |
| 13 | 19.16 | 4.63 | 14.66 | 33 | 30.26 | 2.95 | 5.12 |
| 14 | 19.45 | 4.56 | 21.08 | 34 | 31.34 | 2.85 | 2.07 |
| 15 | 19.63 | 4.52 | 32.13 | 35 | 32.20 | 2.78 | 7.32 |
| 16 | 19.96 | 4.45 | 20.90 | 36 | 33.14 | 2.70 | 0.96 |
| 17 | 20.37 | 4.36 | 29.39 | 37 | 38.14 | 2.36 | 3.66 |
| 18 | 20.72 | 4.29 | 25.99 | 38 | 38.58 | 2.33 | 3.07 |
| 19 | 20.90 | 4.25 | 42.69 | 39 | 38.99 | 2.31 | 1.69 |
| 20 | 21.13 | 4.20 | 33.41 | | | | |

### Example 5: Preparation of crystal form A of sulfate salt of compound of formula I

About 20 mg of the monohydrate crystal form C of the sulfate salt of the compound of formula I obtained in Example 4 was weighed and added to a 5 mL vial, dissolved with 0.1 to 1.5 mL of a good solvent of methanol, and 4.5 mL of an anti-solvent of 2-methyltetrahydrofuran was added dropwise to the clear solution while stirring. A clear solution was obtained after the addition of the anti-solvent, then solid precipitation was observed by stirring at room temperature for 3 days. The solid was separated through centrifugation and dried at room temperature to obtain the anhydrous crystal form A of the sulfate salt of the compound of formula I. The molar ratio of SO₄²⁻ to API was determined to be 1.0:1 through HPLC and IC results. The XRPD, TGA/DSC, and ¹H NMR characterization results are shown in Figure 10, Figure 11, and Figure 12. The X-ray powder diffraction data for the anhydrous crystal form A of the sulfate salt of the compound of formula I are shown in Table 10 below. TGA shows a 3.4% weight loss when heated to 175°C. DSC shows three endothermic peaks at 70.6°C, 123.3°C, and 205.1°C (peak temperature). ¹H NMR results show that no solvent residue is detected.

**Table 10**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 8.75 | 10.11 | 6.80 | 14 | 21.09 | 4.21 | 31.76 |
| 2 | 11.67 | 7.58 | 59.53 | 15 | 21.65 | 4.11 | 9.67 |
| 3 | 13.32 | 6.65 | 18.69 | 16 | 22.08 | 4.03 | 5.59 |
| 4 | 14.60 | 6.07 | 100.00 | 17 | 22.75 | 3.91 | 3.07 |
| 5 | 15.76 | 5.62 | 8.15 | 18 | 23.45 | 3.79 | 9.15 |
| 6 | 16.30 | 5.44 | 3.73 | 19 | 24.42 | 3.65 | 6.49 |
| 7 | 17.38 | 5.10 | 11.85 | 20 | 26.08 | 3.42 | 9.29 |
| 8 | 17.81 | 4.98 | 4.87 | 21 | 26.44 | 3.37 | 8.23 |
| 9 | 18.45 | 4.81 | 2.16 | 22 | 27.29 | 3.27 | 1.61 |
| 10 | 19.20 | 4.62 | 4.41 | 23 | 28.50 | 3.13 | 2.93 |
| 11 | 19.69 | 4.51 | 2.37 | 24 | 29.39 | 3.04 | 5.36 |
| 12 | 20.07 | 4.43 | 5.33 | 25 | 34.32 | 2.61 | 4.27 |
| 13 | 20.48 | 4.34 | 5.23 | 26 | 37.29 | 2.41 | 6.57 |

### Example 6: Preparation of crystal form A of sulfate salt of compound of formula I

The free base anhydrous crystal form A (20 mg, 0.0424 mmol) obtained in Example 1 and an equimolar amount of sulfuric acid (4.16 mg, 0.0424 mmol) were added to an HPLC vial, and 0.5 mL of methanol was added thereto, stirred in suspension for 5 days at room temperature, and the resulting solid was dried under vacuum at room temperature for 1 day. The solid was characterized by XRPD, TGA, DSC, and ¹H NMR, and each pattern was essentially the same as that in Example 5.

### Example 7: Preparation of crystal form B of sulfate salt of compound of formula I

About 20 mg of the monohydrate crystal form C of the sulfate salt of the compound of formula I obtained in Example 4 was weighed into a 3 mL vial. About 4 mL of dichloromethane solvent was added to another 20 mL vial. After the 3 mL vial was placed open-mouthed in the 20 mL vial, the 20 mL vial was then sealed. The solid was collected after standing for about 7 days at room temperature to obtain the crystal form B of the sulfate salt of the compound of formula I. The molar ratio of SO₄²⁻ to API was determined to be 1.1:1 through HPLC and IC results. The XRPD, TGA/DSC, and ¹H NMR results are shown in Figure 13, Figure 14, and Figure 15. The X-ray powder diffraction data for the crystal form B of the sulfate salt of the compound of formula I are shown in Table 11 below. TGA results show a 2.9% weight loss when heated to 150°C. DSC results show that the sample has three endothermic peaks at 77.9°C, 118.8°C, and 196.8°C (peak temperature). ¹H NMR results show that no solvent residue is detected.

**Table 11**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 3.13 | 28.22 | 70.78 | 20 | 20.27 | 4.38 | 43.95 |
| 2 | 6.14 | 14.40 | 11.20 | 21 | 21.03 | 4.22 | 25.76 |
| 3 | 8.68 | 10.19 | 9.29 | 22 | 21.69 | 4.10 | 10.66 |
| 4 | 10.24 | 8.64 | 5.45 | 23 | 22.38 | 3.97 | 11.42 |
| 5 | 11.72 | 7.55 | 4.30 | 24 | 23.28 | 3.82 | 47.88 |
| 6 | 12.20 | 7.25 | 23.13 | 25 | 23.67 | 3.76 | 27.20 |
| 7 | 13.89 | 6.37 | 5.39 | 26 | 24.18 | 3.68 | 20.51 |
| 8 | 14.82 | 5.98 | 28.53 | 27 | 24.82 | 3.59 | 32.61 |
| 9 | 15.17 | 5.84 | 32.16 | 28 | 25.15 | 3.54 | 100.00 |
| 10 | 15.45 | 5.74 | 16.40 | 29 | 25.77 | 3.46 | 16.73 |
| 11 | 15.76 | 5.62 | 8.42 | 30 | 26.36 | 3.38 | 11.47 |
| 12 | 16.33 | 5.43 | 17.05 | 31 | 27.28 | 3.27 | 10.66 |
| 13 | 17.09 | 5.19 | 18.10 | 32 | 27.97 | 3.19 | 9.60 |
| 14 | 17.21 | 5.15 | 19.64 | 33 | 28.76 | 3.10 | 10.63 |
| 15 | 17.51 | 5.06 | 17.39 | 34 | 29.44 | 3.03 | 10.87 |
| 16 | 18.34 | 4.84 | 25.04 | 35 | 29.87 | 2.99 | 9.85 |
| 17 | 18.71 | 4.74 | 78.14 | 36 | 30.76 | 2.91 | 8.63 |
| 18 | 19.05 | 4.66 | 25.25 | 37 | 31.18 | 2.87 | 4.00 |
| 19 | 19.92 | 4.46 | 14.11 | 38 | 32.62 | 2.75 | 3.83 |

### Example 8: Preparation of crystal form B of sulfate salt of compound of formula I

The free base anhydrous crystal form A (20 mg, 0.0424 mmol) obtained in Example 1 and an equimolar amount of sulfuric acid (4.16 mg, 0.0424 mmol) were added to an HPLC vial, and 0.5 mL of acetone was added thereto, stirred in suspension for 5 days at room temperature, and the resulting solid was dried under vacuum at room temperature for 1 day. The solid was characterized by XRPD, TGA, DSC, and ¹H NMR, and each pattern was essentially the same as that in Example 7, which was the crystal form B of the sulfate salt of the compound of formula I.

### Example 9: Preparation of monohydrate crystal form C of sulfate salt of compound of formula I

About 20 mg of the monohydrate crystal form C of the sulfate salt of the compound of formula I obtained in Example 4 was weighed into an HPLC vial. 0.6 mL of acetonitrile solvent was added thereto, respectively, and the resulting suspension was placed at 50°C and magnetically stirred (about 750 rpm) for about 3 days, and the solid was separated through centrifugation and characterized by XRPD, TGA, DSC, and ¹H NMR, and each pattern was essentially the same as that in Example 4, which was the monohydrate crystal form C of the sulfate salt of the compound of formula I.

### Example 10: Preparation of monohydrate crystal form C of sulfate salt of the compound of formula I

The free base anhydrous crystal form A (20 mg, 0.0424 mmol) obtained in Example 1 and an equimolar amount of sulfuric acid (4.16 mg, 0.0424 mmol) were added to an HPLC vial. 0.5 mL of acetonitrile/water (9:1, v/v) was added thereto, stirred in suspension for 5 days at room temperature, and the resulting solid was dried under vacuum at room temperature for 1 day. The solid was characterized by XRPD, TGA, DSC, and ¹H NMR, and each pattern was essentially the same as that in Example 4, which was the monohydrate crystal form C of the sulfate salt of the compound of formula I.

### Example 11: Preparation of crystal form A of citrate salt of compound of formula I

The free base anhydrous crystal form A (20 mg, 0.0424 mmol) obtained in Example 1 and an equimolar amount of anhydrous citrate acid (8.15 mg, 0.0424 mmol) were added to an HPLC vial, and 0.5 mL of ethyl acetate was added thereto, stirred in suspension for 5 days at room temperature, and the resulting solid was dried under vacuum for 1 day at room temperature to obtain the crystal form A of the citrate salt of the compound of formula I. The XRPD, TGA/DSC, and ¹H NMR characterization results are shown in Figure 19, Figure 20, and Figure 21. The X-ray powder diffraction data for the crystal form A of the citrate salt of the compound of formula I are shown in Table 12 below. TGA shows that the sample of the crystal form A of the citrate salt has a 1.1% weight loss when heated to 150°C. DSC shows that the sample has a sharp endothermic peak at 166.4°C. ¹H NMR results show that the molar ratio of citric acid to free base in the sample is 1.0:1.

**Table 12**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 4.45 | 19.86 | 43.67 | 17 | 22.98 | 3.87 | 2.89 |
| 2 | 8.91 | 9.93 | 27.96 | 18 | 23.29 | 3.82 | 4.47 |
| 3 | 11.14 | 7.94 | 42.81 | 19 | 23.86 | 3.73 | 7.35 |
| 4 | 13.38 | 6.62 | 20.78 | 20 | 24.25 | 3.67 | 15.34 |
| 5 | 14.97 | 5.92 | 57.89 | 21 | 24.82 | 3.59 | 5.93 |
| 6 | 16.11 | 5.50 | 9.15 | 22 | 25.56 | 3.49 | 16.13 |
| 7 | 17.01 | 5.21 | 45.63 | 23 | 26.32 | 3.39 | 2.14 |
| 8 | 17.45 | 5.08 | 53.44 | 24 | 27.43 | 3.25 | 3.16 |
| 9 | 17.87 | 4.96 | 100.00 | 25 | 27.78 | 3.21 | 8.80 |
| 10 | 19.08 | 4.65 | 42.58 | 26 | 28.48 | 3.13 | 10.45 |
| 11 | 19.50 | 4.55 | 5.01 | 27 | 29.20 | 3.06 | 5.10 |
| 12 | 20.17 | 4.40 | 3.74 | 28 | 29.99 | 2.98 | 9.33 |
| 13 | 20.64 | 4.30 | 7.40 | 29 | 30.57 | 2.92 | 0.98 |
| 14 | 21.00 | 4.23 | 15.49 | 30 | 31.30 | 2.86 | 3.23 |
| 15 | 21.23 | 4.18 | 20.58 | 31 | 32.30 | 2.77 | 1.28 |
| 16 | 21.50 | 4.13 | 7.26 | 32 | 33.90 | 2.64 | 2.20 |

### Example 12: Preparation of crystal form A of maleate salt of compound of formula I

The free base anhydrous crystal form A (20 mg, 0.0424 mmol) obtained in Example 1 and an equimolar amount of maleic acid (4.92 mg, 0.0424 mmol) were added to an HPLC vial, and 0.5 mL of acetone was added thereto, stirred in suspension for 5 days at room temperature, and the resulting solid was dried under vacuum for 1 day at room temperature to obtain the crystal form A of the maleate salt of the compound of formula I. The XRPD, TGA/DSC, and ¹H NMR characterization results are shown in Figure 22, Figure 23, and Figure 24. The X-ray powder diffraction data for the crystal form A of the maleate salt of the compound of formula I are shown in Table 13 below. TGA shows that the sample of the crystal form A of the maleate salt has a weight loss of 3.3% when heated to 150°C, and a weight loss of 13.6% when heated from 150°C to 250°C. DSC shows that the sample has two endothermic peaks at 176.0°C (starting temperature) and 210.7°C (peak temperature). ¹H NMR results show that the molar ratio of maleic acid to free base in the sample is 0.8:1.

**Table 13**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 4.58 | 19.30 | 90.80 | 11 | 20.26 | 4.38 | 22.26 |
| 2 | 10.97 | 8.07 | 80.55 | 12 | 21.38 | 4.16 | 8.79 |
| 3 | 11.55 | 7.66 | 5.39 | 13 | 22.35 | 3.98 | 23.07 |
| 4 | 13.18 | 6.72 | 3.53 | 14 | 22.91 | 3.88 | 78.43 |
| 5 | 15.04 | 5.89 | 11.79 | 15 | 24.64 | 3.61 | 23.45 |
| 6 | 16.47 | 5.38 | 100.00 | 16 | 26.52 | 3.36 | 12.98 |
| 7 | 18.20 | 4.87 | 35.73 | 17 | 27.52 | 3.24 | 9.95 |
| 8 | 18.86 | 4.71 | 9.88 | 18 | 28.43 | 3.14 | 14.06 |
| 9 | 19.56 | 4.54 | 16.98 | 19 | 32.28 | 2.77 | 3.72 |
| 10 | 19.87 | 4.47 | 25.91 | 20 | 33.24 | 2.70 | 9.29 |

### Example 13: Preparation of crystal form A of fumarate salt of compound of formula I

The free base anhydrous crystal form A (20 mg, 0.0424 mmol) obtained in Example 1 and an equimolar amount of anhydrous fumaric acid (4.92 mg, 0.0424 mmol) were added to an HPLC vial, and 0.5 mL of ethyl acetate was added thereto, stirred in suspension for 5 days at room temperature, and the resulting solid was dried under vacuum for 1 day at room temperature to obtain the crystal form A of the fumarate salt of the compound of formula I. The XRPD, TGA/DSC, and ¹H NMR characterization results are shown in Figure 25, Figure 26, and Figure 27. The X-ray powder diffraction data for the crystal form A of the fumarate salt of the compound of formula I are shown in Table 14 below. TGA shows that the crystal form A of the fumarate salt has a 1.8% weight loss when heated to 150°C. DSC shows that the sample has four endothermic peaks at 182.5°C, 192.9°C, 211.8°C, and 219.2°C (peak temperature) and three exothermic peaks at 123.0°C, 184.8°C, and 196.9°C (peak temperature). ¹H NMR results show that the molar ratio of fumaric acid to free base in the sample is 0.9:1 and no solvent residue is detected.

**Table 14**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 3.25 | 27.20 | 15.67 | 10 | 17.09 | 5.19 | 13.02 |
| 2 | 5.03 | 17.57 | 90.69 | 11 | 17.78 | 4.99 | 27.59 |
| 3 | 6.54 | 13.51 | 11.51 | 12 | 19.82 | 4.48 | 22.93 |
| 4 | 9.69 | 9.13 | 26.82 | 13 | 22.86 | 3.89 | 34.17 |
| 5 | 10.73 | 8.24 | 16.06 | 14 | 24.79 | 3.59 | 6.79 |
| 6 | 11.22 | 7.88 | 11.23 | 15 | 25.99 | 3.43 | 9.47 |
| 7 | 12.39 | 7.14 | 33.44 | 16 | 28.83 | 3.10 | 100.00 |
| 8 | 13.11 | 6.75 | 42.33 | 17 | 29.44 | 3.03 | 28.53 |
| 9 | 16.15 | 5.49 | 64.45 | | | | |

### Example 14: Preparation of crystal form A of methanesulfonate salt of compound of formula I

The free base anhydrous crystal form A (20 mg, 0.0424 mmol) obtained in Example 1 and an equimolar amount of methanesulfonic acid (4.08 mg, 0.0424 mmol) were added to an HPLC vial, and 0.5 mL of acetone was added thereto, stirred in suspension for 5 days at room temperature, and the resulting solid was dried under vacuum for 1 day at room temperature to obtain the crystal form A of the methanesulfonate salt of the compound of formula I. The XRPD, TGA/DSC, and ¹H NMR characterization results are shown in Figure 28, Figure 29, and Figure 30. The X-ray powder diffraction data for the crystal form A of the methanesulfonate salt are shown in Table 15 below. TGA shows that the crystal form A of the methanesulfonate salt has a 2.9% weight loss when heated to 110°C. DSC shows that the sample has two endothermic peaks at 92.5°C and 138.6°C (peak temperature). ¹H NMR results show that the molar ratio of methanesulfonic acid to free base in the sample is 1.0:1 and no solvent residue is detected.

**Table 15**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 5.11 | 17.29 | 80.69 | 16 | 18.43 | 4.81 | 48.53 |
| 2 | 5.76 | 15.34 | 100.00 | 17 | 19.60 | 4.53 | 57.43 |
| 3 | 7.51 | 11.78 | 61.29 | 18 | 20.29 | 4.38 | 73.91 |
| 4 | 8.63 | 10.24 | 31.71 | 19 | 20.49 | 4.34 | 73.12 |
| 5 | 9.71 | 9.11 | 34.24 | 20 | 20.98 | 4.23 | 38.73 |
| 6 | 10.57 | 8.37 | 24.94 | 21 | 21.72 | 4.09 | 57.98 |
| 7 | 12.43 | 7.12 | 29.25 | 22 | 22.27 | 3.99 | 41.18 |
| 8 | 12.84 | 6.90 | 41.20 | 23 | 22.60 | 3.93 | 27.05 |
| 9 | 13.37 | 6.62 | 26.67 | 24 | 23.18 | 3.84 | 17.97 |
| 10 | 14.75 | 6.00 | 93.05 | 25 | 24.56 | 3.62 | 18.72 |
| 11 | 15.51 | 5.71 | 54.54 | 26 | 25.75 | 3.46 | 40.39 |
| 12 | 15.86 | 5.59 | 47.11 | 27 | 26.67 | 3.34 | 34.82 |
| 13 | 16.51 | 5.37 | 60.30 | 28 | 27.63 | 3.23 | 31.47 |
| 14 | 16.84 | 5.26 | 64.99 | 29 | 28.27 | 3.16 | 16.39 |
| 15 | 17.54 | 5.06 | 77.22 | 30 | 28.89 | 3.09 | 12.22 |

### Example 15: Preparation of crystal form B of methanesulfonate salt of compound of formula I

The free base anhydrous crystal form A (20 mg, 0.0424 mmol) obtained in Example 1 and an equimolar amount of methanesulfonic acid (4.08 mg, 0.0424 mmol) were added to an HPLC vial, and 0.5 mL of ethyl acetate was added thereto, stirred in suspension for 5 days at room temperature, and the resulting solid was dried under vacuum for 1 day at room temperature to obtain the crystal form B of the methanesulfonate salt of the compound of formula I. The XRPD, TGA/DSC, and ¹H NMR characterization results are shown in Figure 31, Figure 32, and Figure 33. The X-ray powder diffraction data for the crystal form B of the methanesulfonate salt are shown in Table 16 below. TGA shows that the crystal form B of the methanesulfonate salt has a 2.8% weight loss when heated to 150°C. DSC shows that the sample has two endothermic peaks at 171.3°C and 194.7°C (peak temperature). ¹H NMR results show that the molar ratio of methanesulfonic acid to free base in the sample is 1.0:1 and no solvent residue is detected.

**Table 16**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 5.49 | 16.08 | 37.87 | 17 | 19.48 | 4.56 | 25.49 |
| 2 | 6.96 | 12.71 | 8.47 | 18 | 19.93 | 4.46 | 19.88 |
| 3 | 8.37 | 10.56 | 9.76 | 19 | 20.34 | 4.37 | 35.16 |
| 4 | 10.98 | 8.06 | 55.25 | 20 | 20.95 | 4.24 | 11.41 |
| 5 | 12.22 | 7.24 | 9.42 | 21 | 21.35 | 4.16 | 24.32 |
| 6 | 12.75 | 6.94 | 16.09 | 22 | 21.85 | 4.07 | 9.77 |
| 7 | 13.54 | 6.54 | 5.28 | 23 | 22.32 | 3.98 | 38.38 |
| 8 | 13.92 | 6.36 | 2.43 | 24 | 23.18 | 3.84 | 5.90 |
| 9 | 14.49 | 6.11 | 10.07 | 25 | 24.05 | 3.70 | 5.89 |
| 10 | 15.06 | 5.88 | 23.91 | 26 | 24.61 | 3.62 | 8.70 |
| 11 | 15.33 | 5.78 | 12.70 | 27 | 24.93 | 3.57 | 12.40 |
| 12 | 16.51 | 5.37 | 89.32 | 28 | 25.51 | 3.49 | 61.63 |
| 13 | 16.94 | 5.23 | 28.66 | 29 | 26.34 | 3.38 | 29.56 |
| 14 | 17.57 | 5.05 | 9.25 | 30 | 29.30 | 3.05 | 3.89 |
| 15 | 18.05 | 4.91 | 15.35 | 31 | 30.15 | 2.96 | 12.92 |
| 16 | 18.83 | 4.71 | 100.00 | | | | |

### Example 16: Preparation of crystal form C of methanesulfonate salt of compound of formula I

The free base anhydrous crystal form A (20 mg, 0.0424 mmol) obtained in Example 1 and an equimolar amount of methanesulfonic acid (4.08 mg, 0.0424 mmol) were added to an HPLC vial, and 0.5 mL of ACN/H₂O (9:1, v/v) was added thereto, stirred in suspension for 5 days at room temperature, and the resulting solid was dried under vacuum for 1 day at room temperature to obtain the crystal form C of the methanesulfonate salt of the compound of formula I. The XRPD, TGA/DSC, and ¹H NMR characterization results are shown in Figure 34, Figure 35, and Figure 36. The X-ray powder diffraction data for the crystal form C of the methanesulfonate salt of the compound of formula I are shown in Table 17 below. TGA shows that the crystal form C of the methanesulfonate salt has a 4.9% weight loss when heated to 150°C. DSC shows that the sample has three endothermic peaks at 108.2°C, 181.9°C, and 233.2°C (peak temperature). ¹H NMR results show that the molar ratio of methanesulfonic acid to free base in the sample is 0.3:1.

**Table 17**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 9.80 | 9.02 | 7.59 | 16 | 20.86 | 4.26 | 28.61 |
| 2 | 10.32 | 8.57 | 8.94 | 17 | 21.42 | 4.15 | 14.19 |
| 3 | 10.99 | 8.05 | 31.52 | 18 | 22.36 | 3.98 | 30.17 |
| 4 | 11.60 | 7.63 | 15.04 | 19 | 23.30 | 3.82 | 39.73 |
| 5 | 13.31 | 6.65 | 8.64 | 20 | 24.62 | 3.62 | 28.59 |
| 6 | 14.99 | 5.91 | 11.86 | 21 | 24.92 | 3.57 | 27.50 |
| 7 | 15.31 | 5.79 | 10.95 | 22 | 25.42 | 3.50 | 11.76 |
| 8 | 16.50 | 5.37 | 60.01 | 23 | 26.94 | 3.31 | 30.58 |
| 9 | 17.60 | 5.04 | 11.53 | 24 | 27.41 | 3.25 | 23.93 |
| 10 | 18.20 | 4.88 | 54.04 | 25 | 28.62 | 3.12 | 18.75 |
| 11 | 18.45 | 4.81 | 21.87 | 26 | 29.52 | 3.03 | 32.59 |
| 12 | 18.87 | 4.70 | 14.52 | 27 | 29.93 | 2.99 | 15.58 |
| 13 | 19.57 | 4.54 | 100.00 | 28 | 31.51 | 2.84 | 13.64 |
| 14 | 19.98 | 4.44 | 60.44 | 29 | 33.26 | 2.69 | 6.01 |
| 15 | 20.26 | 4.38 | 43.61 | | | | |

### Example 17: Preparation of crystal form A of p-toluenesulfonate salt of compound of formula I

The free base anhydrous crystal form A (20 mg, 0.0424 mmol) obtained in Example 1 and an equimolar amount of *p*-toluenesulfonic acid (7.30 mg, 0.0424 mmol) were added to an HPLC vial, and 0.5 mL of acetone was added thereto, stirred in suspension for 5 days at room temperature, and the resulting solid was dried under vacuum for 1 day at room temperature to obtain the crystal form A of the *p*-toluenesulfonate salt of the compound of formula I. The XRPD, TGA/DSC, and ¹H NMR characterization results are shown in Figure 37, Figure 38, and Figure 39. The X-ray powder diffraction data for the crystal form A of the *p-*toluenesulfonate salt of the compound of formula I are shown in Table 18 below. TGA shows that the crystal form A of the *p*-toluenesulfonate salt has a 1.6% weight loss when heated to 150°C. DSC shows that the sample has an endothermic peak at 205.6°C (peak temperature). ¹H NMR results show that the molar ratio of p-toluenesulfonic acid to free base in the sample is 0.9:1.

**Table 18**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 5.80 | 15.25 | 100.00 | 20 | 23.07 | 3.86 | 7.64 |
| 2 | 10.90 | 8.11 | 9.50 | 21 | 23.24 | 3.83 | 9.72 |
| 3 | 11.57 | 7.65 | 64.05 | 22 | 23.80 | 3.74 | 6.05 |
| 4 | 12.20 | 7.26 | 3.54 | 23 | 24.04 | 3.70 | 9.90 |
| 5 | 13.07 | 6.77 | 15.72 | 24 | 25.06 | 3.55 | 6.69 |
| 6 | 14.03 | 6.31 | 7.07 | 25 | 25.35 | 3.51 | 5.88 |
| 7 | 14.81 | 5.98 | 49.77 | 26 | 25.52 | 3.49 | 8.05 |
| 8 | 15.18 | 5.84 | 18.50 | 27 | 26.30 | 3.39 | 3.79 |
| 9 | 16.27 | 5.45 | 11.22 | 28 | 27.22 | 3.28 | 63.06 |
| 10 | 16.83 | 5.27 | 25.96 | 29 | 27.91 | 3.20 | 25.11 |
| 11 | 17.39 | 5.10 | 8.07 | 30 | 29.16 | 3.06 | 11.84 |
| 12 | 18.16 | 4.89 | 16.46 | 31 | 29.94 | 2.98 | 7.54 |
| 13 | 18.47 | 4.80 | 14.26 | 32 | 30.66 | 2.92 | 15.05 |
| 14 | 18.54 | 4.78 | 13.84 | 33 | 31.75 | 2.82 | 1.98 |
| 15 | 19.21 | 4.62 | 17.03 | 34 | 32.47 | 2.76 | 14.78 |
| 16 | 19.70 | 4.51 | 88.28 | 35 | 33.07 | 2.71 | 10.72 |
| 17 | 21.28 | 4.18 | 6.53 | 36 | 34.76 | 2.58 | 2.24 |
| 18 | 21.91 | 4.06 | 4.56 | 37 | 38.01 | 2.37 | 2.46 |
| 19 | 22.36 | 3.98 | 70.11 | | | | |

### Example 18: Preparation of crystal form A of cyclamate salt of compound of formula I

The free base anhydrous crystal form A (20 mg, 0.0424 mmol) obtained in Example 1 and an equimolar amount of cyclamic acid (7.60 mg, 0.0424 mmol) were added to an HPLC vial, and 0.5 mL of ethyl acetate was added thereto, stirred in suspension for 5 days at room temperature, and the resulting solid was dried under vacuum for 1 day at room temperature to obtain the crystal form A of the cyclamate salt of the compound of formula I. The XRPD, TGA/DSC, and ¹H NMR characterization results are shown in Figure 40, Figure 41, and Figure 42. The X-ray powder diffraction data for the crystal form A of the cyclamate salt of the compound of formula I are shown in Table 19 below. TGA shows that the crystal form A of the cyclamate salt has a 0.7% weight loss when heated to 150°C. DSC shows that the sample has two endothermic peaks at 202.3°C and 231.3°C (peak temperature) and two exothermic peaks at 118.2°C and 205.4°C (peak temperature). ¹H NMR results show that the molar ratio of cyclamic acid to free base in the sample is 0.9:1 and no solvent residue is detected.

**Table 19**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 6.41 | 13.78 | 100.00 | 14 | 22.33 | 3.98 | 23.03 |
| 2 | 10.95 | 8.08 | 38.70 | 15 | 23.27 | 3.82 | 19.87 |
| 3 | 13.36 | 6.63 | 2.29 | 16 | 24.63 | 3.61 | 18.66 |
| 4 | 14.23 | 6.22 | 1.81 | 17 | 24.93 | 3.57 | 13.15 |
| 5 | 14.98 | 5.92 | 6.95 | 18 | 25.41 | 3.51 | 9.10 |
| 6 | 15.27 | 5.80 | 6.94 | 19 | 25.86 | 3.45 | 4.89 |
| 7 | 16.47 | 5.38 | 63.33 | 20 | 26.49 | 3.36 | 9.80 |
| 8 | 18.16 | 4.89 | 34.12 | 21 | 26.94 | 3.31 | 17.91 |
| 9 | 18.57 | 4.78 | 40.70 | 22 | 27.39 | 3.26 | 16.19 |
| 10 | 19.58 | 4.53 | 32.20 | 23 | 28.61 | 3.12 | 8.60 |
| 11 | 20.24 | 4.39 | 30.21 | 24 | 29.55 | 3.02 | 9.28 |
| 12 | 20.83 | 4.26 | 13.74 | 25 | 31.55 | 2.84 | 5.73 |
| 13 | 21.39 | 4.15 | 14.56 | 26 | 33.22 | 2.70 | 5.99 |

### Example 19: Preparation of crystal form D of sulfate salt of compound of formula I

About 20 mg of the monohydrate crystal form C of the sulfate salt of the compound of formula I obtained in Example 4 was weighed into an HPLC vial. 0.6 mL of n-propanol was added thereto, respectively, and the resulting suspension was placed at 50°C and magnetically stirred (about 750 rpm) for about 3 days, and the solid was separated through centrifugation to obtain the anhydrous crystal form D of the sulfate salt of the compound of formula I. The molar ratio of SO₄²⁻ to API was determined to be 1.1:1 through the HPLC and IC tests. The XRPD, TGA/DSC, and ¹H NMR results are shown in Figure 43, Figure 44, and Figure 45. The X-ray powder diffraction data for the anhydrous crystal form D of the sulfate salt of the compound of formula I are shown in Table 20 below. TGA results show a 3.0% weight loss when heated to 125°C. DSC results show that the sample has three endothermic peaks at 111.9°C, 153.2°C, and 197.7°C (peak temperature). The results show that no solvent residue is detected.

**Table 20**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 6.09 | 14.50 | 13.91 | 18 | 24.03 | 3.70 | 14.32 |
| 2 | 6.59 | 13.42 | 26.06 | 19 | 24.63 | 3.61 | 20.98 |
| 3 | 9.83 | 9.00 | 19.40 | 20 | 25.31 | 3.52 | 93.44 |
| 4 | 11.71 | 7.56 | 6.59 | 21 | 25.63 | 3.48 | 20.57 |
| 5 | 13.18 | 6.72 | 27.73 | 22 | 27.27 | 3.27 | 9.25 |
| 6 | 14.60 | 6.07 | 100.00 | 23 | 27.56 | 3.24 | 15.35 |
| 7 | 15.02 | 5.90 | 25.13 | 24 | 28.22 | 3.16 | 7.34 |
| 8 | 16.01 | 5.54 | 15.34 | 25 | 29.24 | 3.05 | 6.37 |
| 9 | 16.46 | 5.39 | 10.23 | 26 | 29.71 | 3.01 | 9.10 |
| 10 | 16.82 | 5.27 | 8.43 | 27 | 30.27 | 2.95 | 6.08 |
| 11 | 17.36 | 5.11 | 3.07 | 28 | 31.39 | 2.85 | 24.05 |
| 12 | 18.15 | 4.89 | 34.49 | 29 | 32.49 | 2.76 | 4.79 |
| 13 | 19.31 | 4.60 | 44.24 | 30 | 33.42 | 2.68 | 5.23 |
| 14 | 19.85 | 4.47 | 89.09 | 31 | 34.78 | 2.58 | 5.72 |
| 15 | 20.07 | 4.42 | 70.14 | 32 | 35.66 | 2.52 | 2.29 |
| 16 | 21.06 | 4.22 | 44.48 | 33 | 36.91 | 2.44 | 3.80 |
| 17 | 22.14 | 4.02 | 22.32 | 34 | 39.13 | 2.30 | 3.22 |

### Example 20: Preparation of crystal form I of sulfate salt of compound of formula I

The sample of the monohydrate crystal form C of the sulfate salt obtained in Example 4 was heated to 130°C under nitrogen atmosphere and then cooled to 30°C to obtain the crystal form I of the sulfate salt of the compound of formula I. The XRPD results are shown in Figure 46. The X-ray powder diffraction data for the crystal form I of the sulfate salt of the compound of formula I are shown in Table 21 below.

**Table 21**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 6.71 | 13.17 | 6.92 | 20 | 20.79 | 4.27 | 49.41 |
| 2 | 11.22 | 7.89 | 6.61 | 21 | 21.09 | 4.21 | 67.60 |
| 3 | 11.70 | 7.57 | 8.99 | 22 | 21.61 | 4.11 | 38.55 |
| 4 | 12.92 | 6.85 | 100.00 | 23 | 21.75 | 4.09 | 48.54 |
| 5 | 13.83 | 6.40 | 14.04 | 24 | 22.28 | 3.99 | 24.45 |
| 6 | 14.33 | 6.18 | 16.49 | 25 | 23.02 | 3.86 | 77.21 |
| 7 | 14.60 | 6.07 | 18.87 | 26 | 23.28 | 3.82 | 34.78 |
| 8 | 14.99 | 5.91 | 63.53 | 27 | 23.95 | 3.72 | 23.78 |
| 9 | 15.47 | 5.73 | 27.45 | 28 | 24.61 | 3.62 | 51.87 |
| 10 | 15.86 | 5.59 | 84.19 | 29 | 25.31 | 3.52 | 34.60 |
| 11 | 16.30 | 5.44 | 20.86 | 30 | 26.14 | 3.41 | 28.64 |
| 12 | 17.26 | 5.14 | 61.23 | 31 | 26.62 | 3.35 | 33.93 |
| 13 | 17.63 | 5.03 | 26.35 | 32 | 26.90 | 3.32 | 90.44 |
| 14 | 18.31 | 4.84 | 45.56 | 33 | 27.73 | 3.22 | 20.40 |
| 15 | 18.47 | 4.80 | 45.14 | 34 | 28.08 | 3.18 | 20.31 |
| 16 | 19.04 | 4.66 | 28.96 | 35 | 28.36 | 3.15 | 19.93 |
| 17 | 19.34 | 4.59 | 56.16 | 36 | 28.73 | 3.11 | 17.96 |
| 18 | 19.75 | 4.50 | 38.53 | 37 | 30.23 | 2.96 | 9.51 |
| 19 | 20.08 | 4.42 | 36.23 | 38 | 31.24 | 2.86 | 9.79 |

### Example 21: Preparation of crystal form E of sulfate salt of compound of formula I

About 20 mg of the monohydrate crystal form C of the sulfate salt of the compound of formula I obtained in Example 4 was weighed into an HPLC vial. 0.5 to 1.0 mL of (EtOAc/H₂O (19:1, v/v)) solvent was added thereto, respectively, and the resulting suspension was placed at room temperature and magnetically stirred (about 750 rpm) for about 3 days, and the solid was separated through centrifugation to obtain the crystal form E of the sulfate salt of the compound of formula I. The molar ratio of SO₄²⁻ to API was determined to be 1.3:1 through HPLC and IC results. The XRPD, TGA/DSC, and ¹H NMR results are shown in Figure 47, Figure 48, and Figure 49. The X-ray powder diffraction data for the crystal form E of the sulfate salt of the compound of formula I are shown in Table 22 below. TGA results show a 5.9% weight loss when heated to 150°C. DSC results show that the sample has three endothermic peaks at 82.2°C, 138.6°C, and 205.4°C (peak temperature).

**Table 22**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 5.52 | 16.00 | 28.51 | 16 | 19.76 | 4.49 | 46.70 |
| 2 | 7.85 | 11.26 | 36.30 | 17 | 20.28 | 4.38 | 74.12 |
| 3 | 8.81 | 10.04 | 31.32 | 18 | 20.79 | 4.27 | 37.21 |
| 4 | 10.49 | 8.43 | 21.91 | 19 | 21.90 | 4.06 | 38.80 |
| 5 | 11.46 | 7.72 | 44.16 | 20 | 22.48 | 3.96 | 31.96 |
| 6 | 11.70 | 7.56 | 32.64 | 21 | 23.00 | 3.87 | 38.17 |
| 7 | 12.75 | 6.94 | 96.77 | 22 | 23.45 | 3.79 | 55.29 |
| 8 | 14.32 | 6.19 | 92.12 | 23 | 25.25 | 3.53 | 24.62 |
| 9 | 15.14 | 5.85 | 35.79 | 24 | 25.88 | 3.44 | 39.53 |
| 10 | 15.63 | 5.67 | 25.08 | 25 | 26.20 | 3.40 | 47.42 |
| 11 | 16.39 | 5.41 | 100.00 | 26 | 28.25 | 3.16 | 24.04 |
| 12 | 17.47 | 5.08 | 47.08 | 27 | 30.17 | 2.96 | 8.14 |
| 13 | 17.76 | 4.99 | 55.45 | 28 | 31.30 | 2.86 | 8.63 |
| 14 | 18.25 | 4.86 | 37.05 | 29 | 35.91 | 2.50 | 3.91 |
| 15 | 19.06 | 4.66 | 28.36 | 30 | 36.93 | 2.43 | 7.10 |

### Example 22: Preparation of crystal form F of sulfate salt of compound of formula I

About 20 mg of the monohydrate crystal form C of the sulfate salt of the compound of formula I obtained in Example 4 was weighed into a 3 mL vial. About 4 mL of DMSO solvent was added to another 20 mL vial. After the 3 mL vial was placed open-mouthed in the 20 mL vial, the 20 mL vial was then sealed. The solid was collected after standing for about 7 days at room temperature to obtain the crystal form F of the sulfate salt of the compound of formula I. The molar ratio of SO₄²⁻ to API was determined to be 1.1:1 through HPLC and IC results. The XRPD, TGA/DSC, and ¹H NMR results are shown in Figure 50, Figure 51, and Figure 52. The X-ray powder diffraction data for the crystal form F of the sulfate salt of the compound of formula I are shown in Table 23 below. TGA results show a weight loss of 6.4% when heated to 160°C and a weight loss of 6.9% when heated from 160°C to 225°C. DSC results show that the sample has two endothermic peaks at 123.6°C and 185.2°C (peak temperature), and one exothermic peak at 206.0°C (peak temperature).

**Table 23**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 4.75 | 18.60 | 7.92 | 27 | 22.74 | 3.91 | 13.16 |
| 2 | 7.61 | 11.62 | 2.08 | 28 | 23.10 | 3.85 | 33.03 |
| 3 | 9.41 | 9.40 | 8.72 | 29 | 24.14 | 3.68 | 41.22 |
| 4 | 10.75 | 8.23 | 17.10 | 30 | 24.26 | 3.67 | 52.67 |
| 5 | 11.16 | 7.93 | 10.42 | 31 | 24.91 | 3.57 | 100.00 |
| 6 | 12.01 | 7.37 | 4.20 | 32 | 25.33 | 3.52 | 14.69 |
| 7 | 12.89 | 6.87 | 21.92 | 33 | 25.92 | 3.44 | 7.63 |
| 8 | 13.46 | 6.58 | 3.62 | 34 | 26.30 | 3.39 | 6.85 |
| 9 | 14.10 | 6.28 | 15.64 | 35 | 27.02 | 3.30 | 13.88 |
| 10 | 14.96 | 5.92 | 60.75 | 36 | 27.62 | 3.23 | 54.99 |
| 11 | 15.29 | 5.80 | 29.26 | 37 | 28.29 | 3.16 | 27.84 |
| 12 | 16.04 | 5.53 | 53.64 | 38 | 29.11 | 3.07 | 3.11 |
| 13 | 16.74 | 5.30 | 22.30 | 39 | 30.13 | 2.97 | 5.26 |
| 14 | 17.01 | 5.21 | 37.87 | 40 | 30.46 | 2.93 | 6.63 |
| 15 | 17.84 | 4.97 | 8.16 | 41 | 31.04 | 2.88 | 8.26 |
| 16 | 18.30 | 4.85 | 2.75 | 42 | 31.46 | 2.84 | 10.56 |
| 17 | 18.63 | 4.76 | 39.92 | 43 | 32.19 | 2.78 | 3.61 |
| 18 | 19.07 | 4.65 | 5.91 | 44 | 32.63 | 2.74 | 3.05 |
| 19 | 19.73 | 4.50 | 31.58 | 45 | 33.20 | 2.70 | 5.01 |
| 20 | 19.94 | 4.45 | 32.43 | 46 | 33.74 | 2.66 | 9.72 |
| 21 | 20.12 | 4.41 | 27.26 | 47 | 34.42 | 2.61 | 2.82 |
| 22 | 20.60 | 4.31 | 69.30 | 48 | 35.63 | 2.52 | 2.66 |
| 23 | 20.94 | 4.24 | 9.02 | 49 | 36.50 | 2.46 | 1.72 |
| 24 | 21.53 | 4.13 | 36.36 | 50 | 37.71 | 2.39 | 4.35 |
| 25 | 22.11 | 4.02 | 13.66 | 51 | 38.55 | 2.34 | 3.76 |
| 26 | 22.53 | 3.95 | 16.81 | 52 | 39.03 | 2.31 | 1.80 |

### Example 23: Preparation of crystal form C of compound of formula I

About 20 mg of the monohydrate crystal form C of the sulfate salt of the compound of formula I obtained in Example 4 was weighed into a 3 mL vial. The solid was dissolved to clear in about 2.0 mL (EtOH/DMF (9: 1, v/v)) of solvent, and about 2 mg of mixed polymer (a mixture of polycaprolactone, polyethylene glycol, polymethyl methacrylate, sodium alginate, and hydroxypropyl cellulose with equal weights of each component) was added thereto. The vial containing the filtrate was sealed with a sealing film, and 3-5 small holes were punched in the film. The vial was then placed at room temperature for slow volatilization. The resulting solid was collected to obtain the free base crystal form C of the compound of formula I. The X-ray powder diffraction data for the free base crystal form C of the compound of formula I are shown in Table 24 below. The XRPD and TGA/DSC results are shown in Figure 53 and Figure 54, showing that the sample has a weight loss of 3.2% when heated to 150°C; there are three endothermic peaks at 64.9°C, 200.1°C, and 237.0°C (peak temperature).

**Table 24**

| Peak number | 2θ (degrees) | d-Spacing (Å) | Relative intensity (%) | Peak number | 2θ (degrees) | d-Spacing (Å) | Relative intensity (%) |
|---|---|---|---|---|---|---|---|
| 1 | 9.77 | 9.05 | 5.87 | 17 | 22.96 | 3.87 | 5.23 |
| 2 | 10.11 | 8.75 | 8.88 | 18 | 23.30 | 3.82 | 16.50 |
| 3 | 10.36 | 8.54 | 5.62 | 19 | 23.92 | 3.72 | 5.22 |
| 4 | 10.98 | 8.06 | 5.74 | 20 | 24.57 | 3.62 | 3.86 |
| 5 | 11.60 | 7.63 | 6.01 | 21 | 24.93 | 3.57 | 7.78 |
| 6 | 13.29 | 6.66 | 2.40 | 22 | 25.53 | 3.49 | 1.18 |
| 7 | 16.48 | 5.38 | 5.13 | 23 | 26.96 | 3.31 | 5.67 |
| 8 | 17.58 | 5.05 | 0.66 | 24 | 27.45 | 3.25 | 1.11 |
| 9 | 18.27 | 4.86 | 1.21 | 25 | 28.68 | 3.11 | 3.54 |
| 10 | 19.55 | 4.54 | 100.00 | 26 | 29.51 | 3.03 | 37.17 |
| 11 | 19.99 | 4.44 | 35.39 | 27 | 29.92 | 2.99 | 21.88 |
| 12 | 20.26 | 4.38 | 25.99 | 28 | 31.49 | 2.84 | 10.94 |
| 13 | 20.57 | 4.32 | 11.03 | 29 | 32.56 | 2.75 | 1.50 |
| 14 | 20.82 | 4.27 | 7.43 | 30 | 33.29 | 2.69 | 0.71 |
| 15 | 21.53 | 4.13 | 5.35 | 31 | 33.82 | 2.65 | 0.77 |
| 16 | 22.40 | 3.97 | 4.73 | | | | |

### Solubility

The rough solubility of the free base anhydrous crystal form A of the compound of formula I obtained in Example 1 and the monohydrate crystal form C of the sulfate salt of the compound of formula I obtained in Example 4 was tested in different solvent systems at room temperature. About 2 mg of the solid sample was weighed into an HPLC vial, and then the corresponding solvent was gradually (50/50/200/700 µL in sequence) added thereto and shaken until the solid dissolved to clear. If the sample was still not dissolved to clear after the addition of 1 mL of solvent, no more solvent would be added. The rough solubility ranges calculated from the mass of the solid sample, the volume of the added solvent, and the observed dissolution phenomena are shown in Table 25-1 and Table 25-2.

**Table 25-1 Rough solubility at room temperature of the free base anhydrous crystal form A of the compound of formula I obtained in Example 1**

| **Solvent** | **Solubility (mg/mL)** | **Solvent** | **Solubility (mg/mL)** |
|---|---|---|---|
| Methanol | S<1.8 | Acetonitrile | S<1.9 |
| Ethanol | S<2.2 | *n*-Heptane | S<1.7 |
| Isopropanol | S<1.9 | Water | S<2.1 |
| Acetone | S<1.8 | Dimethyl sulfoxide* | 7.7<S<23.0 |

| | | | |
|---|---|---|---|
| *: Dissolve to clear at 50°C. | | | |

**Table 25-2 Rough solubility at room temperature of the monohydrate crystal form C of the sulfate salt of the compound of formula I obtained in Example 4**

| **Solvent** | **Solubility (mg/mL)** | **Solvent** | **Solubility (mg/mL)** |
|---|---|---|---|
| Methanol | 7.3<S<22.0 | Acetonitrile | S<2.4 |
| Ethanol | 2.0<S<6.7 | *n*-Heptane | S<2.3 |
| Isopropanol* | S<2.4 | Water | S<2.4 |
| Acetone | S<2.4 | Dimethyl sulfoxide | S>42.0 |

| | | | |
|---|---|---|---|
| *: Dissolve to clear at 50°C. | | | |

### Study on the transformation relationship between anhydrous crystal form/hydrate crystal form

In order to further study the transformation relationship between the anhydrous crystal form/hydrate crystal form of the sulfate salt.

A suspension competition test between the anhydrous crystal form A of the sulfate salt obtained in Example 5 and the anhydrous crystal form D of the sulfate salt obtained in Example 19 was set up, including conditions of 5°C, 25°C, and 50°C in methanol and 25°C in ACN. (Test numbers are from A1 to A4)

A suspension competition test between the monohydrate crystal form C of the sulfate salt obtained in Example 4 and the anhydrous crystal form A of the sulfate salt obtained in Example 5 was set up at room temperature, including a suspension competition test in ACN/H₂O (a_{w} = 0/0.2/0.4/0.6/.0.8) of different water activities at room temperature. (Test numbers are from B1 to B5)

The specific steps are as follows: 1) saturated solutions of sulfate salt in different solvent systems at room temperature were prepared; 2) equal mass of the corresponding samples of crystal form of sulfate salt (about 5 mg each) were added to 1 mL of saturated solution to form a suspension, respectively; 3) the suspension was magnetically stirred at corresponding temperature; 4) the remaining solid was separated and tested by XRPD.

The test results are summarized in Table 26. In the suspension competition test between anhydrous crystal forms in different solvent systems and under different temperature conditions, anhydrous crystal form A was obtained after stirring; in the room temperature suspension competition test between hydrate and room temperature stable anhydrous crystal form A, anhydrous crystal form A was obtained after stirring in a solvent system with a water activity of about 0; hydrate crystal form C was obtained between water activities of about 0.2 and about 0.8, indicating that the key water activity point for the transition between anhydrous crystal form A and hydrate crystal form C was between 0 and 0.2; after stirring in water (a_{w} of about 1), free base crystal form A was obtained, indicating that sulfate was prone to divergence under high water activity (about 1) conditions.

Based on the results of screening and study on thermodynamic relationships, the thermodynamic stability range of monohydrate crystal form C of sulfate is relatively wide under different water activities at room temperature.

**Table 26 Suspension competition test results for crystal form of sulfate salt**

| **Test number** | **Solvent (v:v)** | Temperature (°C) | **water activity a_{w}** | **Result** |
|---|---|---|---|---|
| A1 | MeOH | 5 | About 0 | Anhydrous crystal form A of sulfate salt |
| A2 | MeOH | 25 | About 0 | Anhydrous crystal form A of sulfate salt |
| A3 | MeOH | 50 | About 0 | Anhydrous crystal form A of sulfate salt |
| A4 | ACN | 25 | About 0 | Anhydrous crystal form A of sulfate salt |
| B1 | ACN | 25 | About 0 | Anhydrous crystal form A of sulfate salt |
| B2 | ACN/H₂O (991:9) | 25 | About 0.2 | Monohydrate crystal form C of sulfate salt |
| B3 | ACN/H₂O (978:22) | 25 | About 0.4 | Monohydrate crystal form C of sulfate salt |
| B4 | ACN/H₂O (96:4) | 25 | About 0.6 | Monohydrate crystal form C of sulfate salt |
| B5 | ACN/H₂O (926:74) | 25 | About 0.8 | Monohydrate crystal form C of sulfate salt |
| B6 | H₂O | 25 | About 1 | Free base crystal form A |

### Hygroscopicity

The hygroscopicity of the sample of the monohydrate crystal form C of the sulfate salt obtained in Example 9 was evaluated through the DVS test from 0%RH to 95%RH at 25°C. The DVS results are shown in Figure 55. As shown in the results, the hygroscopic weight gain of the sample of the monohydrate crystal form C of the sulfate salt is about 0.27% at 25°C/80% RH, indicating that the sample is slightly hygroscopic. The XRPD results show that no crystal form transition in the sample of the monohydrate crystal form C of the sulfate salt after the DVS test.

### Solid state stability

Appropriate amounts of the samples of the monohydrate crystal form C of the sulfate salt obtained in Example 9 or Example 4 were respectively weighed and placed in a sealed state for 24 hours at 60°C, and then placed for one week in an open state of 40°C/75%RH. Solid samples separated under different conditions were respectively subjected to XRPD test to evaluate the transition of crystal forms, and subjected to HPLC test to evaluate purity and chemical stability. The evaluation results are summarized in Table 27. The XRPD comparison results of the monohydrate crystal form C of the sulfate salt before and after the stability test show that the two batches of samples of the monohydrate crystal form C of the sulfate salt do not show any significant decrease in HPLC purity under the two test conditions, and the crystal form does not change, indicating that it has good physical and chemical stability under the test conditions.

**Table 27 Solid state stability evaluation results of monohydrate crystal form C of sulfate salt**

| **Sample** | **Initial purity** (%) | **60°C/sealed/1 week** | | | **40°C/75% RH/1 week** | | |
|---|---|---|---|---|---|---|---|
| | | Purity (%) | Purity/initial purity (%) | Crystal form transition | Purity (%) | Purity/ initial purity (%) | Crystal form transition |
| Example 4 | 99.48 | 99.47 | 99.99 | No | 99.46 | 99.98 | No |
| Example 9 | 99.52 | 99.39 | 99.87 | No | 99.45 | 99.93 | No |

All literatures mentioned in the present disclosure are incorporated by reference in the present disclosure to the same extent as if each individual literature is individually incorporated by reference. In addition, it should be understood that, after reading the above teachings of the present disclosure, a person skilled in the art can make various modifications or changes to the present disclosure, and these equivalent forms fall within the scope of the claims attached to the present disclosure.

## Claims

1. A crystal form of a compound of formula I, wherein the crystal form of the compound of formula I is a crystal form **A** of the compound of formula I, a crystal form **B** of the compound of formula I, or a crystal form **C** of the compound of formula I; 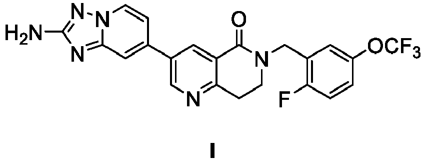
the crystal form **A** of the compound of formula I has an X-ray powder diffraction pattern expressed at a 2θ angle comprising diffraction peaks at the following 2θ angles: 10.94 ± 0.2°, 16.43 ± 0.2°, 19.12 ± 0.2°, and 19.81 ± 0.2°;
the crystal form **B** of the compound of formula I has an X-ray powder diffraction pattern expressed at a 2θ angle comprising diffraction peaks at the following 2θ angles: 27.64 ± 0.2°, 24.48 ± 0.2°, 3.76 ± 0.2°, and 19.30 ± 0.2°;
the crystal form **C** of the compound of formula I has an X-ray powder diffraction pattern expressed at a 2θ angle comprising diffraction peaks at the following 2θ angles: 19.55 ± 0.2°, 29.51 ± 0.2°, 19.99 ± 0.2°, and 20.26 ± 0.2°.

2. The crystal form of the compound of formula I according to claim 1, wherein
when the crystal form of the compound of formula I is the crystal form **A** of the compound of formula I, the crystal form **A** of the compound of formula I satisfies one or more than one of the following conditions:
(1) the crystal form **A** of the compound of formula I has an X-ray powder diffraction pattern expressed at a 2θ angle further comprising diffraction peaks at one or more than one of the following 2θ angles: 21.25 ± 0.2°, 22.32 ± 0.2°, 27.55 ± 0.2°, 20.23 ± 0.2°, and 18.41 ± 0.2°;
(2) the crystal form **A** of the compound of formula I has a thermogravimetric analysis pattern with a weight loss of 0.5%-2% when heated from an onset to 175 ± 5°C, for example, the weight loss is 1.1%;
(3) the crystal form **A** of the compound of formula I has a differential scanning calorimetry pattern showing an endothermic peak at 191.6 ± 5°C and/or 240.5 ± 5°C;
or,
when the crystal form of the compound of formula I is the crystal form **B** of the compound of formula I, the crystal form **B** of the compound of formula I satisfies one or more than one of the following conditions:
(1) the crystal form **B** of the compound of formula I has an X-ray powder diffraction pattern expressed at a 2θ angle further comprising diffraction peaks at one or more than one of the following 2θ angles: 16.11 ± 0.2°, 18.48 ± 0.2°, 21.23 ± 0.2°, 17.80 ± 0.2°, and 21.41 ± 0.2°;
(2) the crystal form **B** of the compound of formula I has a thermogravimetric analysis pattern with a weight loss of 1%-3% when heated from an onset to 200 ± 5°C, for example, the weight loss is 2.2%;
(3) the crystal form **B** of the compound of formula I has a differential scanning calorimetry pattern showing an endothermic peak at 240.1 ± 5°C;
or,
when the crystal form of the compound of formula I is the crystal form **C** of the compound of formula I, the crystal form **C** of the compound of formula I satisfies one or more than one of the following conditions:
(1) the crystal form **C** of the compound of formula I has an X-ray powder diffraction pattern expressed at a 2θ angle further comprising diffraction peaks at one or more than one of the following 2θ angles: 29.92 ± 0.2°, 23.30 ± 0.2°, 20.57 ± 0.2°, 31.49 ± 0.2°, and 10.11 ± 0.2°;
(2) the crystal form **C** of the compound of formula I has a thermogravimetric analysis pattern with a weight loss of 3%-4% when heated from an onset to 150 ± 5°C, for example, the weight loss is 3.2%;
(3) the crystal form **C** of the compound of formula I has a differential scanning calorimetry pattern showing an endothermic peak at one or more than one of 64.9 ± 5°C, 200.1 ± 5°C, and 237.0 ± 5°C.

3. The crystal form of the compound of formula I according to claim 2, wherein
when the crystal form of the compound of formula I is the crystal form **A** of the compound of formula I, the crystal form **A** of the compound of formula I satisfies the following conditions (1) and/or (2):
(1) the crystal form **A** of the compound of formula I has an X-ray powder diffraction pattern expressed at a 2θ angle further comprising diffraction peaks at one or more than one of the following 2θ angles: 18.13 ± 0.2°, 33.21 ± 0.2°, 17.86 ± 0.2°, 23.47 ± 0.2°, 26.91 ± 0.2°, and 16.77 ± 0.2°; preferably, the X-ray powder diffraction pattern expressed at a 2θ angle is basically as shown in Figure 1;
(2) the crystal form **A** of the compound of formula I has a differential scanning calorimetry pattern and a thermogravimetric analysis pattern basically as shown in Figure 2;
or,
when the crystal form of the compound of formula I is the crystal form **B** of the compound of formula I, the crystal form **B** of the compound of formula I satisfies the following conditions (1) and/or (2):
(1) the crystal form **B** of the compound of formula I has an X-ray powder diffraction pattern expressed at a 2θ angle further comprising diffraction peaks at one or more than one of the following 2θ angles: 17.01 ± 0.2°, 28.03 ± 0.2°, 21.96 ± 0.2°, 22.66 ± 0.2°, 30.78 ± 0.2°, and 19.66 ± 0.2°; preferably, the X-ray powder diffraction pattern expressed at a 2θ angle is basically as shown in Figure 4;
(2) the crystal form **B** of the compound of formula I has a differential scanning calorimetry pattern and a thermogravimetric analysis pattern basically as shown in Figure 5;
or,
when the crystal form of the compound of formula I is the crystal form **C** of the compound of formula I, the crystal form **C** of the compound of formula I satisfies the following conditions (1) and/or (2):
(1) the crystal form **C** of the compound of formula I has an X-ray powder diffraction pattern expressed at a 2θ angle further comprising diffraction peaks at one or more than one of the following 2θ angles: 24.93 ± 0.2°, 20.82 ± 0.2°, 11.60 ± 0.2°, 9.77 ± 0.2°, 10.98 ± 0.2°, and 26.96 ± 0.2°; preferably, the X-ray powder diffraction pattern expressed at a 2θ angle is basically as shown in Figure 53;
(2) the crystal form **C** of the compound of formula I has a differential scanning calorimetry pattern and a thermogravimetric analysis pattern basically as shown in Figure 54.

4. A pharmaceutically acceptable salt of a compound of formula I, wherein the pharmaceutically acceptable salt is a salt formed by the compound of formula I and an acid; the acid is an inorganic acid or an organic acid;

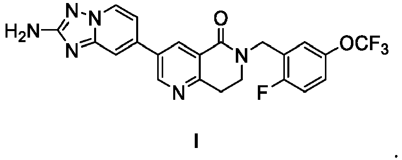

5. The pharmaceutically acceptable salt of the compound of formula I according to claim 4, wherein
the molar ratio of the compound of formula I to the acid is 1: (0.3-2);
and/or, the inorganic acid is hydrochloric acid and/or sulfuric acid;
and/or, the organic acid is one or more than one of fumaric acid, maleic acid, citric acid, methanesulfonic acid, *p*-toluenesulfonic acid, cyclamic acid, mucic acid, glycolic acid, malic acid, and hippuric acid; preferably, the organic acid is one or more than one of *p*-toluenesulfonic acid, methanesulfonic acid, maleic acid, fumaric acid, citric acid, and cyclamic acid; more preferably, the organic acid is *p*-toluenesulfonic acid.

6. The pharmaceutically acceptable salt of the compound of formula I according to claim 4, wherein the pharmaceutically acceptable salt of the compound of formula I is any one of the following:
(1) a hydrochloride salt of the compound of formula I; wherein the molar ratio of the compound of formula I to hydrochloric acid is 1:2;
(2) a sulfate salt of the compound of formula I; wherein the molar ratio of the compound of formula I to sulfuric acid is 1: (1-2), such as 1: (1-1.3), and further such as 1:1, 1:1.1, 1:1.3, or 1:2;
(3) a citrate salt of the compound of formula I; wherein the molar ratio of the compound of formula I to citric acid is 1: 1;
(4) a maleate salt of the compound of formula I; wherein the molar ratio of the compound of formula I to maleic acid is 1: (0.5-1), such as 1:0.8;
(5) a fumarate salt of the compound of formula I; wherein the molar ratio of the compound of formula I to fumaric acid is 1: (0.5-1), such as 1:0.9;
(6) a methanesulfonate salt of the compound of formula I; wherein the molar ratio of the compound of formula I to methanesulfonic acid is 1: (0.1-1), such as 1: (0.3-1), and further such as 1:0.3 or 1:1;
(7) a *p*-toluenesulfonate salt of the compound of formula I; wherein the molar ratio of the compound of formula I to *p*-toluenesulfonic acid is 1: (0.1-1), such as 1:0.9;
(8) a cyclamate salt of the compound of formula I; wherein the molar ratio of the compound of formula I to cyclamic acid is 1: (0.1-1), such as 1:0.9.

7. A crystal form of a pharmaceutically acceptable salt of a compound of formula I, wherein the crystal form of the pharmaceutically acceptable salt of the compound of formula I is a crystal form **A** of a hydrochloride salt of the compound of formula I, a crystal form **A** of a citrate salt of the compound of formula I, a crystal form **A** of a maleate salt of the compound of formula I, a crystal form **A** of a fumarate salt of the compound of formula I, a crystal form **A** of a methanesulfonate salt of the compound of formula I, a crystal form **B** of a methanesulfonate salt of the compound of formula I, a crystal form **C** of a methanesulfonate salt of the compound of formula I, a crystal form **A** of a *p*-toluenesulfonate salt of the compound of formula I, a crystal form **A** of a cyclamate salt of the compound of formula I, a crystal form **A** of a sulfate salt of the compound of formula I, a crystal form **D** of a sulfate salt of the compound of formula I, a crystal form **B** of a sulfate salt of the compound of formula I, a crystal form **C** of a sulfate salt of the compound of formula I, a crystal form **E** of a sulfate salt of the compound of formula I, or a crystal form **F** of a sulfate of the compound of formula I; 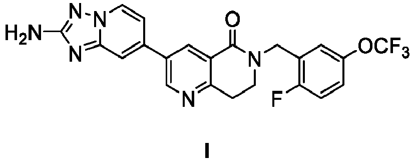
the crystal form **A** of the hydrochloride salt of the compound of formula I has an X-ray powder diffraction pattern expressed at a 2θ angle comprising diffraction peaks at the following 2θ angles: 8.83 ± 0.2°, 13.24 ± 0.2°, 24.25 ± 0.2°, and 18.31 ± 0.2°;
the crystal form **A** of the citrate salt of the compound of formula I has an X-ray powder diffraction pattern expressed at a 2θ angle comprising diffraction peaks at the following 2θ angles: 17.87 ± 0.2°, 14.97 ± 0.2°, 17.45 ± 0.2°, and 17.01 ± 0.2°;
the crystal form **A** of the maleate salt of the compound of formula I has an X-ray powder diffraction pattern expressed at a 2θ angle comprising diffraction peaks at the following 2θ angles: 16.47 ± 0.2°, 4.58 ± 0.2°, 10.97 ± 0.2°, and 22.91 ± 0.2°;
the crystal form **A** of the fumarate salt of the compound of formula I has an X-ray powder diffraction pattern expressed at a 2θ angle comprising diffraction peaks at the following 2θ angles: 28.83 ± 0.2°, 5.03 ± 0.2°, 16.15 ± 0.2°, and 13.11 ± 0.2°;
the crystal form **A** of the methanesulfonate salt of the compound of formula I has an X-ray powder diffraction pattern expressed at a 2θ angle comprising diffraction peaks at the following 2θ angles: 5.76 ± 0.2°, 14.75 ± 0.2°, 5.11 ± 0.2°, and 17.54 ± 0.2°;
the crystal form **B** of the methanesulfonate salt of the compound of formula I has an X-ray powder diffraction pattern expressed at a 2θ angle comprising diffraction peaks at the following 2θ angles: 18.83 ± 0.2°, 16.51 ± 0.2°, 25.51 ± 0.2°, and 10.98 ± 0.2°;
the crystal form **C** of the methanesulfonate salt of the compound of formula I has an X-ray powder diffraction pattern expressed at a 2θ angle comprising diffraction peaks at the following 2θ angles: 19.57 ± 0.2°, 19.98 ± 0.2°, 16.50 ± 0.2°, and 18.20 ± 0.2°;
the crystal form **A** of the *p*-toluenesulfonate salt of the compound of formula I has an X-ray powder diffraction pattern expressed at a 2θ angle comprising diffraction peaks at the following 2θ angles: 5.80 ± 0.2°, 19.70 ± 0.2°, 22.36 ± 0.2°, and 11.57 ± 0.2°;
the crystal form **A** of the cyclamate salt of the compound of formula I has an X-ray powder diffraction pattern expressed at a 2θ angle comprising diffraction peaks at the following 2θ angles: 6.41 ± 0.2°, 16.47 ± 0.2°, 18.57 ± 0.2°, and 10.95 ± 0.2°;
the crystal form **A** of the sulfate salt of the compound of formula I has an X-ray powder diffraction pattern expressed at a 2θ angle comprising diffraction peaks at the following 2θ angles: 14.60 ± 0.2°, 11.67 ± 0.2°, 21.09 ± 0.2°, and 13.32 ± 0.2°;
the crystal form **D** of the sulfate salt of the compound of formula I has an X-ray powder diffraction pattern expressed at a 2θ angle comprising diffraction peaks at the following 2θ angles: 14.60 ± 0.2°, 25.31 ± 0.2°, 19.85 ± 0.2°, and 20.07 ± 0.2°;
the crystal form **I** of the sulfate salt of the compound of formula I has an X-ray powder diffraction pattern expressed at a 2θ angle comprising diffraction peaks at the following 2θ angles: 12.92 ± 0.2°, 26.90 ± 0.2°, 15.86 ± 0.2°, and 23.02 ± 0.2°;
the crystal form **B** of the sulfate salt of the compound of formula I has an X-ray powder diffraction pattern expressed at a 2θ angle comprising diffraction peaks at the following 2θ angles: 25.15 ± 0.2°, 18.71 ± 0.2°, 3.13 ± 0.2°, and 23.28 ± 0.2°;
the crystal form **C** of the sulfate salt of the compound of formula I has an X-ray powder diffraction pattern expressed at a 2θ angle comprising diffraction peaks at the following 2θ angles: 5.55 ± 0.2°, 14.72 ± 0.2°, 16.75 ± 0.2°, and 27.35 ± 0.2°;
the crystal form **E** of the sulfate salt of the compound of formula I has an X-ray powder diffraction pattern expressed at a 2θ angle comprising diffraction peaks at the following 2θ angles: 16.39 ± 0.2°, 12.75 ± 0.2°, 14.32 ± 0.2°, and 20.28 ± 0.2°;
the crystal form **F** of the sulfate salt of the compound of formula I has an X-ray powder diffraction pattern expressed at a 2θ angle comprising diffraction peaks at the following 2θ angles: 24.91 ± 0.2°, 20.60 ± 0.2°, 27.62 ± 0.2°, and 16.04 ± 0.2°.

8. The crystal form of the pharmaceutically acceptable salt of the compound of formula I according to claim 7, wherein
when the crystal form of the pharmaceutically acceptable salt of the compound of formula I is the crystal form **A** of the hydrochloride salt of the compound of formula I, the crystal form **A** of the hydrochloride salt of the compound of formula I satisfies one or more than one of the following conditions:
(1) the crystal form **A** of the hydrochloride salt of the compound of formula I has an X-ray powder diffraction pattern expressed at a 2θ angle further comprising diffraction peaks at one or more than one of the following 2θ angles: 26.66 ± 0.2°, 22.21 ± 0.2°, 4.39 ± 0.2°, 31.19 ± 0.2°, and 19.54 ± 0.2°;
(2) the crystal form **A** of the hydrochloride salt of the compound of formula I has a thermogravimetric analysis pattern with a weight loss of 4%-5.5% when heated from an onset to 100 ± 5°C, for example, the weight loss is 4.8%;
(3) the crystal form **A** of the hydrochloride salt of the compound of formula I has a differential scanning calorimetry pattern showing an endothermic peak at 156.8 ± 5°C;
(4) the hydrochloride salt of the compound of formula I in the crystal form **A** of the hydrochloride salt of the compound of formula I is as defined in claim 6;
or,
when the crystal form of the pharmaceutically acceptable salt of the compound of formula I is the crystal form **A** of the citrate salt of the compound of formula I, the crystal form **A** of the citrate salt of the compound of formula I satisfies one or more than one of the following conditions:
(1) the crystal form **A** of the citrate salt of the compound of formula I has an X-ray powder diffraction pattern expressed at a 2θ angle further comprising diffraction peaks at one or more than one of the following 2θ angles: 4.45 ± 0.2°, 11.14 ± 0.2°, 19.98 ± 0.2°, 8.91 ± 0.2°, and 13.38 ± 0.2°;
(2) the crystal form **A** of the citrate salt of the compound of formula I has a thermogravimetric analysis pattern with a weight loss of 0.5%-2% when heated from an onset to 150 ± 5°C, for example, the weight loss is 1.1%;
(3) the crystal form **A** of the citrate salt of the compound of formula I has a differential scanning calorimetry pattern showing an endothermic peak at 166.4 ± 5°C;
(4) the citrate salt of the compound of formula I in the crystal form **A** of the citrate salt of the compound of formula I is as defined in claim 6; or,
when the crystal form of the pharmaceutically acceptable salt of the compound of formula I is the crystal form **A** of the maleate salt of the compound of formula I, the crystal form **A** of the maleate salt of the compound of formula I satisfies one or more than one of the following conditions:
(1) the crystal form **A** of the maleate salt of the compound of formula I has an X-ray powder diffraction pattern expressed at a 2θ angle further comprising diffraction peaks at one or more than one of the following 2θ angles: 18.2 ± 0.2°, 19.87 ± 0.2°, 24.64 ± 0.2°, 22.35 ± 0.2°, and 20.26 ± 0.2°;
(2) the crystal form **A** of the maleate salt of the compound of formula I has a thermogravimetric analysis pattern with a weight loss of 3%-4% at 150 ± 5°C, for example, the weight loss is 3.3%; and a weight loss of 10%-15% from 150°C to 250°C, for example, the weight loss is 13.6%;
(3) the crystal form **A** of the maleate salt of the compound of formula I has a differential scanning calorimetry pattern showing an endothermic peak at 176.0 ± 5°C and/or 210.7 ± 5°C;
(4) the maleate salt of the compound of formula I in the crystal form **A** of the maleate salt of the compound of formula I is as defined in claim 6;
or,
when the crystal form of the pharmaceutically acceptable salt of the compound of formula I is the crystal form **A** of the fumarate salt of the compound of formula I, the crystal form **A** of the fumarate salt of the compound of formula I satisfies one or more than one of the following conditions:
(1) the crystal form **A** of the fumarate salt of the compound of formula I has an X-ray powder diffraction pattern expressed at a 2θ angle further comprising diffraction peaks at one or more than one of the following 2θ angles: 22.86 ± 0.2°, 12.39 ± 0.2°, 29.44 ± 0.2°, 17.78 ± 0.2°, and 9.69 ± 0.2°;
(2) the crystal form **A** of the fumarate salt of the compound of formula I has a thermogravimetric analysis pattern with a weight loss of 1%-3% when heated from an onset to 150 ± 5°C, for example, the weight loss is 1.8%;
(3) the crystal form **A** of the fumarate salt of the compound of formula I has a differential scanning calorimetry pattern showing an endothermic peak at one or more than one of 182.5 ± 5°C, 192.9 ± 5°C, 211.8 ± 5°C, and 219.2 ± 5°C, and/or, an exothermic peak at one or more than one of 123.0 ± 5°C, 184.8 ± 5°C, and 196.9 ± 5°C;
(4) the fumarate salt of the compound of formula I in the crystal form **A** of the fumarate salt of the compound of formula I is as defined in claim 6; or,
when the crystal form of the pharmaceutically acceptable salt of the compound of formula I is the crystal form **A** of the methanesulfonate salt of the compound of formula I, the crystal form **A** of the methanesulfonate salt of the compound of formula I satisfies one or more than one of the following conditions:
(1) the crystal form **A** of the methanesulfonate salt of the compound of formula I has an X-ray powder diffraction pattern expressed at a 2θ angle further comprising diffraction peaks at one or more than one of the following 2θ angles: 20.29 ± 0.2°, 20.49 ± 0.2°, 16.84 ± 0.2°, 7.51 ± 0.2°, and 16.51 ± 0.2°;
(2) the crystal form **A** of the methanesulfonate salt of the compound of formula I has a thermogravimetric analysis pattern with a weight loss of 2%-4% when heated from an onset to 110 ± 5°C, for example, the weight loss is 2.9%;
(3) the crystal form **A** of the methanesulfonate salt of the compound of formula I has a differential scanning calorimetry pattern showing an endothermic peak at 92.5 ± 5°C and/or 138.6 ± 5°C;
(4) the methanesulfonate salt of the compound of formula I in the crystal form **A** of the methanesulfonate salt of the compound of formula I is as defined in claim 6;
or,
when the crystal form of the pharmaceutically acceptable salt of the compound of formula I is the crystal form **B** of the methanesulfonate salt of the compound of formula I, the crystal form **B** of the methanesulfonate salt of the compound of formula I satisfies one or more than one of the following conditions:
(1) the crystal form **B** of the methanesulfonate salt of the compound of formula I has an X-ray powder diffraction pattern expressed at a 2θ angle further comprising diffraction peaks at one or more than one of the following 2θ angles: 22.32 ± 0.2°, 5.49 ± 0.2°, 20.34 ± 0.2°, 26.34 ± 0.2°, and 16.94 ± 0.2°;
(2) the crystal form **B** of the methanesulfonate salt of the compound of formula I has a thermogravimetric analysis pattern with a weight loss of 2%-4% when heated from an onset to 150 ± 5°C, for example, the weight loss is 2.8%;
(3) the crystal form **B** of the methanesulfonate salt of the compound of formula I has a differential scanning calorimetry pattern showing an endothermic peak at 171.3 ± 5°C and/or 194.7 ± 5°C;
(4) the methanesulfonate salt of the compound of formula I in the crystal form **B** of the methanesulfonate salt of the compound of formula I is as defined in claim 6;
or,
when the crystal form of the pharmaceutically acceptable salt of the compound of formula I is the crystal form **C** of the methanesulfonate salt of the compound of formula I, the crystal form **C** of the methanesulfonate salt of the compound of formula I satisfies one or more than one of the following conditions:
(1) the crystal form **C** of the methanesulfonate salt of the compound of formula I has an X-ray powder diffraction pattern expressed at a 2θ angle further comprising diffraction peaks at one or more than one of the following 2θ angles: 20.26 ± 0.2°, 23.30 ± 0.2°, 29.52 ± 0.2°, 10.99 ± 0.2°, and 26.94 ± 0.2°;
(2) the crystal form **C** of the methanesulfonate salt of the compound of formula I has a thermogravimetric analysis pattern with a weight loss of 4%-6% when heated from an onset to 150 ± 5°C, for example, the weight loss is 4.9%;
(3) the crystal form **C** of the methanesulfonate salt of the compound of formula I has a differential scanning calorimetry pattern showing an endothermic peak at one or more than one of 108.2 ± 5°C, 181.9 ± 5°C, and 233.2 ± 5°C;
(4) the methanesulfonate salt of the compound of formula I in the crystal form **C** of the methanesulfonate salt of the compound of formula I is as defined in claim 6;
or,
when the crystal form of the pharmaceutically acceptable salt of the compound of formula I is the crystal form **A** of the *p*-toluenesulfonate salt of the compound of formula I, the crystal form **A** of the *p*-toluenesulfonate salt of the compound of formula I satisfies one or more than one of the following conditions:
(1) the crystal form **A** of the *p*-toluenesulfonate salt of the compound of formula I has an X-ray powder diffraction pattern expressed at a 2θ angle further comprising diffraction peaks at one or more than one of the following 2θ angles: 27.22 ± 0.2°, 14.81 ± 0.2°, 16.83 ± 0.2°, 27.91 ± 0.2°, and 15.18 ± 0.2°;
(2) the crystal form **A** of the *p*-toluenesulfonate salt of the compound of formula I has a thermogravimetric analysis pattern with a weight loss of 1%-3% when heated from an onset to 150 ± 5°C, for example, the weight loss is 1.6%;
(3) the crystal form **A** of the *p*-toluenesulfonate salt of the compound of formula I has a differential scanning calorimetry pattern showing an endothermic peak at 205.6 ± 5°C;
(4) the *p*-toluenesulfonate salt of the compound of formula I in the crystal form **A** of the p-toluenesulfonate salt of the compound of formula I is as defined in claim 6;
or,
when the crystal form of the pharmaceutically acceptable salt of the compound of formula I is the crystal form **A** of the cyclamate salt of the compound of formula I, the crystal form **A** of the cyclamate salt of the compound of formula I satisfies one or more than one of the following conditions:
(1) the crystal form **A** of the cyclamate salt of the compound of formula I has an X-ray powder diffraction pattern expressed at a 2θ angle further comprising diffraction peaks at one or more than one of the following 2θ angles: 18.16 ± 0.2°, 19.58 ± 0.2°, 20.24 ± 0.2°, 22.33 ± 0.2°, and 23.27 ± 0.2°;
(2) the crystal form **A** of the cyclamate salt of the compound of formula I has a thermogravimetric analysis pattern with a weight loss of 0.1%-2% when heated from an onset to 150 ± 5°C, for example, the weight loss is 0.7%;
(3) the crystal form **A** of the cyclamate salt of the compound of formula I has a differential scanning calorimetry pattern showing an endothermic peak at 202.3 ± 5°C and/or 231.3 ± 5°C; and/or, an exothermic peak at 118.2 ± 5°C and/or 205.4 ± 5°C;
(4) the cyclamate salt of the compound of formula I in the crystal form **A** of the cyclamate salt of the compound of formula I is as defined in claim 6;
or,
when the crystal form of the pharmaceutically acceptable salt of the compound of formula I is the crystal form **A** of the sulfate salt of the compound of formula I, the crystal form **A** of the sulfate salt of the compound of formula I satisfies one or more than one of the following conditions:
(1) the crystal form **A** of the sulfate salt of the compound of formula I has an X-ray powder diffraction pattern expressed at a 2θ angle further comprising diffraction peaks at one or more than one of the following 2θ angles: 17.38 ± 0.2°, 21.65 ± 0.2°, 26.08 ± 0.2°, 23.45 ± 0.2°, and 26.44 ± 0.2°;
(2) the crystal form **A** of the sulfate salt of the compound of formula I has a thermogravimetric analysis pattern with a weight loss of 2%-4% when heated from an onset to 175 ± 5°C, for example, the weight loss is 3.4%;
(3) the crystal form **A** of the sulfate salt of the compound of formula I has a differential scanning calorimetry pattern showing an endothermic peak at 205.1 ± 5°C;
(4) the sulfate salt of the compound of formula I in the crystal form **A** of the sulfate salt of the compound of formula I is as defined in claim 6;
or,
when the crystal form of the pharmaceutically acceptable salt of the compound of formula I is the crystal form **D** of the sulfate salt of the compound of formula I, the crystal form **D** of the sulfate salt of the compound of formula I satisfies one or more than one of the following conditions:
(1) the crystal form **D** of the sulfate salt of the compound of formula I has an X-ray powder diffraction pattern expressed at a 2θ angle further comprising diffraction peaks at one or more than one of the following 2θ angles: 21.06 ± 0.2°, 19.31 ± 0.2°, 18.15 ± 0.2°, 13.18 ± 0.2°, and 6.59 ± 0.2°;
(2) the crystal form **D** of the sulfate salt of the compound of formula I has a thermogravimetric analysis pattern with a weight loss of 1%-4% when heated from an onset to 125 ± 5°C, for example, the weight loss is 3%;
(3) the crystal form **D** of the sulfate salt of the compound of formula I has a differential scanning calorimetry pattern showing an endothermic peak at one or more than one of 111.9 ± 5°C, 153.2 ± 5°C, and 197.7 ± 5°C;
(4) the sulfate salt of the compound of formula I in the crystal form **D** of the sulfate salt of the compound of formula I is as defined in claim 6;
or,
when the crystal form of the pharmaceutically acceptable salt of the compound of formula I is the crystal form **I** of the sulfate salt of the compound of formula I, the crystal form **I** of the sulfate salt of the compound of formula I has an X-ray powder diffraction pattern expressed at a 2θ angle further comprising diffraction peaks at one or more than one of the following 2θ angles: 21.09 ± 0.2°, 14.99 ± 0.2°, 17.26 ± 0.2°, 19.34 ± 0.2°, and 24.61 ± 0.2°;
and/or, the sulfate salt of the compound of formula I in the crystal form **I** of the sulfate salt of the compound of formula I is as defined in claim 6;
or,
when the crystal form of the pharmaceutically acceptable salt of the compound of formula I is the crystal form **B** of the sulfate salt of the compound of formula I, the crystal form **B** of the sulfate salt of the compound of formula I satisfies one or more than one of the following conditions:
(1) the crystal form **B** of the sulfate salt of the compound of formula I has an X-ray powder diffraction pattern expressed at a 2θ angle further comprising diffraction peaks at one or more than one of the following 2θ angles: 20.27 ± 0.2°, 24.82 ± 0.2°, 15.17 ± 0.2°, 14.82 ± 0.2°, and 23.67 ± 0.2°;
(2) the crystal form **B** of the sulfate salt of the compound of formula I has a thermogravimetric analysis pattern with a weight loss of 2%-4% when heated from an onset to 150 ± 5°C, for example, the weight loss is 2.9%;
(3) the crystal form **B** of the sulfate salt of the compound of formula I has a differential scanning calorimetry pattern showing an endothermic peak at one or more than one of 77.9 ± 5°C, 118.8 ± 5°C, and 196.8 ± 5°C;
(4) the sulfate salt of the compound of formula I in the crystal form **B** of the sulfate salt of the compound of formula I is as defined in claim 6;
or,
when the crystal form of the pharmaceutically acceptable salt of the compound of formula I is the crystal form **C** of the sulfate salt of the compound of formula I, the crystal form **C** of the sulfate salt of the compound of formula I satisfies one or more than one of the following conditions:
(1) in the crystal form **C** of the sulfate salt of the compound of formula I, the sulfate salt of the compound of formula I is a hydrate, the molar ratio of the compound of formula I to water is 1: (1-2), such as 1: (1-1.3), also such as 1: (1-1.1);
(2) the crystal form **C** of the sulfate salt of the compound of formula I has an X-ray powder diffraction pattern expressed at a 2θ angle further comprising diffraction peaks at one or more than one of the following 2θ angles: 22.92 ± 0.2°, 20.90 ± 0.2°, 21.13 ± 0.2°, 19.63 ± 0.2°, and 20.37 ± 0.2°;
(3) the crystal form **C** of the sulfate salt of the compound of formula I has a thermogravimetric analysis pattern with a weight loss of 3%-4.5% when heated from an onset to 175 ± 5°C;
(4) the crystal form **C** of the sulfate salt of the compound of formula I has a differential scanning calorimetry pattern showing an endothermic peak at 152.8 ± 5°C;
(5) the crystal form **C** of the sulfate salt of the compound of formula I, wherein the molar ratio of the compound of formula I to the sulfate salt is 1: (1-2), such as 1: (1-1.3), 1: (1-1.1), or 1:1, and further such as 1:1, 1:1.1, 1:1.3, or 1:2;
or,
when the crystal form of the pharmaceutically acceptable salt of the compound of formula I is the crystal form **E** of the sulfate salt of the compound of formula I, the crystal form **E** of the sulfate salt of the compound of formula I satisfies one or more than one of the following conditions:
(1) the crystal form **E** of the sulfate salt of the compound of formula I has an X-ray powder diffraction pattern expressed at a 2θ angle further comprising diffraction peaks at one or more than one of the following 2θ angles: 17.76 ± 0.2°, 23.45 ± 0.2°, 26.20 ± 0.2°, 17.47 ± 0.2°, and 19.76 ± 0.2°;
(2) the crystal form **E** of the sulfate salt of the compound of formula I has a thermogravimetric analysis pattern with a weight loss of 5%-6.5% when heated from an onset to 150 ± 5°C, for example, the weight loss is 5.9%;
(3) the crystal form **E** of the sulfate salt of the compound of formula I has a differential scanning calorimetry pattern showing an endothermic peak at one or more than one of 82.2 ± 5°C, 138.6 ± 5°C, and 205.4 ± 5°C;
(4) the sulfate salt of the compound of formula I in the crystal form **E** of the sulfate salt of the compound of formula I is as defined in claim 6;
or,
when the crystal form of the pharmaceutically acceptable salt of the compound of formula I is the crystal form **F** of the sulfate salt of the compound of formula I, the crystal form **F** of the sulfate salt of the compound of formula I satisfies one or more than one of the following conditions:
(1) the crystal form **F** of the sulfate salt of the compound of formula I has an X-ray powder diffraction pattern expressed at a 2θ angle further comprising diffraction peaks at one or more than one of the following 2θ angles: 24.26 ± 0.2°, 24.14 ± 0.2°, 18.63 ± 0.2°, 17.01 ± 0.2°, and 21.53 ± 0.2°;
(2) the crystal form **F** of the sulfate salt of the compound of formula I has a thermogravimetric analysis pattern with a weight loss of 5%-7% when heated from an onset to 160°C, for example, the weight loss is 6.4%; and a weight loss of 6%-8% when heated from 160°C to 225°C, for example, the weight loss is 6.9%;
(3) the crystal form **F** of the sulfate salt of the compound of formula I has a differential scanning calorimetry pattern showing an endothermic peak at 123.6 ± 5°C and/or 185.2 ± 5°C; and/or, one exothermic peak at 206.0 ± 5°C;
(4) the sulfate salt of the compound of formula I in the crystal form **F** of the sulfate salt of the compound of formula I is as defined in claim 6.

9. The crystal form of the pharmaceutically acceptable salt of the compound of formula I according to claim 8, wherein
when the crystal form of the pharmaceutically acceptable salt of the compound of formula I is the crystal form **A** of the hydrochloride salt of the compound of formula I, the crystal form **A** of the hydrochloride salt of the compound of formula I satisfies the following conditions (1) and/or (2):
(1) the crystal form **A** of the hydrochloride salt of the compound of formula I has an X-ray powder diffraction pattern expressed at a 2θ angle further comprising diffraction peaks at one or more than one of the following 2θ angles: 35.77 ± 0.2°, 19.71 ± 0.2°, 27.3 ± 0.2°, 32.11 ± 0.2°, 28.38 ± 0.2°, and 21.52 ± 0.2°; preferably, the X-ray powder diffraction pattern expressed at a 2θ angle is basically as shown in Figure 7;
(2) the crystal form **A** of the hydrochloride salt of the compound of formula I has a differential scanning calorimetry pattern and a thermogravimetric analysis pattern basically as shown in Figure 8;
or,
when the crystal form of the pharmaceutically acceptable salt of the compound of formula I is the crystal form **A** of the citrate salt of the compound of formula I, the crystal form **A** of the citrate salt of the compound of formula I satisfies the following conditions (1) and/or (2):
(1) the crystal form **A** of the citrate salt of the compound of formula I has an X-ray powder diffraction pattern expressed at a 2θ angle further comprising diffraction peaks at one or more than one of the following 2θ angles: 21.23 ± 0.2°, 25.56 ± 0.2°, 21.00 ± 0.2°, 24.25 ± 0.2°, 28.48 ± 0.2°, and 29.9 ± 0.2°; preferably, the X-ray powder diffraction pattern expressed at a 2θ angle is basically as shown in Figure 19;
(2) the crystal form **A** of the citrate salt of the compound of formula I has a differential scanning calorimetry pattern and a thermogravimetric analysis pattern basically as shown in Figure 20;
or,
when the crystal form of the pharmaceutically acceptable salt of the compound of formula I is the crystal form **A** of the maleate salt of the compound of formula I, the crystal form **A** of the maleate salt of the compound of formula I satisfies the following conditions (1) and/or (2):
(1) further comprising diffraction peaks at one or more than one of the following 2θ angles: 19.56 ± 0.2°, 28.43 ± 0.2°, 26.52 ± 0.2°, 15.04 ± 0.2°, 27.52 ± 0.2°, and 18.86 ± 0.2°; preferably, the X-ray powder diffraction pattern expressed at a 2θ angle is basically as shown in Figure 22;
(2) the crystal form **A** of the maleate salt of the compound of formula I has a differential scanning calorimetry pattern and a thermogravimetric analysis pattern basically as shown in Figure 23;
or,
when the crystal form of the pharmaceutically acceptable salt of the compound of formula I is the crystal form **A** of the fumarate salt of the compound of formula I, the crystal form A of the fumarate salt of the compound of formula I satisfies the following conditions (1) and/or (2):
(1) the crystal form **A** of the fumarate salt of the compound of formula I has an X-ray powder diffraction pattern expressed at a 2θ angle further comprising diffraction peaks at one or more than one of the following 2θ angles: 19.82 ± 0.2°, 10.73 ± 0.2°, 3.25 ± 0.2°, 17.09 ± 0.2°, 6.54 ± 0.2°, and 11.22 ± 0.2°; preferably, the X-ray powder diffraction pattern expressed at a 2θ angle is basically as shown in Figure 25;
(2) the crystal form **A** of the fumarate salt of the compound of formula I has a differential scanning calorimetry pattern and a thermogravimetric analysis pattern basically as shown in Figure 26;
or,
when the crystal form of the pharmaceutically acceptable salt of the compound of formula I is the crystal form **A** of the methanesulfonate salt of the compound of formula I, the crystal form **A** of the methanesulfonate salt of the compound of formula I satisfies the following conditions (1) and/or (2):
(1) the crystal form **A** of the methanesulfonate salt of the compound of formula I has an X-ray powder diffraction pattern expressed at a 2θ angle further comprising diffraction peaks at one or more than one of the following 2θ angles: 21.72 ± 0.2°, 19.60 ± 0.2°, 15.51 ± 0.2°, 18.43 ± 0.2°, 15.86 ± 0.2°, and 12.84 ± 0.2°; preferably, the X-ray powder diffraction pattern expressed at a 2θ angle is basically as shown in Figure 28;
(2) the crystal form **A** of the methanesulfonate salt of the compound of formula I has a differential scanning calorimetry pattern and a thermogravimetric analysis pattern basically as shown in Figure 29;
or,
when the crystal form of the pharmaceutically acceptable salt of the compound of formula I is the crystal form **B** of the methanesulfonate salt of the compound of formula I, the crystal form **B** of the methanesulfonate salt of the compound of formula I satisfies the following conditions (1) and/or (2):
(1) the crystal form **B** of the methanesulfonate salt of the compound of formula I has an X-ray powder diffraction pattern expressed at a 2θ angle further comprising diffraction peaks at one or more than one of the following 2θ angles: 19.48 ± 0.2°, 21.35 ± 0.2°, 15.06 ± 0.2°, 19.93 ± 0.2°, 12.75 ± 0.2°, and 18.05 ± 0.2°; preferably, the X-ray powder diffraction pattern expressed at a 2θ angle is basically as shown in Figure 31;
(2) the crystal form **B** of the methanesulfonate salt of the compound of formula I has a differential scanning calorimetry pattern and a thermogravimetric analysis pattern basically as shown in Figure 32;
or,
when the crystal form of the pharmaceutically acceptable salt of the compound of formula I is the crystal form **C** of the methanesulfonate salt of the compound of formula I, the crystal form **C** of the methanesulfonate salt of the compound of formula I satisfies the following conditions (1) and/or (2):
(1) the crystal form **C** of the methanesulfonate salt of the compound of formula I has an X-ray powder diffraction pattern expressed at a 2θ angle further comprising diffraction peaks at one or more than one of the following 2θ angles: 22.36 ± 0.2°, 20.86 ± 0.2°, 24.62 ± 0.2°, 24.92 ± 0.2°, 27.41 ± 0.2°, and 18.45 ± 0.2°; preferably, the X-ray powder diffraction pattern expressed at a 2θ angle is basically as shown in Figure 34;
(2) the crystal form **C** of the methanesulfonate salt of the compound of formula I has a differential scanning calorimetry pattern and a thermogravimetric analysis pattern basically as shown in Figure 35;
or,
when the crystal form of the pharmaceutically acceptable salt of the compound of formula I is the crystal form **A** of the *p*-toluenesulfonate salt of the compound of formula I, the crystal form **A** of the *p*-toluenesulfonate salt of the compound of formula I satisfies the following conditions (1) and/or (2):
(1) the crystal form **A** of the *p*-toluenesulfonate salt of the compound of formula I has an X-ray powder diffraction pattern expressed at a 2θ angle further comprising diffraction peaks at one or more than one of the following 2θ angles: 19.21 ± 0.2°, 18.16 ± 0.2°, 13.07 ± 0.2°, 30.66 ± 0.2°, 32.47 ± 0.2°, and 18.47 ± 0.2°; preferably, the X-ray powder diffraction pattern expressed at a 2θ angle is basically as shown in Figure 37;
(2) the crystal form **A** of the *p*-toluenesulfonate salt of the compound of formula I has a differential scanning calorimetry pattern and a thermogravimetric analysis pattern basically as shown in Figure 38;
or,
when the crystal form of the pharmaceutically acceptable salt of the compound of formula I is the crystal form **A** of the cyclamate salt of the compound of formula I, the crystal form **A** of the cyclamate salt of the compound of formula I satisfies the following conditions (1) and/or (2):
(1) the crystal form **A** of the cyclamate salt of the compound of formula I has an X-ray powder diffraction pattern expressed at a 2θ angle further comprising diffraction peaks at one or more than one of the following 2θ angles: 24.63 ± 0.2°, 26.94 ± 0.2°, 27.39 ± 0.2°, 21.39 ± 0.2°, 20.83 ± 0.2°, and 24.93 ± 0.2°; preferably, the X-ray powder diffraction pattern expressed at a 2θ angle is basically as shown in Figure 40;
(2) the crystal form **A** of the cyclamate salt of the compound of formula I has a differential scanning calorimetry pattern and a thermogravimetric analysis pattern basically as shown in Figure 41;
or,
when the crystal form of the pharmaceutically acceptable salt of the compound of formula I is the crystal form **A** of the sulfate salt of the compound of formula I, the crystal form **A** of the sulfate salt of the compound of formula I satisfies the following conditions (1) and/or (2):
(1) the crystal form **A** of the sulfate salt of the compound of formula I has an X-ray powder diffraction pattern expressed at a 2θ angle further comprising diffraction peaks at one or more than one of the following 2θ angles: 15.76 ± 0.2°, 8.75 ± 0.2°, 37.29 ± 0.2°, 24.42 ± 0.2°, 22.08 ± 0.2°, and 29.39 ± 0.2°; preferably, the X-ray powder diffraction pattern expressed at a 2θ angle is basically as shown in Figure 10;
(2) the crystal form **A** of the sulfate salt of the compound of formula I has a differential scanning calorimetry pattern and a thermogravimetric analysis pattern basically as shown in Figure 11;
or,
when the crystal form of the pharmaceutically acceptable salt of the compound of formula I is the crystal form **D** of the sulfate salt of the compound of formula I, the crystal form **D** of the sulfate salt of the compound of formula I satisfies the following conditions (1) and/or (2):
(1) the crystal form **D** of the sulfate salt of the compound of formula I has an X-ray powder diffraction pattern expressed at a 2θ angle further comprising diffraction peaks at one or more than one of the following 2θ angles: 15.02 ± 0.2°, 31.39 ± 0.2°, 22.14 ± 0.2°, 24.63 ± 0.2°, 25.63 ± 0.2°, and 9.83 ± 0.2°; preferably, the X-ray powder diffraction pattern expressed at a 2θ angle is basically as shown in Figure 43;
(2) the crystal form **D** of the sulfate salt of the compound of formula I has a differential scanning calorimetry pattern and a thermogravimetric analysis pattern basically as shown in Figure 44;
or,
when the crystal form of the pharmaceutically acceptable salt of the compound of formula I is the crystal form **I** of the sulfate salt of the compound of formula I, the crystal form **I** of the sulfate salt of the compound of formula I has an X-ray powder diffraction pattern expressed at a 2θ angle further comprising diffraction peaks at one or more than one of the following 2θ angles: 20.79 ± 0.2°, 21.75 ± 0.2°, 18.31 ± 0.2°, 18.47 ± 0.2 °, 21.61 ± 0.2° and 19.75 ± 0.2°; preferably, the X-ray powder diffraction pattern expressed at a 2θ angle is basically as shown in Figure 46;
or,
when the crystal form of the pharmaceutically acceptable salt of the compound of formula I is the crystal form **B** of the sulfate salt of the compound of formula I, the crystal form **B** of the sulfate salt of the compound of formula I satisfies the following conditions (1) and/or (2):
(1) the crystal form **B** of the sulfate salt of the compound of formula I has an X-ray powder diffraction pattern expressed at a 2θ angle further comprising diffraction peaks at one or more than one of the following 2θ angles: 21.03 ± 0.2°, 19.05 ± 0.2°, 18.34 ± 0.2°, 12.20 ± 0.2°, 24.18 ± 0.2°, and 17.21 ± 0.2°; preferably, the X-ray powder diffraction pattern expressed at a 2θ angle is basically as shown in Figure 13;
(2) the crystal form **B** of the sulfate salt of the compound of formula I has a differential scanning calorimetry pattern and a thermogravimetric analysis pattern basically as shown in Figure 14;
or,
when the crystal form of the pharmaceutically acceptable salt of the compound of formula I is the crystal form **C** of the sulfate salt of the compound of formula I, the crystal form **C** of the sulfate salt of the compound of formula I satisfies one or more than one of the following conditions:
(1) in the crystal form **C** of the sulfate salt of the compound of formula I, the sulfate salt of the compound of formula I is a monohydrate, the molar ratio of the compound of formula I to sulfuric acid is 1:1;
(2) the crystal form **C** of the sulfate salt of the compound of formula I has an X-ray powder diffraction pattern expressed at a 2θ angle further comprising diffraction peaks at one or more than one of the following 2θ angles: 20.72 ± 0.2°, 12.52 ± 0.2°, 19.45 ± 0.2°, 19.96 ± 0.2°, 25.16 ± 0.2°, and 12.75 ± 0.2°; preferably, the X-ray powder diffraction pattern expressed at a 2θ angle is basically as shown in Figure 16;
(3) the crystal form **C** of the sulfate salt of the compound of formula I has a differential scanning calorimetry pattern and a thermogravimetric analysis pattern basically as shown in Figure 17;
(4) the crystal form **C** of the sulfate salt of the compound of formula I has a dynamic vapor sorption pattern basically as shown in Figure 55;
or,
when the crystal form of the pharmaceutically acceptable salt of the compound of formula I is the crystal form **E** of the sulfate salt of the compound of formula I, the crystal form **E** of the sulfate salt of the compound of formula I satisfies the following conditions (1) and/or (2):
(1) the crystal form **E** of the sulfate salt of the compound of formula I has an X-ray powder diffraction pattern expressed at a 2θ angle further comprising diffraction peaks at one or more than one of the following 2θ angles: 11.46 ± 0.2°, 25.88 ± 0.2°, 21.90 ± 0.2°, 23.00 ± 0.2°, 20.79 ± 0.2°, and 18.25 ± 0.2°; preferably, the X-ray powder diffraction pattern expressed at a 2θ angle is basically as shown in Figure 47;
(2) the crystal form **E** of the sulfate salt of the compound of formula I has a differential scanning calorimetry pattern and a thermogravimetric analysis pattern basically as shown in Figure 48;
or,
when the crystal form of the pharmaceutically acceptable salt of the compound of formula I is the crystal form **F** of the sulfate salt of the compound of formula I, the crystal form **F** of the sulfate salt of the compound of formula I satisfies the following conditions (1) and/or (2):
(1) the crystal form **F** of the sulfate salt of the compound of formula I has an X-ray powder diffraction pattern expressed at a 2θ angle further comprising diffraction peaks at one or more than one of the following 2θ angles: 23.10 ± 0.2°, 19.94 ± 0.2°, 19.73 ± 0.2°, 15.29 ± 0.2°, 28.29 ± 0.2°, and 20.12 ± 0.2°; preferably, the X-ray powder diffraction pattern expressed at a 2θ angle is basically as shown in Figure 50;
(2) the crystal form **F** of the sulfate salt of the compound of formula I has a differential scanning calorimetry pattern and a thermogravimetric analysis pattern basically as shown in Figure 51.

10. A preparation method for the crystal form of the pharmaceutically acceptable salt of the compound of formula I according to any one of claims 7 to 9, wherein the preparation method is a preparation method for the crystal form **A** of the sulfate salt of the compound of formula I, a preparation method for the crystal form **C** of the sulfate salt of the compound of formula I, a preparation method for the crystal form **D** of the sulfate salt of the compound of formula I, a preparation method for the crystal form **E** of the sulfate salt of the compound of formula I, or a preparation method for the crystal form **F** of the sulfate salt of the compound of formula I, wherein
the preparation method for the crystal form **A** of the sulfate salt of the compound of formula I is method **a** or method **b:**
method **a,** which comprises the following steps: crystallizing the compound of formula I and sulfuric acid in methanol at room temperature to obtain the crystal form **A** of the sulfate salt of the compound of formula I;
method **b,** which comprises the following steps: crystallizing a sulfate salt of the compound of formula I in a solvent to obtain the crystal form **A** of the sulfate salt of the compound of formula I; the solvent is a solvent of C₁₋₃ alcohol - 2-methyltetrahydrofuran;
the preparation method for the crystal form **C** of the sulfate salt of the compound of formula I is method **1** or method **2:**
method **1,** which comprises the following steps: dissolving the compound of formula I to clear in a nitrile solvent with a sulfuric acid aqueous solution and then cooling to obtain the crystal form **C** of the sulfate salt of the compound of formula I;
method **2,** which comprises the following steps: crystallizing the compound of formula I and sulfuric acid in a water-nitrile solvent at room temperature to obtain the crystal form **C** of the sulfate salt of the compound of formula I;
the preparation method for the crystal form **D** of the sulfate salt of the compound of formula I comprises the following steps: forming a suspension of a sulfate salt of the compound of formula I in propanol for crystal form transition to obtain the crystal form **D** of the sulfate salt of the compound of formula I;
the preparation method for the crystal form **E** of the sulfate salt of the compound of formula I comprises the following steps: forming a suspension of a monohydrate sulfate salt of the compound of formula I in a mixed solvent of ester-water for crystal form transition to obtain the crystal form **E** of the sulfate salt of the compound of formula I;
the preparation method for the crystal form **F** of the sulfate salt of the compound of formula I comprises the following steps: volatilizing a monohydrate sulfate salt of the compound of formula I in a sulfoxide solvent to obtain the crystal form **E** of the sulfate salt of the compound of formula I.

11. The preparation method for the crystal form of the pharmaceutically acceptable salt of the compound of formula I according to claim 10, wherein
when the preparation method for the crystal form of the pharmaceutically acceptable salt of the compound of formula I is the preparation method for the crystal form **A** of the sulfate salt of the compound of formula I, the preparation method for the crystal form **A** of the sulfate salt of the compound of formula I satisfies one or more than one of the following conditions:
(1) in method **a,** the compound of formula I is a crystal form A of the compound of formula I;
(2) in method **b,** the crystallization comprises the following steps: dissolving a sulfate salt of the compound of formula I in a solvent of C₁₋₃ alcohol to obtain a mixed solution, and adding 2-methyltetrahydrofuran to the resulting mixed solution; the solvent of C₁₋₃ alcohol in the solvent of C₁₋₃ alcohol - 2-methyltetrahydrofuran is one or more than one of methanol, ethanol, and isopropanol, such as methanol;
(3) in method **a,** the crystallization comprises the following steps: suspending, stirring, and crystallizing the compound of formula I and sulfuric acid in methanol;
or,
when the preparation method for the crystal form of the pharmaceutically acceptable salt of the compound of formula I is the preparation method for the crystal form **C** of the sulfate salt of the compound of formula I, the preparation method for the crystal form **C** of the sulfate salt of the compound of formula I satisfies one or more than one of the following conditions:
(1) in method **1** or **2,** the compound of formula I is the crystal form A of the compound of formula I;
(2) in method **2,** the nitrile solvent in the water-nitrile solvent is acetonitrile;
(3) in method **1,** the nitrile solvent is acetonitrile;
(4) in method **1** or **2,** the mass to volume ratio of the compound of formula I to the nitrile solvent is 120-20 mg/mL, such as 100-40 mg/mL;
or,
when the preparation method for the crystal form of the pharmaceutically acceptable salt of the compound of formula I is the preparation method for the crystal form **D** of the sulfate salt of the compound of formula I, the preparation method for the crystal form **D** of the sulfate salt of the compound of formula I satisfies the following conditions (1) and/or (2):
(1) the propanol is n-propanol and/or isopropanol, such as n-propanol;
(2) the mass to volume ratio of the sulfate salt of the compound of formula I to the propanol is preferably 25-40 mg/mL, such as 33.3 mg/mL;
or,
when the preparation method for the crystal form of the pharmaceutically acceptable salt of the compound of formula I is the preparation method for the crystal form **E** of the sulfate salt of the compound of formula I, the preparation method for the crystal form **E** of the sulfate salt of the compound of formula I satisfies the following conditions (1) and/or (2):
(1) the mixed solvent of ester-water is a mixed solvent of ethyl acetate-water;
(2) the volume ratio of the ester solvent to water in the mixed solvent of ester-water is 19:1;
or,
when the preparation method for the crystal form of the pharmaceutically acceptable salt of the compound of formula I is the preparation method for the crystal form **E** of the sulfate salt of the compound of formula I, in the preparation method for the crystal form **F** of the sulfate salt of the compound of formula I, the sulfoxide solvent is dimethyl sulfoxide.

12. A pharmaceutical composition, comprising a therapeutically effective dose of substance **A** and a pharmaceutically acceptable excipient; the substance **A** is the crystal form of the compound of formula I according to any one of claims 1 to 3, the pharmaceutically acceptable salt of the compound of formula I according to any one of claims 4 to 6, or the crystal form of the pharmaceutically acceptable salt of the compound of formula I according to any one of claims 7 to 9.

13. A use of substance **B** in the manufacture of a drug or RIPK1 inhibitor for the treatment and/or prevention of a disease, the substance **B** is the pharmaceutical composition according to claim 12, the crystal form of the compound of formula I according to any one of claims 1 to 3, the pharmaceutically acceptable salt of the compound of formula I according to any one of claims 4 to 6, or the crystal form of the pharmaceutically acceptable salt of the compound of formula I according to any one of claims 7 to 9;
the disease is preferably a RIPK1-mediated disease, and the disease is preferably one or more than one of stroke, inflammatory bowel disease, ulcerative colitis, Crohn's disease, psoriasis, rheumatoid arthritis, NASH, and heart failure; or the disease is preferably one or more than one of stroke, inflammatory bowel disease, ulcerative colitis, Crohn's disease, psoriasis, rheumatoid arthritis, NASH, and heart failure;
the RIPK1 inhibitor is preferably an *in vivo* or *in vitro* RIPK1 inhibitor.
